(19) Europäisches Patentamt
European Patent Office
Office européen des brevets

(11) EP 4 496 786 B1

# (12) EUROPEAN PATENT SPECIFICATION

(45) Date of publication and mention of the grant of the patent:
**08.10.2025 Bulletin 2025/41**

(21) Application number: **24710746.9**

(22) Date of filing: **13.03.2024**

(51) International Patent Classification (IPC):
***C07C 309/15*** *(2006.01)*

(52) Cooperative Patent Classification (CPC):
(C-Sets available)
**C07C 309/15; C08F 220/56;** C07B 2200/13
(Cont.)

(86) International application number:
**PCT/EP2024/056689**

(87) International publication number:
**WO 2024/189090 (19.09.2024 Gazette 2024/38)**

(54) **CRYSTALLINE FORM OF 2-ACRYLAMIDO-2-METHYLPROPANESULPHONIC ACID POTASSIUM SALT**

KRISTALLINE FORM DES KALIUMSALZES DER 2-ACRYLAMIDO-2-METHYLPROPANSULFONSÄURE

FORME CRISTALLINE DE SEL DE POTASSIUM D'ACIDE 2-ACRYLAMIDO-2-MÉTHYLPROPANESULFONIQUE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **13.03.2023 FR 2302310**

(43) Date of publication of application:
**29.01.2025 Bulletin 2025/05**

(60) Divisional application:
**25172353.2 / 4 570 786**

(73) Proprietor: **SNF Group**
**42160 Andrézieux-Bouthéon (FR)**

(72) Inventors:
- **FAVERO, Cédrick**
  **42160 ANDREZIEUX-BOUTHEON (FR)**
- **LEGRAS, Benoit**
  **42160 ANDREZIEUX-BOUTHEON (FR)**
- **KIEFFER, Johann**
  **42160 ANDREZIEUX-BOUTHEON (FR)**

(74) Representative: **Ipsilon**
**Le Contemporain**
**50 Chemin de la Bruyère**
**69574 Dardilly Cedex (FR)**

(56) References cited:
**WO-A1-2014/117685** **WO-A2-2013/079507**
**US-B1- 6 331 647**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C08F 220/56, C08F 220/06, C08F 220/585;**
**C08F 220/56, C08F 220/585**

## Description

### Field of the invention

The present invention relates to a crystalline form of 2-acrylamido-2-methylpropanesulphonic acid. More specifically, the present invention relates to a crystalline form of 2-acrylamido-2-methylpropanesulphonic acid potassium salt. The invention also relates to the method for obtaining the crystalline form of 2-acrylamido-2-methylpropanesulphonic acid potassium salt.

### Prior art

2-acrylamido-2-methylpropanesulphonic acid, which is also referred to as ATBS, is widely used as an additive in acrylic fibers, or as a raw material for producing polymers used as dispersants, thickeners, friction reducers, flocculants or superabsorbents in various sectors including the oil and gas industry, mining, construction, textiles, water treatment (seawater desalination, mineral industry, etc.) or cosmetics.

The reaction carried out in the method for preparing 2-acrylamido-2-methylpropanesulphonic acid corresponds to the reaction scheme below, in which acrylonitrile is present in excess so as to be both the reaction solvent and a reagent. The acrylonitrile is brought into contact with fuming sulphuric acid (oleum) and isobutylene.

[Chem. 1]

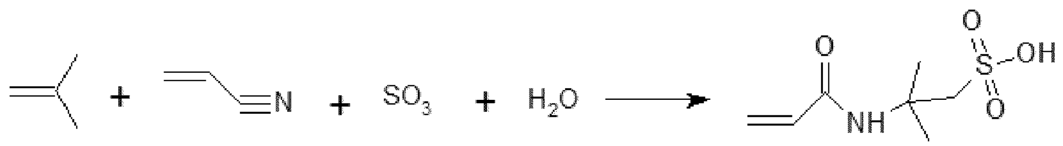

One by-product that may be produced during this synthesis is acrylamide.

2-acrylamido-2-methylpropanesulphonic acid is not soluble in acrylonitrile solvent. As a result, the reaction product is in the form of a suspension of crystals in the reaction solvent.

For example, documents US 6,448,347 and CN 102351744 describe a method for the continuous production of 2-acrylamido-2-methylpropanesulphonic acid. The 2-acrylamido-2-methylpropanesulphonic acid is then separated from the acrylonitrile, generally by filtration, and then dried.

Document US 6,331,647 relates to a process for the preparation of a purified monomer of 2-acrylamido-2-methylpropanesulphonic acid in salt form.

It is necessary to dry the 2-acrylamido-2-methylpropanesulphonic acid in order to reduce the quantity of residual acrylonitrile and acrylamide present in the crystal. These two compounds are classified as carcinogenic, mutagenic or reprotoxic (CMR). It is therefore necessary to carry out effective filtration to dewater the acrylonitrile as thoroughly as possible, and then dry the 2-acrylamido-2-methylpropanesulphonic acid in order to obtain low levels of acrylonitrile and acrylamide.

A person skilled in the art is aware that 2-acrylamido-2-methylpropanesulphonic acid crystals have a crystallographic arrangement that produces a needle-shaped solid crystals.

Needle-shaped crystals are known to a person skilled in the art to have macroscopic properties that pose difficulties in solid handling and transport operations (poor solid flowability, caking, low resistance to shear stress), and processing operations (poor filterability, drying difficulties, attrition).

In the case of 2-acrylamido-2-methylpropanesulphonic acid, the additional problems encountered are generally the small particle size of the crystals in the form of needles, the density of the solid in question, and the explosive nature of the fine dust.

These macroscopic properties are linked directly to the morphology of the crystals and to their specific surface area. Crystals in the form of needles have a high specific surface area.

Patents WO 2009/072480, JP 2008/307822 and JP 2003/137857 describe that the 2-acrylamido-2-methylpropanesulphonic acid crystals obtained are needle-shaped.

Document WO 2018/172676 filed by the Applicant describes a novel form of 2-acrylamido-2-methylpropanesulphonic acid crystals referred to as a "hydrated crystalline form of 2-acrylamido-2-methylpropanesulphonic acid". This novel crystalline form has different physico-chemical properties to the needle form and also gives the polymers comprising 2-acrylamido-2-methylpropanesulphonic acid in this novel form improved properties.

However, regardless of the form of 2-acrylamido-2-methylpropanesulphonic acid, it remains a strong acid due to

its sulphonic acid function, which is highly corrosive to metals. Due to the powdery nature of 2-acrylamido-2-methylpropanesulphonic acid powder, there is also a risk of chemical burns due to fine airborne particles coming into contact with the skin or eyes or being breathed into the lungs during powder handling operations.

When 2-acrylamido-2-methylpropanesulphonic acid is used in a polymerization method, it must be in aqueous form. The aqueous phase may be used as it is, i.e., in acid form, or after neutralization with an alkali metal, an alkaline-earth metal or a molecule containing an unsubstituted or substituted amine function.

The shelf life of this aqueous solution is generally short because of self-polymerization phenomena caused by exposure to temperature, UV light or pollutants such as iron or its oxidized forms, which can be produced by the corrosion of metal pipes or containers caused by the acid form of 2-acrylamido-2-methylpropanesulphonic acid. In addition, the increase in temperature generated by the self-polymerization of 2-acrylamido-2-methylpropanesulphonic acid is well above the boiling point of water, which can lead to an increase in pressure in the container and cause an explosion. Therefore, self-polymerization phenomena present a certain risk to the safety of people and installations.

The Applicant has discovered a novel form of 2-acrylamido-2-methylpropanesulphonic acid referred to as a "crystalline form of 2-acrylamido-2-methylpropanesulphonic acid potassium salt", referred hereafter as ATBS.K. This novel form offers improved physico-chemical and application properties (as with the "hydrated crystalline" form) while avoiding the neutralization stage. The risks of burning, corrosion and self-polymerization are also reduced. Finally, the crystalline form of ATBS.K has a longer shelf life than an aqueous solution of ATBS.K.

Using the crystalline form of ATBS.K according to the invention is in line with the principle of environmental awareness and the impact of industry and mankind on the planet. The novel form of the product means that it is safer for handlers to use and reduces the energy impact due to the neutralization step that is no longer necessary during the polymerization of ATBS and due to the powder form, which allows more active ingredient to be transported (100% for the powder compared with a maximum of 50% for a solution). The improved shelf life of the product also means less waste resulting from the increased product dosage required as a result of the reduced performance of a product that is too old. Moreover, the improved performances of the polymers obtained from the crystalline form of ATBS.K of the invention help reduce the quantity of product necessary for the applications in which they are used, reducing the overall water consumption and emissions of greenhouse gases such as the $CO_2$.

## Disclosure of the invention

The object of the present invention is a specific form of 2-acrylamido-2-methylpropanesulphonic acid referred to hereinafter as a "crystalline form of 2-acrylamido-2-methylpropanesulphonic acid potassium salt".

The present invention also relates to a method for producing the crystalline form of ATBS.K.

## Description of the invention

The term "polymer" should be understood to mean a homopolymer or a copolymer. The term "copolymer" should be understood to mean a polymer obtained from at least two different monomers. It may therefore be a copolymer of at least two monomers chosen from hydrophilic anionic monomers, hydrophilic cationic monomers, hydrophilic non-ionic monomers, hydrophilic zwitterionic monomers, hydrophobic monomers and the mixtures thereof.

The term "hydrophilic monomer" should be understood to mean a monomer that has an octanol-water partition coefficient, $K_{ow}$, equal or less than 1, in which the partition coefficient $K_{ow}$ is determined at 25°C in an octanol-water mixture with a volume ratio of 1/1, at a pH of between 6 and 8.

The term "crystal" or "crystalline form" refers to a solid material whose constituents (such as atoms, molecules, or ions) are arranged in a highly ordered microscopic structure, forming a crystal lattice that extends in all directions. It does not encompass amorphous solid.

The term "hydrophobic monomer" should be understood to mean a monomer that has an octanol-water partition coefficient, $K_{ow}$, greater than 1, in which the partition coefficient $K_{ow}$ is determined at 25°C in an octanol-water mixture with a volume ratio of 1/1, at a pH of between 6 and 8.

The octanol-water partition coefficient, $K_{ow}$, represents the ratio of concentrations (g/L) of a monomer between the octanol phase and the aqueous phase. It is defined as follows:

[Math 1]

$$K_{ow} = \frac{[\ monomer\ ]_{octanol}}{[\ monomer\ ]_{water}}$$

By definition, a water-soluble polymer is a polymer that gives an aqueous solution when it is dissolved while stirring at 25°C and with a concentration of 10 $g.L^{-1}$ in water.

"X and/or Y" should be understood to mean "X", or "Y", or "X and Y".

The invention also includes all possible combinations of the various embodiments disclosed, whether they are preferred embodiments or given by way of example. Furthermore, when ranges of values are indicated, the limit values are included in these ranges. The disclosure also includes all of the combinations between the limit values of these ranges of values. For example, the ranges of values "1-20, preferably 5-15" imply disclosure of the ranges "1-5", "1-15", "5-20" and "15-20" and the values 1, 5, 15 and 20.

**Crystalline form of ATBS.K**

The present invention relates to a crystalline form of 2-acrylamido-2-methylpropanesulphonic acid potassium salt, referred hereafter as ATBS.K, having an X-ray powder diffraction pattern comprising peaks at 13.1°; 14.4°; 16.3°; 19.8°; 23.5°; 24.3°; 26.9°; 27.6°; 29.3°; 30.6°; 31.6°; 34.3°; 36.1°; 41.7°; 44.6°; 46.7°, 2-theta angles. The uncertainty of these peaks is generally of the order of +/- 0.1°.

X-ray crystallography, radiocrystallography or X-ray diffractometry is an analytical technique used to study the structure of crystalline matter on an atomic scale. It is based on the physical phenomenon of X-ray diffraction. A diffractometer with a copper source can be used.

A powder formed from a particular crystalline phase always shows diffraction peaks in the same directions. This diffraction pattern thus forms a true signature of the crystalline phase. It is therefore possible to determine the nature of each crystalline phase within a mixture or a pure product.

This signature is specific to each organic or inorganic compound, and is in the form of a list of peaks positioned at an angle of $2\theta$ (2-theta).

This technique is used to characterize matter, in particular the different crystalline forms that can exist for the same chemical molecule, also known as polymorphs.

Another aspect of the invention relates to a crystalline form of ATBS.K having a Fourier-transform infrared spectrum comprising peaks at 3293 $cm^{-1}$, 3075 $cm^{-1}$, 3000 $cm^{-1}$, 2979 $cm^{-1}$, 1655 $cm^{-1}$, 1625 $cm^{-1}$, 1550 $cm^{-1}$, 1405 $cm^{-1}$, 1209 $cm^{-1}$, 1190 $cm^{-1}$, 1162 $cm^{-1}$, 1048 $cm^{-1}$, 979 $cm^{-1}$, 824 $cm^{-1}$, 803 $cm^{-1}$, 756 $cm^{-1}$, 633 $cm^{-1}$, 523 $cm^{-1}$. The uncertainty of these peaks is generally of the order of +/- 8 $cm^{-1}$.

The infrared measurement is carried out by Fourier transform, for example using a Perkin Elmer Spectrum 100 spectrometer fitted with a single reflection ATR polarization accessory, with an accuracy of 8 $cm^{-1}$.

Fourier-transform infrared spectroscopy is the analysis of the vibrations emitted, absorbed or scattered by molecules. This technique is sensitive to so-called short interactions (influence of the unit mesh on the bonds). In most cases, the Fourier-transform infrared spectra of different crystalline systems differ significantly. The Fourier-transform infrared spectrum therefore reflects the details of the crystalline structure of a chemical compound.

Generally, and unless otherwise indicated, the X-ray diffraction pattern and the infrared spectrum are obtained at 20°C and at a pressure of 1 atmosphere absolute (101,325 Pa).

Another aspect of the invention relates to a crystalline form of ATBS.K having a minimum ignition energy greater than 500 mJ, and preferably greater than 1000 mJ.

The minimum ignition energy represents the minimum energy that must be supplied to a product (chemical compound) to cause it to ignite. The energy may be electrical or thermal. The minimum ignition energy is an essential piece of information when considering the risk of explosion during product handling (transfer, storage, reaction, shaping, etc.).

The minimum ignition energy depends on the properties of the powder (composition) and its macromolecular structure (particle size, crystalline form, specific surface area).

In the case of solids, this energy is the minimum energy of an electric spark likely to ignite a cloud of dust. The higher the value of the minimum ignition energy, the less risk the solid presents when used, handled or stored.

The minimum ignition energy is measured in accordance with standard NF EN 13821.

Another aspect of the present invention relates to a crystalline form of ATBS.K exhibiting 2 thermal phenomena with the differential scanning calorimetry technique, at 79.4°C; 207°C. The uncertainty relating to the observation of these phenomena is generally of the order of 10°C (+/-10°C), advantageously 5°C or less.

The thermal phenomena are measured by differential scanning calorimetry (DSC). This technique uses the measurement of the variation in heat associated with the thermal denaturation of the compound when it is heated at constant speed, for example with a heating ramp of 10°C/minute.

**Method for producing the crystalline form of ATBS.K**

The present invention also relates to the method for producing the crystalline form of ATBS.K comprising at least the following successive steps:

1) mixing of 2-acrylamido-2-methylpropanesulphonic acid with an aqueous solution $SA_1$ and at least one potassium salt base, advantageously for at least 1 minute, in order to form an aqueous solution or an aqueous suspension $SA_2$;
2) distillation, at a pressure of 700 mbar or less, of the aqueous solution or of the aqueous suspension $SA_2$ in order to form a suspension $S_1$ and cooling of the aqueous solution or aqueous suspension $SA_2$ or a suspension $S_1$ wherein the temperature of the cooling step is decreased at a ramp of between 0.1 and 8°C/hour;
3) solid-liquid separation of the suspension $S_1$ and isolation of the crystals of the suspension $S_1$ obtained at the end of step 2) in the form of a composition $C_1$.

The crystals obtained are in the crystalline form of ATBS.K.

In step 1), "potassium salt(s) base" should be understood to mean at least one inorganic potassium salt Brønsted base, for example potassium hydroxide, potassium carbonate, potassium bicarbonate or mixtures thereof.

The temperature and the mixing time of step 1) may vary as a function, in particular, of the concentration of 2-acrylamido-2-methylpropanesulphonic acid ATBS. A person skilled in the art knows how to adapt the temperature variation and the mixing time to optimize the formation of the crystals.

The method for producing the crystalline form of ATBS.K can be carried out on any form of ATBS, such as, for example, the needle form or the hydrated form.

The production method may be carried out on ATBS of any degree of purity.

Therefore, the method may be carried out downstream of any type of method for producing ATBS. It may also be carried out on crystals of ATBS that have already been obtained.

**Step 1) of the method for producing the crystalline form of ATBS.K:**

ATBS is produced by a production method as previously described (acrylonitrile, fuming sulphuric acid and isobutylene). The 2-acrylamido-2-methylpropanesulphonic acid may be in the form of fine powder or shaped in a controlled manner by a method such as compaction, granulation or extrusion.

ATBS may be added to an aqueous solution $SA_1$ before, after or in parallel with the potassium salt base, and preferably in parallel. Preferably, the aqueous solution $SA_1$ is water.

The potassium salt base may be added as an aqueous solution. In this case, the aqueous solution of potassium salt base can be partially or totally the aqueous solution $SA_1$.

Advantageously, the concentration of ATBS potassium salt in the aqueous solution or in the aqueous suspension $SA_2$ is between 1% by weight and saturation, preferably between 10% by weight and saturation, more preferably between 20% by weight and saturation, more preferably between 30% by weight and saturation, more preferably between 40% by weight and saturation, and even more preferably between 50% by weight and saturation, by weight relative to the weight of the aqueous solution or of the aqueous suspension $SA_2$.

The ATBS and the potassium salt base may be added all at once or in several stages. They are preferably added in several stages. They are preferably added all at once.

When they are added in several stages, the ATBS and the potassium salt base are added in fractions.

When the ATBS and the potassium salt base are added in fractions, there is no limit to the number of fractions, advantageously there are at least two fractions, and preferably at least three fractions.

There is no limitation to the order of addition of the ATBS and the potassium salt base. They may be added at the same time (i.e., in parallel), one after the other (the ATBS first, then the potassium salt base, or vice versa), or alternately (a first fraction pf ATBS, then a first fraction of the potassium salt base, followed by a second fraction of ATBS then a second fraction of the potassium salt base, and so on); they are preferably added at the same time.

When they are added one after the other or alternately, the second compound (whether it is the ATBS or the potassium salt base) can start being added before the first compound has finished being added.

A first fraction F1 of ATBS advantageously represents at least 1 mol% of the total of the ATBS present in the aqueous solution or the aqueous suspension $SA_2$, preferably at least 5 mol%, more preferably at least 10 mol%, even more preferably at least 15 mol% and even more preferably at least 20 mol%.

A second fraction F2 of ATBS advantageously represents at least 1 mol% of the total of the ATBS present in the aqueous solution or the aqueous suspension $SA_2$, preferably at least 5 mol%, more preferably at least 10 mol%, even more preferably at least 15 mol% and even more preferably at least 20 mol%.

A third fraction F3 of ATBS advantageously represents at least 1 mol% of the total of the ATBS present in the aqueous solution or the aqueous suspension $SA_2$, preferably at least 5 mol%, more preferably at least 10 mol%, even more preferably at least 15 mol% and even more preferably at least 20 mol%.

In one particular embodiment, the method is carried out continuously, in which case the ATBS and the potassium salt base are added continuously.

The quantity of ATBS in the aqueous solution or the aqueous suspension $SA_2$ is advantageously between 10 and 90% by weight relative to the total weight of the aqueous solution or of the aqueous suspension $SA_2$, preferably between 20

and 85% by weight, more preferably between 30 and 80% by weight.

The mixing of step 1) (ATBS + potassium salt base) is advantageously carried out at a temperature of between 0 and 90°C, preferably between 5 and 60°C, more preferably between 10 and 40°C, in order to obtain the aqueous solution or the aqueous suspension $SA_2$.

In one particular embodiment, the aqueous solution or the suspension $SA_2$ may comprise one or more organic solvents.

In some embodiments, the aqueous solution $SA_1$ may comprise one or more organic solvents.

The quantity of organic solvent may vary as a function of the temperature and the quantity of ATBS or potassium salt base. This quantity is not limited, provided it does not prevent the crystalline form of ATBS.K from being obtained. A person skilled in the art knows how to determine this limit, which is a routine task. Generally, the aqueous solution or the aqueous suspension $SA_2$ comprises more water (by volume) than organic solvent.

The organic solvent or solvents are advantageously chosen from the following compounds:

- organic acids, advantageously carboxylic acids comprising between 1 and 8 carbon atoms;
- amides advantageously comprising between 1 and 8 carbon atoms;
- alcohols advantageously comprising between 1 and 8 carbon atoms;
- ketones advantageously comprising between 3 and 8 carbon atoms;
- ethers advantageously comprising between 2 and 8 carbon atoms;
- esters advantageously comprising between 2 and 8 carbon atoms;
- alkanes advantageously comprising between 4 and 8 carbon atoms, preferably between 5 and 6 carbon atoms;
- halogenated hydrocarbon compounds advantageously comprising between 1 and 8 carbon atoms;
- nitriles advantageously comprising between 1 and 8 carbon atoms; or
- mixtures thereof.

When an organic solvent is used in the invention, the temperature may be adjusted so that the solvent + water mixture remains in liquid form.

These compounds may be linear or branched. They may be saturated or comprise unsaturated bonds. An unsaturated bond corresponds to a double or triple bond (for example C=C or C≡C).

The organic solvent is preferably chosen from acrylonitrile, isopropanol, acetic acid or mixtures thereof. The organic solvent is preferably acrylonitrile.

The organic solvent is generally in liquid form at the temperature at which steps 2) and 3) are carried out. Furthermore, it is advantageously partially miscible in water, and preferably completely miscible in water.

The organic solvent may, if necessary, be used to solubilize any impurities or by-products present with the ATBS used to form the aqueous solution or the aqueous suspension $SA_2$. However, ATBS is not necessarily soluble in the solvent.

In a preferred embodiment according to the invention, the aqueous solution or the aqueous suspension $SA_2$ does not contain organic solvent.

In a preferred embodiment according to the invention, the aqueous solution $SA_1$ does not contain organic solvent.

The time allowed for mixing the aqueous solution $SA_1$ and the ATBS is advantageously at least 1 minute, preferably between 1 minute and 600 minutes, more preferably between 5 minutes and 400 minutes, and even more preferably between 10 minutes and 240 minutes.

The compounds of step 1) can be mixed using various technologies. Examples include, but are not limited to, reactors with stirrers, loop reactors, static mixers, microreactors, piston reactors, agitated filter dryers, for example by Nutsche, paddle mixers, twin-cone mixers, ploughshare mixers and disc mixers.

The pH in step 1) is advantageously controlled between 6 and 14, preferably between 8 and 14, more preferably between 10 and 14, even more preferably between 12 and 14, and even more preferably between 13 and 14.

The quantity of ATBS.K in the aqueous solution $SA_2$ or the aqueous suspension $SA_2$ is advantageously between 10 and 90% by weight relative to the total weight of the aqueous solution or of the aqueous suspension $SA_2$, preferably 20 and 90%, preferably between 30 and 90% by weight, preferably between 50 and 90% by weight, preferably between 20 and 85% by weight, more preferably between 30 and 80% by weight.

**Step 2) of the method for producing the crystalline form of ATBS.K:**

The distillation of the aqueous solution or of the aqueous suspension $SA_2$ takes place at a pressure of 700 mbar or less. It generally takes place in a vacuum distillation device, which is typically an evaporator. It is therefore also referred to here as "vacuum distillation".

When the aqueous solution or the aqueous suspension $SA_2$ is distilled, typically by passing it through an

evaporator, crystals of ATBS.K begin to form. Thus ATBS, at least one potassium salt base, and crystalline solid particles of ATBS.K coexist in the aqueous solution or the aqueous suspension $SA_2$.

The aqueous solution or an aqueous suspension $SA_2$ can be distilled using an evaporator. This may be a falling film evaporator, or a rising film evaporator, or a scraped thin film evaporator, or a short path evaporator, or a forced circulation evaporator, or a spiral tube evaporator, or a flash evaporator. It may also be a continuously stirred reactor. Preferably, distillation takes place in a scraped thin film evaporator, a short path evaporator or a forced circulation evaporator. Even more preferably, distillation takes place in a scraped thin film evaporator.

Generally, an evaporator is a device comprising an inlet for solution to be treated (aqueous solution or aqueous suspension $SA_2$), an outlet for discharging the distilled solvent (the water and any organic solvents) and an outlet for discharging the suspension $S_1$.

The residence time of the aqueous solution or of the aqueous suspension $SA_2$ in the distillation device (advantageously under vacuum), which is advantageously an evaporator, in other words the distillation time at a pressure of 700 mbar or less, is advantageously comprised between 1 second and 600 seconds, preferably between 3 seconds and 300 seconds, more preferably between 30 seconds and 100 seconds. The residence time corresponds to the time necessary to carry out step 2), i.e., the time required to prepare the suspension $S_1$ by distillation of the aqueous solution or of the aqueous suspension $SA_2$. In other words, when an evaporator is used, it is the residence time of the ATBS (and/or the crystalline form of its potassium salt) between the inlet and the outlet of the device. This residence time depends on the quantity of water (and any organic solvents), ATBS and potassium salt base present in the aqueous solution or the aqueous suspension $SA_2$. A person skilled in the art knows how to adapt this residence time in order to obtain ATBS.K in the crystalline form depending on the quantity of the constituents of the aqueous solution or the aqueous suspension $SA_2$.

The distillation can be carried out in a vertical or horizontal evaporator. Preferably, it is carried out in a vertical evaporator.

The aqueous solution or the aqueous suspension $SA_2$ can circulate co-current or counter-current to the vapours generated by the evaporation. Preferably, it circulates counter-current to the vapours in the distillation device. In other words, the aqueous solution or the aqueous suspension $SA_2$ is preferably introduced into the distillation device, advantageously an evaporator, co-current or counter-current to the distilled solvent.

The aqueous solution or the aqueous suspension $SA_2$ may circulate in one or more evaporators in series before obtaining the suspension $S_1$. Preferably, it circulates in a single evaporator.

The pressure during distillation is advantageously between 1 and less than 700 mbar absolute (1 mbar = 100 Pa). It is preferably less than 700 mbar absolute, more preferably less than 600 mbar absolute, more preferably less than 500 mbar absolute, more preferably less than 400 mbar absolute, more preferably less than 300 mbar absolute, more preferably less than 200 mbar absolute, more preferably less than 100 mbar absolute and even more preferably less than 50 mbar absolute, and advantageously greater than 1 mbar absolute. The absolute pressure corresponds to the pressure relative to zero pressure (vacuum).

In general, the pressure during distillation is preferably comprised between 10 and 700 mbar, preferably between 20 and 700 mbar, preferably between 40 and 700 mbar more preferably between 50 and 600 mbar, more preferably between 50 and 500 mbar, more preferably between 50 and 400 mbar, more preferably between 50 and 300 mbar, more preferably between 100 and 700 mbar, more preferably between 200 and 700 mbar, more preferably between 500 and 700 mbar, more preferably between 40 and 100 mbar.

In one particular embodiment, step 2) comprises a step 2') (optional) for helping the solvent to evaporate. Step 2') consists in increasing the temperature of the aqueous solution or of the aqueous suspension $SA_2$, in other words the distillation according to step 2') is carried out under heat.

In some embodiments, in step 2), the aqueous solution or the aqueous suspension $SA_2$ is heated, advantageously at a temperature of between 5°C and 95°C, preferably between more than 10°C and 60°C, more preferably between more than 20°C and 40°C.

The heating during distillation may be carried out by various technologies. Examples include, but are not limited to, heating with steam, with hot water, with electricity, by steam compression, or indeed by using a heat pump. Thus, the distillation device may be of the double-walled type, with a hot heat-transfer fluid circulating between the two walls.

The aqueous solution or the aqueous suspension $SA_2$ is advantageously heated to a temperature between more than 5°C and 95°C, preferably between more than 10°C and 60°C, more preferably between more than 20°C and 40°C.

When the aqueous solution or the aqueous suspension $SA_2$ is heated, the temperature is advantageously greater than the temperature of step 1).

The temperature of the aqueous solution or of the aqueous suspension $SA_2$ advantageously increases at a ramp of between 0.1 and 10°C/hour, preferably between 0.2 and 9°C/hour, more preferably between 0.3 and 8°C/hour, and even more preferably between 0.5 and 5°C/hour.

In some embodiments, the temperature of the aqueous solution or of the aqueous suspension $SA_2$ advantageously increases at a ramp of between 10 and 150°C/hour, preferably between 30 and 110°C/hour, more preferably between 50 and 100°C/hour, and even more preferably between 60 and 90°C/hour.

The increase in temperature may not be constant throughout the whole process. For example, the aqueous solution or the aqueous suspension $SA_2$ may be heated by 5°C per hour for the first three hours, and then heated at a speed of 10°C per hour until the final temperature is reached.

According to another particular embodiment of the invention, step 2) comprises a step 2"), after step 2'), or instead of step 2'), that helps to increase the productivity and the profitability of the method of the invention by accelerating the crystallization of the ATBS into the crystalline form of its potassium salt. Step 2") consists in decreasing the temperature of the aqueous solution or of the aqueous suspension $SA_2$ or the suspension S1.

The aqueous solution or the aqueous suspension $SA_2$ is advantageously cooled to a temperature of between 5 and less than 95°C, preferably between 10 and less than 60°C, more preferably between 20 and less than 40°Cand even more preferably between 10 and 40°C.

Step 2) further comprises a cooling step.

The cooling step is advantageously carried out at a temperature of between 5°C and 95°C, preferably between more than 10°C and 60°C, more preferably between more than 10°C and 40°C.

The temperature of the cooling step is decreased at a ramp of between 0.1 and 8°C/hour, preferably between 0.2 and 8°C/hour, more preferably between 0.3 and 8°C/hour, and even more preferably between 0.5 and 5°C/hour.

In some embodiments, the temperature of the cooling step is advantageously inferior to the temperature of heating of step 2) and/or step 1).

The cooling step is carried out on the aqueous solution or the aqueous suspension $SA_2$ and/or the concentrated aqueous solution or the aqueous suspension $SA_2$ and/or the suspension $S_1$.

When the aqueous solution or the aqueous suspension $SA_2$ is cooled (step 2''), the temperature is advantageously less than the temperature of steps 2) and optionally 2').

According to a preferred embodiment, the temperature of step 2") is the same or less than the temperature of step 1).

In some embodiments, no organic solvent or aqueous solution is added in step 2'' for obtaining the crystals of ATBS.K.

The temperature of the solution or of the aqueous suspension $SA_2$ decreases at a ramp of between 0.1 and 8°C/hour, preferably between 0.2 and 8°C/hour, more preferably between 0.3 and 8°C/hour, and even more preferably between 0.5 and 5°C/hour.

The decrease in temperature may not be constant throughout the whole process. For example, the aqueous solution or the aqueous suspension $SA_2$ may be cooled by 5°C per hour for the first three hours, and then cooled at a speed of 8°C per hour until the final temperature is reached.

While the aqueous solution or the aqueous suspension $SA_2$ is being cooled, crystals of ATBS.K form and a suspension $S_1$ is obtained.

In one particular embodiment, previously obtained crystals of ATBS.K may be added during this step in order to modify the formation of the suspension $S_1$, a process referred to as crystal seeding which makes it possible to better control the crystallization temperature, the particle size of the crystals, the particle-size distribution, the purity of the end product and, possibly, the yield. The crystals of potassium salt of 2-acrylamido-2-methylpropanesulphonic acid added in this way advantageously have an X-ray powder diffraction pattern comprising peaks at 13.1°; 14.4°; 16.3°; 19.8°; 23.5°; 24.3°; 26.9°; 27.6°; 29.3°; 30.6°; 31.6°; 34.3°; 36.1°; 41.7°; 44.6°; 46.7° 2 theta (+/- 0.1°).

According to one particular embodiment of the invention, the solvent distilled in step 2) may be partially or totally recycled in order to form the aqueous solution or aqueous suspension $SA_2$ of step 1). In other words, the distilled solvent is advantageously at least partially recycled in the aqueous solution or aqueous suspension $SA_2$.

According to another particular embodiment of the invention, the distilled solvent may be partially or totally recycled, generally to wash the crystals of ATBS.K obtained after step 3) of solid-liquid separation, in an optional step 4), with or without a prior treatment step.

The obtained suspension $S_1$ advantageously comprises between 30 and 80% by weight of the crystalline form of ATBS.K, relative to the total weight of the suspension $S_1$, and preferably between 50 and 60% by weight.

During step 2), the pH is advantageously greater than 10, preferably greater than 11, more preferably greater than 12, and the pH is even more preferably between 13 and 14.

**Step 3) of the method for producing the crystalline form of ATBS.K:**

The crystals of ATBS.K contained in the suspension $S_1$ obtained at the end of step 2) are isolated in a solid-liquid separation step and are in the form of a composition $C_1$.

The solid-liquid separation step can be carried out using various technologies. Examples include, but are not limited to, the use of a centrifuge, a decanter, a filter press, an agitated filter, a belt filter, a disc filter or a rotary drum filter. The solid-liquid separation is preferably carried out using a centrifuge. The solid-liquid separation may also be carried out by gravitational settling.

Step 3) is advantageously carried out at a temperature of between -20 and 40°C, and preferably between -5 and 30°C.

After step 3) of solid-liquid separation, the crystals of potassium salt of 2-acrylamido-2-methylpropanesulphonic acid are preferably not dried.

The isolated composition $C_1$ has a content of crystals of potassium salt of 2-acrylamido-2-methylpropanesulphonic acid advantageously between 40 and 99%, preferably between 60 and 99% by weight and more preferably between 60 and 98% by weight and even more preferably between 80 and 99% by weight relative to the weight of the composition $C_1$. The remainder of the composition $C_1$ may be water and/or solubilized ATBS.K, and possibly potassium salt base introduced in step 1).

At the end of this step 3), the crystals are characterized as being crystals of ATBS.K.

In one particular embodiment, all or part of the liquid phase obtained following the solid-liquid separation is used in the aqueous solution or aqueous suspension $SA_2$ of step 1).

During step 4), the pH is advantageously controlled between 6 and 14, preferably between 8 and 14, more preferably between 10 and 14, even more preferably between 12 and 14, and even more preferably between 13 and 14.

**Step 4) of the method for producing the crystalline form of ATBS.K:**

In an optional step 4), the composition $C_1$ containing the crystals of ATBS.K obtained at the end of step 3) is washed using a washing solution.

The washing solution may be water, an aqueous solution of potassium salt base (which may or may not be saturated), or a solution (which may or may not be saturated) of potassium salt of 2-acrylamido-2-methylpropanesulphonic acid (advantageously in the crystalline form of ATBS.K), and it is preferably a saturated solution of ATBS.K.

Examples of solutions of potassium salt base include a solution of potassium hydroxide, potassium carbonate, potassium bicarbonate or mixtures thereof.

The washing solution may comprise one or more organic solvents.

The quantity of organic solvent may vary as a function of the temperature and the quantity of potassium salt of 2-acrylamido-2-methylpropanesulphonic acid or the quantity of potassium salt.

Advantageously, the washing solution does not comprise organic solvent.

As already indicated for step 1), the organic solvent is advantageously chosen from organic acids, amides, alcohols, ketones, ethers, esters, alkanes, halogenated hydrocarbon compounds, nitriles, or mixtures thereof. The organic solvent is preferably chosen from acrylonitrile, isopropanol, acetic acid or mixtures thereof. More preferably, the organic solvent is acrylonitrile.

In one particular embodiment, the composition $C_1$ obtained at the end of step 3) is washed by spraying washing solution over said composition $C_1$.

In one particular embodiment, the composition $C_1$ obtained at the end of step 3) is washed by placing the composition $C_1$ in suspension in the washing solution.

The weight ratio of the aqueous washing solution to the composition $C_1$ obtained at the end of step 3) is advantageously between 0.05:1 and 10:1 and more preferably between 0.1:1 and 5: 1.

This washing step is advantageously carried out at a temperature of between -5 and 40°C, and preferably between 0 and 30°C. A person skilled in the art knows how to adjust the temperature so as not to solubilize the crystals of ATBS.K.

The crystals of ATBS.K obtained at the end of this optional step 4) can be isolated from the washing solution by a solid-liquid separation step, in the form of a composition $C_2$.

The solid-liquid separation step can be carried out using various technologies. Examples include, but are not limited to, the use of a vertical or horizontal centrifuge, a decanter, a filter press, a belt filter, a disc filter, a push filter or a rotary drum filter. The solid-liquid separation may also be carried out by gravitational settling.

In one particular embodiment, all or part of the recovered washing solution may be used again in step 4), with or without a prior treatment step.

In one particular embodiment, all or part of the recovered washing solution may be used in the aqueous solution or aqueous suspension $SA_2$ in step 1), with or without a prior treatment step.

The pH of the washing solution of step 5 is advantageously controlled between 6 and 14, and preferably between 8 and 14.

**Step 5) of the method for producing the crystalline form of ATBS.K:**

In an optional step 5), the composition $C_1$ obtained at the end of step 3) or the composition $C_2$ obtained at the end of step 4) is dried.

The drying step can be carried out using various technologies. Examples include, but are not limited to, the use of

all convection, conduction or radiation drying technologies (fluidized bed dryer, through-bed dryer, drying by conveyor belt, microwave, heated agitated filter, high-frequency radiation, infrared radiation, spraying).

The drying operation may be carried out at atmospheric pressure or else under vacuum.

The drying step may be carried out discontinuously (batch drying) or continuously.

**Other steps of the method for producing the crystalline form of ATBS.K:**

During the production method, i.e., during steps 1) to 5), and regardless of the step, at least one polymerization inhibitor may be introduced in order to prevent the possible polymerization of ATBS or its salt. This inhibitor may be chosen, in a non-limiting manner, from hydroquinone, paramethoxyphenol, phenothiazine, 2,2,6,6-tetramethyl(piperidin-1-yl)oxyl, 4-hydroxy-2,2,6,6-tetramethyl(piperidin-1-yl)oxyl, phenylene diamine derivatives, or mixtures thereof.

The inhibitor is preferably paramethoxyphenol or 4-hydroxy-2,2,6,6-tetramethyl(piperidin-1-yl)oxyl.

The quantity of inhibitor that is introduced relative to the quantity of ATBS introduced in step 1) is advantageously between 0.001% and 5% by weight, and more preferably between 0.01% and 1% by weight.

The inhibitor may be introduced during any one or more of the steps of the method. An additional quantity of it is preferably introduced during step 1). More preferably, the inhibitor is part of the aqueous solution $SA_1$ or the aqueous solution or aqueous suspension $SA_2$ introduced in step 1).

The production method (steps 1) to 5)) may be carried out continuously or discontinuously (batch production).

**Polymer**

In some embodiments, the novel crystalline form of ATBS.K is used for producing polymers.

The polymer is obtained at least from ATBS, which is at least partially in the crystalline form of ATBS.K having an X-ray powder diffraction pattern comprising peaks at 13.1°; 14.4°; 16.3°; 19.8°; 23.5°; 24.3°; 26.9°; 27.6°; 29.3°; 30.6°; 31.6°; 34.3°; 36.1°; 41.7°; 44.6°; 46.7°, 2-theta angles (+/- 0.1°).

The polymer is obtained at least partially from the crystalline form of ATBS.K, and advantageously from at least one other monomer chosen from: hydrophilic non-ionic monomers, hydrophilic anionic monomers (distinct from the crystalline form of ATBS.K), hydrophilic cationic monomers, hydrophilic zwitterionic monomers and hydrophobic monomers.

It may therefore be a polymer of several distinct monomers or a homopolymer.

Advantageously, at least 10 mol%, preferably at least 30 mol%, more preferably at least 50 mol%, and even more preferably at least 70 mol% of the ATBS used to obtain the polymer is in the crystalline form of ATBS.K. Even more preferably, 100 mol% of the ATBS is in the crystalline form of ATBS.K.

The polymer advantageously comprises between 1 and 100 mol% of ATBS, preferably between 2 and 60 mol%, more preferably between 3 and 25 mol% and, advantageously, at least 10 mol%, preferably at least 30 mol%, more preferably at least 50 mol%, and even more preferably at least 70 mol% is in the crystalline form of ATBS.K. Even more preferably, 100 mol% of ATBS that is used is in the crystalline form of ATBS.K.

In one particular embodiment, the polymer advantageously comprises at least 10 mol% of ATBS, preferably at least 20 mol%, more preferably at least 30 mol%, more preferably at least 40 mol%, more preferably at least 50 mol%, more preferably at least 60 mol%, more preferably at least 70 mol%, more preferably at least 80 mol%, more preferably at least 90 mol% and, advantageously, at least 10 mol%, preferably at least 30 mol%, more preferably at least 50 mol%, and even more preferably at least 70 mol% is in the crystalline form of ATBS.K and, even more preferably, 100% of ATBS that is used is in the crystalline form of ATBS.K.

In one particular embodiment, the polymer is a homopolymer of ATBS and, advantageously, at least 10 mol%, preferably at least 30 mol%, more preferably at least 50 mol%, and even more preferably at least 70 mol% is in the crystalline form of ATBS.K, and even more preferably 100% of ATBS that is used is in the crystalline form of ATBS.K.

In one particular embodiment, the polymer is a homopolymer of the crystalline form of ATBS.K.

In one particular embodiment, the polymer is a polymer obtained from ATBS (at least 10 mol% of which is advantageously in the crystalline form of ATBS.K) and at least one non-ionic monomer.

**Composition of the polymer**

The polymer is obtained from the crystalline form of ATBS.K, and advantageously from at least one other monomer which may be chosen from hydrophilic non-ionic monomers and/or hydrophilic anionic monomers and/or hydrophilic cationic monomers and/or hydrophilic zwitterionic monomers and/or hydrophobic monomers and mixtures thereof. It may be a polymer of several distinct monomers or a homopolymer.

Advantageously, the hydrophilic non-ionic monomer or monomers that can be used in the invention are chosen, in particular, from the group comprising water-soluble vinyl monomers such as acrylamide, methacrylamide, N-alkylacrylamides, N-alkylmethacrylamides, N,N-dialkyl acrylamides (e.g. N,N-dimethylacrylamide or N,N-diethylacrylamide), N,N-dialkylmethacrylamides, alkoxylated esters of acrylic acid, N-vinylpyrrolidone, N-methylol(meth)acrylamide, N-vinyl caprolactam, N-vinylformamide (NVF), N-vinyl acetamide, N-vinyl imidazole, N-vinyl succinimide, acryloyl morpholine (ACMO), glycidyl methacrylate, vinyl acetate, glyceryl methacrylate, diacetone acrylamide, methacrylic any hydride, acrylonitrile, maleic anydride, itaconamide, hydroxyalkyl (meth)acrylate, thioalkyl (meth)acrylate, isoprenol and its alkoxylated derivatives, hydroxyethyl (meth)acrylates and their alkoxylated derivatives, hydroxypropylacrylate and its alkoxylated derivatives, and mixtures thereof. Among non-ionic monomers, the alkyl groups are advantageously $C_1$-$C_5$, and more advantageously $C_1$-$C_3$. They are preferably linear alkyls. Preferably, the hydrophilic non-ionic monomer is acrylamide.

The polymer advantageously comprises between 0 and 99 mol% of hydrophilic non-ionic monomer(s), preferably between 40 and 98 mol%, and more preferably between 75 and 97 mol%.

Advantageously, apart from the the crystalline form of ATBS.K, the hydrophilic anionic monomer or monomers which can be used in the invention can be chosen from a large group. These monomers may have vinyl functions (advantageously acrylic, maleic, fumaric, malonic, itaconic, or allylic), and contain a carboxylate, phosphonate, phosphate, sulphate or sulphonate group, or another anionically charged group. Examples of suitable monomers include acrylic acid; methacrylic acid; dimethylacrylic acid; itaconic acid; C1-C3 hemiesters of itaconic acid; acryloyl chloride; crotonic acid; maleic acid; fumaric acid; 3-acrylamido-3-methylbutanoic acid; strong acid monomers with, for example, a sulfonic acid or phosphonic acid function, such as vinylsulfonic acid, vinylphosphonic acid, allylsulfonic acid, methallylsulfonic acid, 2-methylidenepropane-1,3-disulfonic acid, 2-sulfoethylmethacrylate, sulfopropylmethacrylate, sulfopropylacrylate, allylphosphonic acid, ethylene glycol methacrylate phosphate, 2-acrylamido-2-methylpropane sulfonic acid (ATBS), 2-acrylamido-2-methylpropane disulfonic acid, 3-allyloxy-2-hydroxypropane sulfonic acid, diethylallylphosphonate, carboxyethyl acrylate; water-soluble salts of these monomers such as their alkali metal salts (distinct from the crystalline form of ATBS.K), alkaline-earth metal salts or ammonium salts; and mixtures thereof. Preferably, the hydrophilic anionic monomer or monomers is acrylic acid and/or its salts.

The polymer advantageously comprises between 0 and 99 mol% of hydrophilic anionic monomer(s) (distinct from the crystalline form of ATBS.K), preferably between 5 and 70 mol%, more preferably between 10 and 50 mol%. Above 5 mol%, these percentages also include the monomer in crystalline form of ATBS.K according to the invention.

In one particular embodiment, the hydrophilic anionic monomer or monomers, apart from the crystalline form of ATBS.K, may be salified.

By salified, we mean the substitution of a proton of at least one acid function of the - $R^a$(=O)-OH type (with R representing P, S or C) of the anionic monomer by a metal or ammonium cation to form a salt of the -$R^a$(=O)-OX type (X being a metal cation or an organic cation). In other words, the non-salified form corresponds to the acid form of the monomer, for example $R^b$-C(=O)-OH in the case of the carboxylic acid function, while the salified form of the monomer corresponds to the $R^b$-C(=O)-$O^-$ $X^+$ form, $X^+$ corresponding to an alkaline cation or an organic cation. The salification of the acid functions of the branched water-soluble polymer can be partial or total. The salified form advantageously corresponds to the salts of alkali metals (Li, Na, K, etc.), alkaline-earth metals (Ca, Mg, etc.) or ammonium (for example the ammonium ion or a tertiary ammonium). The preferred salt is potassium salt.

The salification may take place before, during or after polymerization.

In one particular embodiment, the polymer advantageously comprises between 1 and 100 mol% of hydrophilic anionic monomer(s) in salified form, preferably between 50 and 100 mol%. These percentages include the monomer in crystalline form of ATBS.K according to the invention.

Advantageously, the hydrophilic cationic monomer or monomers that can be used in the invention are chosen from monomers derived from vinyl-type units (advantageously acrylamide, acrylic, allylic or maleic), these monomers having a phosphonium or quaternary ammonium function. Mention may be made, in particular and in non-limiting manner, diallyldialkyl ammonium salts such as diallyl dimethyl ammonium chloride (DADMAC); acidified or quaternized salts of dialkylaminoalkyl(meth)acrylamides, e.g. methacrylamido-propyl trimethyl ammonium chloride (MAPTAC), acrylamidopropyl trimethyl ammonium chloride (APTAC); acidified or quaternized salts of dialkylaminoalkyl acrylate, such as quaternized or salified dimethylaminoethyl acrylate (DMAEA); acidified or quaternized salts of dialkylaminoalkyl methacrylate, such as quaternized or salified dimethylaminoethyl methacrylate (DMAEMA); acidified or quaternized salts of N,N-dimethylallylamine; acidified or quaternized salts of diallylmethylamine; acidified or quaternized salts of diallylamine; vinylamine obtained by the hydrolysis (basic or acid) of an amide group -N($R_2$)-CO-$R_1$ with $R_1$ and $R_2$ being, independently, a hydrogen atom or an alkylated chain of 1 to 6 carbons, for example vinylamine obtained from the hydrolysis of vinylformamide; vinylamine obtained by Hofmann degradation; and mixtures thereof. Advantageously, the alkyl groups are $C_1$-$C_7$, preferably $C_1$-$C_3$, and can be linear, cyclic, saturated or unsaturated chains. Preferably quaternized dimethylaminoethyl acrylate.

A person skilled in the art knows how to prepare quaternized monomers, for example using a quaternizing agent of the R-X type, where R is an alkyl group and X is a halogen or sulfate.

The term "quaternizing agent" refers to a molecule capable of alkylating a tertiary amine.

The quaternizing agent may be chosen from dialkyl sulfates containing from 1 to 6 carbon atoms or alkyl halides containing from 1 to 6 carbon atoms. Preferably, the quaternizing agent is chosen from methyl chloride, benzyl chloride, dimethyl sulfate or diethyl sulfate. In addition, the present invention also covers DADMAC, APTAC and MAPTAC monomers whose counterion is a sulfate, fluoride, bromide or iodide instead of chloride.

The polymer advantageously comprises between 0 and 20 mol% hydrophilic cationic monomer(s), and preferably between 0 and 6 mol%.

Advantageously, the hydrophilic zwitterionic monomer or monomers may be a derivative of a vinyl-type unit (advantageously acrylamide, acrylic, allylic or maleic), this monomer having a quaternary amine or ammonium function and a carboxylic (or carboxylate), sulphonic (or sulphonate) or phosphoric (or phosphate) acid function. Mention may be made, in particular and in a non-limiting manner, of dimethylaminoethyl acrylate derivatives, such as 2-((2-(acryloyloxy) ethyl) dimethylammonio) dimethylammonio) ethane-1-sulphonate, 3-((2-(acryloyloxy)ethyl) dimethylammonio) propane-1-sulphonate, 4-((2-(acryloyloxy)ethyl) dimethylammonio) butane-1-sulphonate, [2-(acryloyloxy)ethyl] (dimethylammonio) acetate, dimethylaminoethyl methacrylate derivatives, such as 2-((2-(methacryloyloxy) ethyl) dimethylammonio) ethane-1-sulphonate, 3-((2-(methacryloyloxy) ethyl) dimethylammonio) propane-1-sulphonate, 4-((2-(methacryloyloxy) ethyl) dimethylammonio) butane-1-sulphonate, [2-(methacryloyloxy)ethyl] (dimethylammonio) acetate, dimethylamino propylacrylamide derivatives, such as 2-((3-acrylamidopropyl) dimethylammonio) ethane-1-sulphonate, 3-((3-acrylamidopropyl) dimethylammonio) propane-1-sulphonate, 4-((3-acrylamidopropyl) dimethylammonio) butane-1-sulphonate, [3-(acryloyloxy) propyl] (dimethylammonio) acetate, dimethylamino propyl methylacrylamide derivatives such as 2-((3-methacrylamidopropyl) dimethylammonio) ethane-1-sulphonate, 3-((3-methacrylamidopropyl) dimethylammonio) propane-1-sulphonate, 4-((3-methacrylamidopropyl) dimethylammonio) butane-1-sulphonate and [3-(methacryloyloxy)propyl] (dimethylammonio) acetate and mixtures thereof.

Other hydrophilic zwitterionic monomers can be used, in particular those described by the Applicant in document WO2021/123599.

The polymer advantageously comprises between 0 and 20 mol% hydrophilic zwitterionic monomer(s), more preferably between 0 and 10 mol%.

Hydrophobic monomers with a coefficient partition $K_{ow}$ greater than 1 can also be used in the preparation of the polymer. They are preferably chosen from the following list: :: (meth)acrylic acid esters with a (i) $C_4$-$C_{30}$ alkyl, or (ii) arylalkyl ($C_4$-$C_{30}$ alkyl, $C_4$-$C_{30}$ aryl), or (iii) propoxylated, or (iv) ethoxylated, or (v) ethoxylated and propoxylated chain; alkyl aryl sulfonates ($C_4$-$C_{30}$ alkyl, $C_4$-$C_{30}$ aryl) ; mono- or di-substituted (meth)acrylamide amides bearing a (i) $C_4$-$C_{30}$ alkyl, or (ii) arylalkyl ($C_4$-$C_{30}$ alkyl, $C_4$-$C_{30}$ aryl), or (iii) propoxylated, or (iv) ethoxylated, or (v) ethoxylated and propoxylated chain; anionic or cationic monomer derivatives of (meth)acrylamide or (meth)acrylic acid bearing a hydrophobic chain; vinylpyridine and mixtures thereof. The hydrophobic monomers may comprise halogen atoms, for example chlorine.

Among these hydrophobic monomers:

- alkyl groups are preferably $C_4$-$C_{20}$, more preferably $C_4$-$C_8$. The $C_6$-$C_{20}$ alkyls are preferably linear, while the $C_4$-$C_5$ alkyls are preferably branched,
- arylalkyl groups are preferably $C_7$-$C_{25}$, more preferably $C_7$-$C_{15}$,
- the ethoxylated chains advantageously comprise between 1 and 200 $-CH_2$-$CH_2$-O-groups, preferably between 6 and 100, more preferably between 10 and 40,
- the propoxylated chains advantageously comprise between 1 and 50 $-CH_2$-$CH_2$-$CH_2$-O-groups, more preferably between 1 and 20.

Preferred hydrophobic monomers belonging to these classes are, for example:

- n-hexyl (meth)acrylate, n-octyl (meth)acrylate, octyl (meth)acrylamide, lauryl (meth)acrylate, lauryl (meth)acrylamide, myristyl (meth)acrylate, myristyl (meth)acrylamide, pentadecyl (meth)acrylate, pentadecyl (meth)acrylamide, cetyl (meth)acrylate, cetyl (meth)acrylamide, oleyl (meth)acrylate, oleyl (meth)acrylamide, erucyl (meth)acrylate, erucyl (meth)acrylamide, N-tert-Butyl (meth)acrylamide, 2-ethylhexyl acrylate, $C_4$-$C_{22}$ itaconic acid hemiesters, acidified or quaternized salts of $C_4$-$C_{22}$ dialkylaminoalkyl (meth)acrylate, acidified or quaternized salts of $C_4$-$C_{22}$ dialkylaminoalkyl (meth)acrylamides, vinylpyridine, acrylamido undecanoic acid, and mixtures thereof,
- cationic allyl derivatives of formula (I) or (II):

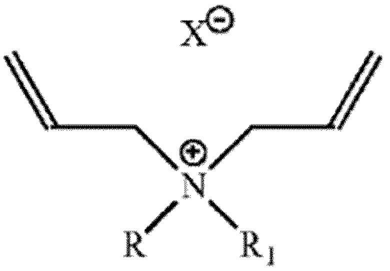

(I)

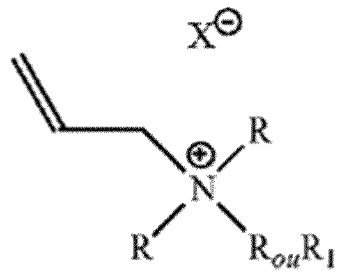

(II)

in which:

R: independently an alkyl chain containing 1 to 4 carbons;
$R_1$: an alkyl or arylalkyl chain containing 8 to 30 carbons;
X: a halide selected from the group consisting of bromides, chlorides, iodides, fluorides and any negatively charged counterion;
and, preferably, hydrophobic cationic derivatives of the (meth)acryloyl type corresponding to formula (III):

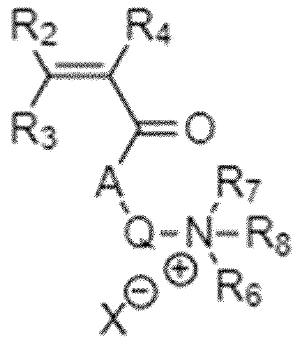

(III)

in which:

- A represents O or N-$R_5$ (preferably A represents N-$R_5$),
- $R_2$, $R_3$, $R_4$, $R_5$, $R_6$, $R_7$: independently hydrogen or an alkyl chain containing 1 to 4 carbons,
- Q: an alkyl chain containing 1 to 20 carbons,
- $R_8$: an alkyl or arylalkyl chain containing 8 to 30 carbons,
- X: a halide selected from the group consisting of bromides, chlorides, iodides, fluorides, and any negatively charged counterion.

When the polymer is water soluble, it advantageously comprises less than 5 mol% of hydrophobic monomers and the quantity thereof is adjusted in order for the polymer to remain soluble in water.

Monomers having a fluorescent function can also be used in the invention. A monomer with a fluorescent function may be detected by any suitable method, for example by fluorometry with a fixed wavelength fluorometer. Generally, the monomer with a fluorescent function is detected at the excitation and emission maxima, which can be determined using a scanning fluorometer.

Monomers with a fluorescent function are chosen, for example, from the following monomers: sodium or potassium styrene sulfonate, styrene sulfonic acid, vinylimidazole and its derivatives, 9-vinyl anthracene and its derivatives, N-9-xanthenylacrylamide and its derivatives, allyl dibenzosuberenol and its derivatives, chinconicin and its derivatives, quininone and its derivatives, cinchoninone and its derivatives, N,N-dimethyl-N-[3-[N'-(4-methoxy naphthalimide)]]propyl-N-(2-hydroxy-3-allyloxy)propyl ammonium hydroxide and mixtures thereof.

Other fluorescent compounds can be used when functionalized with an allyl, vinyl or acrylic double bond, such as pyranine and its derivatives, coumarin and its derivatives, quinolaxine and its derivatives, pinacyanol and its derivatives, xanthydrol and its derivatives, dabsyl and its derivatives, 3-hydroxy-2-methylene-3-(1-naphthyl)propionic acid and its derivatives, rhodamine and its derivatives, N-dibenzosuberylacrylamide and its derivatives, naphthalic derivatives,

fluorescein and its derivatives, pyrene and its derivatives, carbostyril and its derivatives, pyrazoline and its derivatives and mixtures thereof.

In a preferred mode, the polymer does not comprise a monomer with a fluorescent function.

In one particular embodiment, the polymer may comprise at least one cyclic monomer with a hydrolyzable function. Advantageously, the or the cyclic monomers with a hydrolyzable function are chosen from cyclic ketene acetals, thionolactones and mixtures thereof.

The cyclic ketene acetal is advantageously chosen from: 2-methylene-1,3-dioxepane (MDO), 5,6-benzo-2-methylene-1,3-dioxepane (BMDO), 2-methylene-4-phenyl-1,3-dioxolane (MPDL), 2-methylene-1,3,6-trioxocane (MTC), and mixtures thereof. Preferably, it is 2-methylene-1,3-dioxepane (MDO).

The thionolactone is advantageously chosen from: dibenzo[c,e]oxepine(7H)-5-thione (DOT), ε-thionocaprolactone, 3,3-dimethyl-2,3-dihydro-5H-benzo[e][1,4]dioxepine-5-thione (DBT) and mixtures thereof. Preferably, it is 3,3-dimethyl-2,3-dihydro-5Hbenzo[e][1,4]dioxepine-5-thione.

In one particular embodiment, the polymer may comprise at least one group with an LCST. According to the general knowledge of a person skilled in the art, a group with an LCST corresponds to a group whose water solubility, for a given concentration, is modified above a certain temperature and as a function of salinity. It is a group with a heating transition temperature that defines its lack of affinity with the solvent medium. The lack of affinity with the solvent results in opacification or loss of transparency, which may be due to precipitation, aggregation, gelation or viscosification of the medium. The minimum transition temperature is known as the LCST (Lower Critical Solution Temperature). For each concentration a group with an LCST, a heating transition temperature is observed. It is higher than the LCST, which is the minimum point on the curve. Below this temperature, the polymer is soluble in water; above this temperature, the polymer loses its solubility in water.

In one particular embodiment, the polymer may comprise at least group with a UCST.

According to the general knowledge of a person skilled in the art, a group with a UCST corresponds to a group whose water solubility, for a given concentration, is modified below a certain temperature and as a function of salinity. It is a group with a cooling transition temperature that defines its lack of affinity with the solvent medium. The lack of affinity with the solvent results in opacification or loss of transparency, which may be due to precipitation, aggregation, gelation or viscosification of the medium. The maximum transition temperature is known as the UCST (Upper Critical Solution Temperature). For each concentration a group with a UCST, a cooling transition temperature is observed. It is lower than the UCST, which is the maximum point on the curve. Above this temperature, the polymer is soluble in water; below this temperature, the polymer loses its solubility in water.

The quantities of the different monomer(s) will be adjusted by a person skilled in the art in order not to exceed 100 mol% when preparing the polymer.

In some embodiments, the polymer may have a linear, branched, cross-linked, star-shaped or comb-shaped structure. This structure can be obtained, according to the general knowledge of a person skilled in the art, for example by selecting the initiator, the transfer agent, the polymerization technique such as Reversible Addition Fragmentation chain Transfer (RAFT) polymerization, Nitroxide Mediated Polymerization (NMP) or Atom Transfer Radical Polymerization (ATRP), the incorporation of structural monomers, or the concentration.

The polymer may further be structured by a branching agent. A structured polymer is a non-linear polymer that has side chains such that, when dissolved in water, the polymer has a high degree of entanglement leading to very high low-gradient viscosities.

The branching agent is advantageously chosen from:

- structural agents, which can be chosen from the group comprising polyethylenically unsaturated monomers (having at least two unsaturated functions), such as vinyl functions, in particular allyl or acrylic functions, and we can cite for example methylene bis acrylamide (MBA), triallyamine, or tetraallylammonium chloride or 1,2 dihydroxyethylene bis-(N-acrylamide),
- monomers with at least two epoxy functions,
- monomers with at least one unsaturated and one epoxy function,
- macroinitiators such as polyperoxides, polyazoids and transfer agents such as polymercaptant polymers, and polyols,
- functionalized polysaccharides,
- water-soluble metal complexes composed of:

    * a metal with a valence greater than 3, such as, by way of example and non-limitation, aluminum, boron, zirconium or titanium, and
    * a ligand bearing a hydroxyl function.

The quantity of branching agent in the polymer is advantageously less than 40,000 ppm by weight relative to the

total weight of the monomers of the polymer, preferably less than 10,000 ppm by weight, and more preferably less than 5,000 ppm by weight.

In one particular embodiment, the quantity of branching agent is at least equal to 0.1 ppm by weight relative to the total weight of the monomers of the polymer, preferably at least 1 ppm by weight, more preferably at least 10 ppm by weight, more preferably at least 100 ppm by weight and even more preferably at least 1 000 ppm by weight.

When the polymer is water soluble and comprises a branching agent, the polymer may remain soluble in water. A person skilled in the art knows how to adjust the quantity of branching agent and, possibly, the quantity of transfer agent needed to obtain this result.

In a preferred embodiment, the polymer is a water-soluble polymer comprising no branching agent.

In one particular embodiment, the polymer may comprise a transfer agent.

The transfer agent is advantageously chosen from methanol; isopropyl alcohol; sodium hypophosphite; calcium hypophosphite; magnesium hypophosphite; potassium hypophosphite; ammonium hypophosphite; formic acid; sodium formate; calcium formate; magnesium formate; potassium formate; ammonium formate; 2-mercaptoethanol; 3-mercaptopropanol; dithiopropylene glycol; thioglycerol; thioglycolic acid; thiohydracrylic acid; thiolactic acid; thiomalic acid; cysteine; aminoethanethiol; thioglycolates; allyl phosphites; allyl mercaptans, such as n-dodecyl mercaptan; sodium methallysulfonate; calcium methallysulfonate; magnesium methallysulfonate; potassium methallysulfonate; ammonium methallysulfonate; alkyl phosphites such as trialkyl ($C_{12}$-$C_{15}$) phosphites, di-oleyl-hydrogen phosphites, dibutyl phosphite; dialkyldithiophosphates such as dioctyl phosphonate; tertiary nonylmercaptan; 2-ethylhexyl thioglycolate; n-octyl mercaptan; n-dodecyl mercaptan; tertiary-dodecyl mercaptan; iso-octylthioglycolate; 2-ethylhexyl thioglycolate; 2-ethylhexyl mercaptoacetate; polythiols; and mixtures thereof. Preferably sodium hypophosphite or sodium formate.

The quantity of transfer agent in the polymer is advantageously between 0 and 100,000 ppm by weight relative to the total weight of the monomers of the polymer, preferably between 0 and 10,000 ppm by weight, more preferably between 0 and 1,000 ppm by weight, and even more preferably between 0 and 100 ppm by weight. When it is present, the transfer agent represents at least 0.1 ppm by weight relative to the total weight of the monomers of the polymer, and preferably at least 1 ppm by weight.

In one particular embodiment, the polymer comprises no transfer agent.

Generally speaking, the polymer does not require the development of any particular polymerization method. Indeed, it may be obtained using any of the polymerization techniques that are well known to a person skilled in the art. These include solution polymerization; gel polymerization; precipitation polymerization; emulsion polymerization (aqueous or inverse); suspension polymerization; reactive extrusion polymerization; water-in-water polymerization; or micellar polymerization.

The polymerization is generally radical polymerization, preferably by inverse emulsion polymerization or gel polymerization. Radical polymerization includes free radical polymerization using UV, azo, redox or thermal initiators, as well as controlled radical polymerization (CRP) or matrix polymerization techniques.

Controlled radical polymerization techniques include, but are not limited to, techniques such as Iodine Transfer Polymerization (ITP), Nitroxide Mediated Polymerization (NMP), Atom Transfer Radical Polymerization (ATRP), Reversible Addition Fragmentation chain Transfer (RAFT) Polymerization, which includes MADIX (MAcromolecular Design by Interchange of Xanthates) technology, various variations of Organometallic Mediated Radical Polymerization (OMRP), and OrganoHeteroatom-mediated Radical Polymerization (OHRP).

The polymer may be partially or totally post-hydrolyzed.

Post-hydrolysis is the hydrolysis reaction of the polymer after it has been formed by polymerization of the monomer(s). This step consists in reacting hydrolysable functional groups of monomers, advantageously non-ionic functional groups, more advantageously amide or ester functional groups, with a hydrolysis agent. This hydrolysis agent may, for example, be an enzyme, an ion-exchange resin, or a Brønsted acid (for example a hydrohalogenic acid) or a Brønsted base (for example an alkali hydroxide or an alkaline-earth hydroxide). Preferably, the hydrolysis agent is a Brønsted base. During this step of post-hydrolyzing the polymer, the number of carboxylic acid functions increases. Indeed, the reaction between the base and the amide or ester functions present in the polymer produces carboxylate groups.

The polymer may be in liquid, gel or solid form when its preparation includes a drying step such as spray drying, drum drying, radiation drying such as microwave drying, or drying in a fluidized bed.

The polymer advantageously has a molecular weight of at least 0.5 million g/mol, preferably between 0.5 and 40 million g/mol, more preferably between 5 and 30 million g/mol. Molecular weight is defined as weight-average molecular weight. The polymer may also have a molecular weight of between 5,000 and 100,000 g/mol or between 100,000 and 500,000 g/mol.

The molecular weight is determined by the intrinsic viscosity of the polymer. The intrinsic viscosity can be measured by methods known to a person skilled in the art and can be calculated from the reduced viscosity values for different polymer concentrations by a graphical method consisting in plotting the reduced viscosity values (y-axis) against the concentration (x-axis) and extrapolating the curve to zero concentration. The intrinsic viscosity value is plotted on the y-axis or using the least-squares method. The molecular weight can then be determined using the Mark-Houwink equation:

$$[\eta] = K.M^{\alpha}$$

where [n] represents the intrinsic viscosity of the polymer as determined by the solution viscosity method.
K represents an empirical constant.
M represents the molecular weight of the polymer.
$\alpha$ represents the Mark-Houwink coefficient.
K and $\alpha$ depend on the particular polymer-solvent system.

**Properties of the polymer**

The polymer, when it is water soluble, advantageously has a filter ratio referred to as FR of less than 1.5, preferably less than 1.3 and more preferably less than 1.1.

The term "filter ratio" is used in the present document to denote a test used to determine the performance of the polymer solution under conditions resembling the permeability of the deposit, which consists in measuring the time taken by given volumes/concentrations of solution to pass through a filter. The FR generally compares the filterability of the polymer solution for two consecutive equivalent volumes, which indicates the tendency of the solution to clog the filter. Lower FR's indicate better performance.

The test used to determine the FR involves measuring the time taken for given volumes of 1000 ppm (by weight) polymer solution to flow through a filter. The solution is contained in a cell pressurized to two bars and the filter is 47 mm in diameter and has a defined pore size. The FR is typically measured using filters with a pore size of 1.2 $\mu$m, 3 $\mu$m, 5 $\mu$m or 10 $\mu$m.

The times needed to obtain 100 ml ($t_{100ml}$); 200 ml ($t_{200ml}$) and 300 ml ($t_{300ml}$) of filtrate are measured, and an FR is then defined as:

[Math 2]

$$FR = \frac{t_{300\ ml} - t_{200\ ml}}{t_{200\ ml} - t_{100\ ml}}$$

Times are measured to the nearest 0.1 second.

The FR thus represents the capacity of the polymer solution to clog the filter for two consecutive equivalent volumes.

The polymers have improved resistance to chemical and thermal degradation compared with polymers of equivalent molecular weight obtained from ATBS which is not in the crystalline form of ATBS.K.

The test used to determine resistance to chemical degradation involves preparing a solution of polymer at a given concentration in a given brine under aerobic conditions and bringing it into contact with a chemical contaminant such as iron or hydrogen sulphide. The viscosity of the polymer solution is measured before and after 24 hours of exposure to the contaminant. The viscosity measurements are carried out under the same temperature and shear gradient conditions.

The test used to determine resistance to mechanical degradation involves preparing a solution of polymer at a given concentration in a brine of given composition under anaerobic conditions (using an inert glove box, inert with nitrogen, for example) and allowing it to age in a stainless steel cell set to a given temperature for a predefined time. The stainless steel cell is then cooled to room temperature and the viscosity of the polymer solution it contains is measured and compared with its initial value. All handling of the stainless steel cell is carried out inside the glove box to avoid exposure to oxygen. The stainless steel cells are sealed to prevent oxygen from entering the solution during heat ageing. The viscosity measurements before and after ageing are carried out in a glove box under the same temperature and speed gradient conditions.

Resistance to chemical and thermal degradation is quantified by the viscosity loss value expressed in percent and determined at the end of the test by:

[Math 3]

$$Loss\ of\ viscosity\ (\%) = \frac{Viscosity_{inital} - Viscosity_{end}}{Viscosity_{initial}} \text{ x } 100$$

**Method for treating a suspension of solid particles in water**

The Applicant has surprisingly discovered that using a water-soluble polymer obtained from the crystalline form of ATBS.K improves the performances of suspension treatments, which include:

- increasing sludge concentration at the outlet of a thickener,
- the dehydration step and the steps of drying and solidifying the suspensions when they are discharged onto the ground, and
- mechanically treating the treated suspensions.

In some embodiments, a method for treating a suspension of solid particles in water, comprises bringing said suspension into contact with at least one water-soluble polymer, said polymer being obtained from the crystalline form of ATBS.K. This method therefore involves mixing said suspension with the water-soluble polymer.

Such a treatment may be carried out in a thickener, which is a retention zone generally in the form of a section of tube several metres in diameter with a conical bottom in which particles can settle. According to one specific embodiment, the aqueous suspension is transported to a thickener by means of a pipe (pipeline), and the water-soluble polymer is added into the pipe.

In one particular embodiment, the water-soluble polymer is added into a thickener that already contains the suspension to be treated. In one typical mineral treatment operation, the suspensions are often concentrated in a thickener. This results in a higher density sludge, which exits at the bottom of the thickener, and an aqueous fluid released from the treated suspension (called liquor), which exits by overflow at the top of the thickener. Adding the water-soluble polymer increases the concentration of the sludge and increases the clarity of the liquor.

In one particular embodiment, the water-soluble polymer is added to the suspension of particles while said suspension is being transported through a pipe to a deposition zone. The water-soluble polymer is preferably added into the pipe which transports said suspension to a deposition zone. The treated suspension is spread over this deposition zone to be dehydrated and solidified. The deposition zones may be unenclosed, being an undefined area of ground, for example, or enclosed, for example being a basin or cell.

One example of these treatments while the suspension is being transported is the spreading of the suspension treated with the water-soluble polymer on the ground in order to dehydrate and solidify it, followed by the spreading of a second layer of treated suspension on the first solidified layer.

Another example is the continuous spreading of the suspension treated with the water-soluble polymer in such a way that the treated suspension falls continuously onto the suspension previously discharged into the deposition zone, thus forming a mass of treated material from which the water is extracted.

In one particular embodiment, the water-soluble polymer is added to the suspension, after which mechanical treatment such as centrifugation, pressing or filtration is carried out. The water-soluble polymer can be added simultaneously at different stages of the suspension treatment process, i.e., for example, into the pipe (pipeline) transporting the suspension to a thickener and into the slurry leaving the thickener, which will be conveyed either to a deposition zone or to a mechanical treatment device.

The water-soluble polymer may be added in liquid form or in solid form to the aqueous suspension to be treated. It can be added in the form of an emulsion (preferably a water-in-oil emulsion), an aqueous or oily multiphase particulate suspension or a powder. The water-soluble polymer is preferably added in the form of an aqueous solution obtained from a concentrated form of the water-soluble polymer such as a powder, a water-in-oil emulsion or an aqueous or oily multiphase particulate suspension.

In one particular embodiment, the aqueous multiphase particulate suspension preferably comprises:

- 15 to 60% by mass of at least one water-soluble polymer in the form of solid particles with an average size of between 5 and 500 μm;
- 15 to 45% by mass of at least one alkali metal salt and/or at least one alkaline-earth metal salt;
- at least one viscosifying agent other than the water-soluble polymer;
- at least 10 % by mass of water; and

said suspension having a Brookfield viscosity of between 500 and 20,000 cps (0.5 Pa.s to 20 Pa.s) at a temperature of

20°C; and
said suspension having a density of between 1.1 and 2 $kg.L^{-1}$.

In one particular embodiment, the oily multiphase particulate suspension preferably comprises:

- 15 to 60% by mass of at least one water-soluble polymer in the form of solid particles with an average size of between 5 and 500 μm;
- at least one viscosifying agent other than the water-soluble polymer;
- at least 10% by mass of oil; and

said suspension having a Brookfield viscosity of between 500 and 20,000 cps (0.5 Pa.s to 20 Pa.s) at a temperature of 20°C; and
said suspension having a density of between 0.6 and 1.4 $kg.L^{-1}$.

Brookfield viscosity is measured with a Brookfield apparatus, fitted with an LV module, the module being able to rotate at a speed of 30 rpm, for example, the measurement being advantageously carried out at 20°C. The density is measured at 20°C, at a pressure of 1 atm, i.e., 101,325 Pa.
When the water-soluble polymer is in solid form, it can be partially or totally dissolved in water using a polymer preparation unit such as the Polymer Slicing Unit (PSU) disclosed in EP 2 203 245.
In one particular embodiment, the water-soluble polymer is added to the suspension in combination with at least one other synthetic or natural polymer. These polymers may be added simultaneously or separately (before or after the water-soluble polymer has been added). The other polymer may be water soluble or water swelling. It may be a dispersant, a coagulant or a flocculant.
In one particular embodiment, the water-soluble polymer is added to the suspension in combination with a salt such as a calcium and/or magnesium salt. The water-soluble polymer and the salt may be added simultaneously or separately. The salts may be inorganic or organic. Suitable salts include calcium chloride, calcium acetate, calcium sulphate, calcium nitrate, calcium hydroxide, calcium carbonate, magnesium chloride, magnesium acetate, magnesium sulphate, magnesium nitrate, magnesium hydroxide, magnesium carbonate, calcium formate, calcium gluconate, calcium propionate, tricalcium phosphate and calcium succinate.
The quantity of water-soluble polymer added to the aqueous suspension is advantageously between 50 and 5,000 g per tonne of solid particles, by dry weight, of the suspension, preferably between 250 and 2000 g/t, and more preferably between 500 and 1500 g/t. The quantity depends on the nature and the composition of the suspensions to be treated. A person skilled in the art knows how to adjust this quantity and would consider such an adjustment to be a matter of routine.
In some embodiments, the method can be used to effectively treat a suspension of solid particles and, more particularly, mineral particles.
The suspensions of solid particles in water comprise all types of sludge, residues or waste materials. The suspensions result, more particularly, from mineral extraction and are in the form of mineral particle suspensions. They may, for example, be industrial sludges or residues and all the washing and waste products from mining operations, such as coal mines, diamond mines, phosphate mines, metal mines (aluminium, platinum, iron, gold, copper, silver, etc.). The suspensions can also come from the extraction of oil sand, for example sludge or extraction residues derived from the processing of oil sand. These suspensions generally comprise organic and/or mineral particles, such as clays, sediments, sand, metal oxides, oil, etc., for example, mixed with water.
Generally, the suspensions of solid particles are concentrated and contain between 5% and 60% by weight of solid particles, and preferably between 20 and 50% by weight of solid particles, relative to the total weight of said suspensions.
The method may also be useful for treating residues from oil sand extraction, which are referred to as "fines" or "fine tailings", i.e., containing a large quantity of clays, and for treating fine residues known as Mature Fine Tailings (MFT), i.e., these same fine residues after a few years of sedimentation, and containing an even greater quantity of clays. The method may also be used to treat so-called "fresh" residues, i.e., those resulting directly from the operation of separating bitumen from the soil from which it (the bitumen) is extracted.

**Method for flocculating a suspension of solid particles in water**

In some embodiments, a method for flocculating a suspension of solid particles in water, comprises bringing said suspension into contact with at least one water-soluble polymer obtained from the crystalline form of ATBS.K having an X-ray powder diffraction pattern comprising peaks at 13.1°; 14.4°; 16.3°; 19.8°; 23.5°; 24.3°; 26.9°; 27.6°; 29.3°; 30.6°; 31.6°; 34.3°; 36.1°; 41.7°; 44.6°; 46.7° 2 theta (+/- 0.1°).

All of the embodiments described above concerning the method for treating a suspension of solid particles in water are also applicable to the method for flocculating a suspension of solid particles in water.

**Method for enhanced hydrocarbon (oil and/or gas) recovery**

In some embodiments, a method for enhanced hydrocarbon (oil and/or gas) recovery comprises the following steps:

a) Preparing an injection fluid comprising at least one water-soluble polymer of 2-acrylamido-2-methylpropanesulphonic acid, with water or brine;
the 2-acrylamido-2-methylpropanesulphonic acid being, prior to polymerization, in the crystalline form of ATBS.K having an X-ray powder diffraction pattern comprising peaks at 13.1°; 14.4°; 16.3°; 19.8°; 23.5°; 24.3°; 26.9°; 27.6°; 29.3°; 30.6°; 31.6°; 34.3°; 36.1°; 41.7°; 44.6°; 46.7° 2 theta (+/- 0.1°);
b) Injecting the injection fluid into a subterranean formation;
c) Sweeping the subterranean formation using the injected fluid;
d) Recovering an aqueous hydrocarbon mixture.

When the water-soluble polymer implemented in the preparation of the injection fluid is in the form of powder, the average size of the particles of water-soluble polymer is advantageously less than 1.5 millimetres, and preferably less than 850 micrometres. The average size of the particles of water-soluble polymer is advantageously greater than 5 μm.

The average size of the particles of water-soluble polymer is the average size of the largest dimension, for example the diameter in the case of spherical particles. It is advantageously measured using a laser measuring device using conventional techniques that are part of the knowledge of a person skilled in the art. For example, a Malvern Mastersizer, the MS2000, for example, could be used for this purpose. An apparatus of this type can be used to measure, by laser diffraction, the particle-size distribution of particles in a liquid medium or in solid form.

When the water-soluble polymer is in the form of particles, it may be dissolved in an aqueous medium in a dispersion device. The Polymer Slicing Unit (PSU) described in document US 8,186,871 is one example of a dispersion device that can be used to prepare a concentrated aqueous polymer solution.

The water or brine used to prepare the injection fluid may be production water. "Production water" refers to all saline or non-saline water, brine, seawater or aquifer water from a hydrocarbon reservoir. This production water may be treated before the injection fluid is prepared, as described in patent application WO 2018/020175.

The water-soluble polymers may be combined with stabilizing compounds. The stabilizing compounds (stabilizing agents) may be compounds that suitably protect the polymers, for example against thermal, chemical and/or mechanical degradation. Examples of suitable stabilizing agents are provided in patent application WO 2010/133258.

Depending on the techniques used, the injection fluid comprising the water-soluble polymer is injected on its own or in combination with one or more chemical compounds that can be used for enhanced hydrocarbon (oil and/or gas) recovery. These chemical compounds include the use of weak, strong or super-strong mineral or organic bases that can saponify crude oil and produce surface-active species in-situ that solubilize hydrocarbons, in particular oil. Examples include sodium or potassium carbonate, caustic soda, borate and metaborate compounds, amines and basic polymeric species. Another family of compounds commonly injected with polymers is that of surface-active compounds, which are often anionic, zwitterionic, cationic and sometimes non-ionic. These compounds are rarely injected pure, but generally with a co-surfactant and a cosolvent to improve their compatibility and effectiveness in the reservoir (subterranean formation).

The injection fluid advantageously comprises between 10 and 15,000 ppm by weight of water-soluble polymer, preferably between 50 and 10,000 ppm by weight, and more preferably between 100 and 5,000 ppm by weight.

Surprisingly the Applicant has discovered that the water-soluble polymers obtained from the crystalline form of ATBS.K have better filterability and better resistance to chemical and thermal degradation than polymers of equivalent molecular weight obtained from 2-acrylamido-2-methylpropanesulphonic acid that is not in the crystalline form of ATBS.K. It is also known that filterability deteriorates as the molecular weight of the polymer increases. One of the advantages of the invention lies in the possibility of obtaining water-soluble polymers of very high molecular weight which, at the same time, exhibit good filterability. In addition, the concentration of water-soluble polymer required to bring the injection fluid to a target viscosity is reduced, which improves the economic conditions for recovering the hydrocarbons (oil and/or gas) contained in the subterranean formation.

The purpose of the water-soluble polymers according to the invention is to viscosify the water injected into reservoirs (subterranean formations) containing hydrocarbons (oil and/or gas) in order to ensure mobility control without the need for cross-linking, i.e., chemical interchain bridging.

In one particular embodiment, the method for enhanced hydrocarbon (oil and/or gas) recovery comprises the following steps :

a) Preparing an injection fluid comprising a water-soluble polymer with a molecular weight greater than 5 million g/mol:

- the injection fluid having a salt concentration greater than 100 g/l, including, at most, 50 g/l of divalent salt(s);
- the polymer comprising at least 80% mol of 2-acrylamido-2-methylpropanesulphonic acid in which, advantageously, at least 50 mol% of the 2-acrylamido-2-methylpropanesulphonic acid, preferably at least 70 mol% and more preferably 100 mol% is, before polymerization, in the crystalline form of ATBS.K;
- the concentration of water-soluble polymer in the injection fluid being less than 3,000 ppm by weight;
- the injection fluid having a viscosity $V_1$ before the shearing step b);

b) Shearing the injection fluid in order to obtain a drop in viscosity of more than 25% relative to $V_1$, in which:

[Math 4]

$$\frac{V2 - V_{water}}{V_{water}} \geq 3$$

where,

$V_1$ is the viscosity of the injection fluid before the shearing step at the temperature of the formation;
$V_2$ is the viscosity of the injection fluid after the shearing step at the temperature of the formation;
$V_{water}$ is the viscosity of the water used to prepare the injection fluid at the temperature of the formation;

c) Injecting the injection fluid into a subterranean formation,
the subterranean formation being a carbonate formation with a permeability of less than 300 millidarcy and a temperature greater than 100°C;
c) Sweeping the subterranean formation using the injected fluid;
d) Recovering an aqueous hydrocarbon (oil and/or gas) mixture.

The shearing step may be carried out, for example, using a valve, a port or a pump.
Preferably, the concentration of divalent salt(s) in the injection fluid is between 3 and 50 g/l.
Carbonate formations are sedimentary rock formations with a carbonate composition of at least 50%.

**Fracturing fluid**

In some embodiments, a fracturing fluid comprises an aqueous phase, at least one propping agent and at least one water-soluble polymer obtained from 2-acrylamido-2-methylpropanesulphonic acid in the crystalline form of ATBS.K having an X-ray powder diffraction pattern comprising peaks at 13.1°; 14.4°; 16.3°; 19.8°; 23.5°; 24.3°; 26.9°; 27.6°; 29.3°; 30.6°; 31.6°; 34.3°; 36.1°; 41.7°; 44.6°; 46.7° 2 theta (+/- 0.1°).
The aqueous phase is advantageously chosen from sea water, brine and fresh water, and is advantageously a brine.
Brine is a solution comprising water and organic or inorganic salts. The salts may include monovalent salts, divalent salts, trivalent salts and mixtures thereof. Advantageously, the brine comprises at least 1,000 mg/L of salts, preferably at least 5,000 mg/L, more preferably at least 10,000 mg/L, even more preferably at least 50,000 mg/L and even more preferably the brine is saturated with salts.
The propping agent may be chosen from a non-restrictive list of sand, ceramics, bauxite, glass beads and resin-impregnated sand.
Advantageously, the quantity of propping agent in the fracturing fluid is between 0.5 and 40% by weight relative to the total weight of the fracturing fluid, preferably between 1 and 25%, and more preferably between 1.5 and 20%.
Advantageously, the fracturing fluid comprises between 0.001% and 1% by weight of water-soluble polymer obtained from the crystalline form of ATBS.K relative to the total weight of the fracturing fluid, and preferably between 0.002% and 0.2%.
The fracturing fluid may comprise other compounds known to a person skilled in the art, such as those cited in document SPE 152596, for example:

- Clay anti-swelling agents such as potassium chloride or choline chloride, and/or
- Biocides for preventing the development of bacteria, in particular sulphate-reducing bacteria, which can form viscous masses reducing the passage surfaces. Examples include glutaraldehyde, which is the most used, or indeed formaldehyde or the isothiazolinones, and/or
- Oxygen reducers such as ammonium bisulphite for preventing the destruction of the other components by oxidation and corrosion of the injection tubes, and/or
- Anti-corrosion additives for protecting the tubes from oxidation by residual quantities of oxygen, with N,N dimethylformamide being preferred, and/or
- Lubricants such as oil distillates, and/or
- Iron chelating agents such as citric acid, EDTA (ethylene diamine tetra-acetic) acid, phosphonates, and/or
- Antiscaling agents such as phosphates, phosphonates, polyacrylates or ethylene glycol.

Before being used in the fracturing fluid, the water-soluble polymer according to the invention may be in various solid or liquid forms. Preferably, it is in the form of powder, a water-in-oil inverse emulsion, an aqueous multiphase particulate suspension, or an oily multiphase particulate suspension.

**Method for preparing a fracturing fluid**

In some embodiments, the at least one water-soluble polymer obtained from 2-acrylamido-2-methylpropanesulphonic acid in the crystalline form of ATBS.K (described above) is added, to water or brine, in a method for preparing a fracturing fluid in which the water-soluble polymer is, before the formation of the fracturing fluid:

- either in the form of powder;
- or in the form of a water-in-oil inverse emulsion;
- or in the form of an aqueous or oily multiphase particulate suspension.

The method for preparing a fracturing fluid preferably comprises a step of adding at least one propping agent to said fluid as previously described.

When the water-soluble polymer added to the fracturing fluid is, before the formation of the fracturing fluid, in the form of powder, the average size of the polymer particles is advantageously less than 1.5 millimetres, preferably less than 850 micrometres, and more preferably less than 200 micrometres. The average size of the particles of water-soluble polymer is advantageously greater than 5 μm.

The average size of the particles of water-soluble polymer is the average size of the largest dimension, for example the diameter in the case of spherical particles. It is advantageously measured using a laser measuring device using conventional techniques that are part of the knowledge of a person skilled in the art. For example, a Malvern Mastersizer, the MS2000, for example, could be used for this purpose. An apparatus of this type can be used to measure, by laser diffraction, the particle-size distribution of particles in a liquid medium or in solid form.

When the water-soluble polymer is in solid form, it can be partially or totally dissolved in water using a polymer preparation unit such as the Polymer Slicing Unit (PSU) disclosed in EP 2 203 245.

When the water-soluble polymer added to the fracturing fluid is, before the formation of the fracturing fluid, in the form of a water-in-oil inverse emulsion, the concentration of water-soluble polymer in the emulsion is preferably between 5 and 60% by weight and more preferably between 15 and 40% by weight relative to the weight of the emulsion.

In a preferred embodiment, the water-in-oil inverse emulsion may comprise between 0.01% and 70% by weight of an organic and/or inorganic salt relative to the weight of the emulsion, and preferably between 5 and 20%. The salts may be chosen from a non-restrictive list of sodium chloride, sodium sulphate, sodium bromide, ammonium sulphate, ammonium chloride, lithium chloride, lithium bromide, potassium chloride, potassium bromide, magnesium sulphate, aluminium sulphate and mixtures thereof. The preferred salts are ammonium chloride and the ammonium sulphate.

When the water-soluble polymer added to the fracturing fluid is, before the formation of the fracturing fluid, in the form of an aqueous multiphase particulate suspension, said suspension preferably comprises:

- 15 to 60% by weight of at least one water-soluble polymer in the form of solid particles with an average size of between 5 and 500 μm;
- 15 to 45% by weight of at least one alkali metal salt and/or at least one alkaline-earth metal salt;
- at least one viscosifying agent other than the water-soluble polymer;
- at least 10% by weight of water; and

said suspension having a Brookfield viscosity of between 500 and 20,000 cps (0.5 Pa.s to 20 Pa.s) at a temperature of 20°C; and

said suspension having a density of between 1.1 and 2 kg.L$^{-1}$.

When the water-soluble polymer added to the fracturing fluid is, before the formation of the fracturing fluid, in the form of an oily multiphase particulate suspension, said suspension preferably comprises:

- 15 to 60% by weight of at least one water-soluble polymer in the form of solid particles with an average size of between 5 and 500 μm;
- at least one viscosifying agent other than the water-soluble polymer;
- at least 10% by weight of oil; and

said suspension having a Brookfield viscosity of between 500 and 20,000 cps (0.5 Pa.s to 20 Pa.s) at a temperature of 20°C; and
said suspension having a density of between 0.6 and 1.4 kg.L$^{-1}$.

Brookfield viscosity is measured with a Brookfield apparatus, fitted with an LV module, the module being able to rotate at a speed of 30 rpm, for example, the measurement being advantageously carried out at 20°C. The density is measured at 20°C, at a pressure of 1 atm, i.e., 101,325 Pa.

**Method for the hydraulic fracturing of an unconventional underground oil or gas reservoir**

In some embodiments, a method for the hydraulic fracturing of an unconventional underground oil or gas reservoir comprises preparing a fracturing fluid as previously described, and injecting said fracturing fluid into the underground reservoir.

Injection is carried out under pressure in order to create fractures distributed along the entire length of the production well.

Optionally, before, during or after the creation of the fractures, at least one oxidizing compound and/or at least one surface-active compound is injected into the reservoir.

Injecting a surface-active compound helps eliminate the viscosity generated by the polymer by inhibiting hydrophobic interchain interactions, while injecting the oxidising compound destroys the polymer. In both cases, injection allows a fluid viscosity close to that of water to be re-established.

Oxidizing compounds include bleach (an aqueous solution of a hypochlorite salt), hydrogen peroxide, ozone, chloramines, persulphates, permanganates and perchlorates.

The chemical nature of the surface-active compound(s) is not critical. They can be anionic, non-ionic, amphoteric, zwitterionic and/or cationic. The surface-active compound(s) of the invention preferably carry anionic charges.

Preferably, the surface-active compounds used are chosen from anionic surfactants and their zwitterions chosen from the group comprising derivatives of alkylsulphates, alkyl ether sulphates, arylalkylsulphates, arylalkylether sulphates, alkylsulphonates, alkyl ether sulphonates, arylalkylsulphonates, arylalkylether sulphonates, alkylphosphates, alkyletherphosphates, arylalkylphosphates, arylalkyletherphosphates, alkylphosphonates, alkyletherphosphonates, arylalkylphosphonates, arylalkyletherphosphonates, alkylcarboxylates, alkylethercarboxylates, arylalkylcarboxylates, arylalkylethercarboxylates, polyalkyl ethers, arylalkyl polyethers, etc.

An alkyl chain is defined as a chain of 6 to 24 carbons, branched or unbranched, with or without several units, which may optionally include one or more heteroatoms (O, N, S). An arylalkyl chain is defined as a chain of 6 to 24 carbons, branched or unbranched, comprising one or more aromatic rings and possibly comprising one or more heteroatoms (O, N, S).

The surface-active compounds most commonly used, for reasons of cost, stability and availability, are of the sulphonate or sulphate type, in the form of alkali metal or ammonium salts.

**Method for reducing the friction of a fracturing fluid in an operation for the hydraulic fracturing of an unconventional underground oil or gas reservoir**

In some embodiments, a method for reducing the friction of a fracturing fluid in an operation for the hydraulic fracturing of an unconventional underground oil or gas reservoir comprises preparing a fracturing fluid as previously described, and injecting said fracturing fluid into the underground reservoir.

Friction reduction reduces or eliminates losses (pressure) due to friction during injection of the fracturing fluid.

**Other uses of the polymer**

In some embodiments, the polymer may be used in: drilling or cementing wells; conformance, diversion; open,

closed or semi-closed circuit water treatment; treatment of fermentation broth; sludge treatment; construction; paper or cardboard manufacture; batteries; wood treatment; treatment of hydraulic compositions (concrete, cement, mortar and aggregates); formulation of cosmetic products; formulation of detergents; textile manufacture; geothermal energy; manufacture of diapers; or agriculture.

In some embodiments, the polymer may be sued as a coagulant, binding agent, absorbent agent, draining agent, filler retention agent, dehydrating agent, conditioning agent, stabilising agent, fixing agent, film-forming agent, sizing agent, superplasticizing agent, clay inhibitor or dispersant.

The invention and its advantages will be better understood in the light of the following figures and examples provided in order to illustrate the invention in a non-limiting manner.

**Description of the Figures**

Figure 1 shows the proton NMR spectrum of ATBS needle-shape crystals obtained according to Example 1.

Figure 2 shows the proton NMR spectrum of ATBS.K crystals obtained according to Example 2a.

Figure 3 shows the X-ray diffraction pattern of the ATBS crystals obtained according to Example 1.

Figure 4 shows the X-ray diffraction pattern of the ATBS.K crystals obtained according to Example 2a.

Figure 5 shows the Fourier-transform infrared spectrum of the ATBS crystals obtained in Example 1.

Figure 6 shows the Fourier-transform infrared spectrum of the ATBS.K crystals obtained in Example 2a.

Figure 7 shows the thermogram of the ATBS crystals obtained according to Example 1.

Figure 8 shows the thermogram of the ATBS.K crystals obtained according to Example 2a.

Figure 9 shows the particle-size distribution of the ATBS crystals obtained according to example 1.

Figure 10 shows the particle-size distribution of the ATBS.K crystals obtained according to example 2a.

Figure 11 shows an optical microscope view of the ATBS crystals obtained according to example 1.

Figure 12a shows an optical microscope view of the ATBS.K crystals obtained according to example 2a.

Figure 12b shows the thermogram of the ATBS.K crystals obtained according to example 2b.

Figure 12c shows the thermogram of the ATBS.K crystals obtained according to example 2c.

Figure 12d shows a photo of the ATBS.K product obtained in solution according to comparative example 2b (according to US6331647 (example 27)).

Figure 12e shows an optical microscope view of the ATBS.K crystals obtained according to comparative example 2c (according to WO2013079507 (example 3).

Figure 12f shows an optical microscope view of the ATBS.K crystals obtained according to comparative example 2d.

Figure 12g shows an optical microscope view of the ATBS.K crystals obtained according to comparative example 2e.

Figure 13 shows the corrosion effect of the ATBS used, whether in acid form (example 1) or as crystals of potassium salt (example 2a), on carbon steel plates at 50°C, after 15 days.

Figure 14 shows the percentage reduction in friction as a function of time for polymers.

Figure 15 shows the percentage reduction in friction as a function of time for homopolymers.

Figure 16 shows the percentage reduction in friction as a function of time for terpolymers.

Figure 17 shows the percentage reduction in friction as a function of time for post-hydrolyzed polymers.

Figure 18 shows the effect of the ATBS form on the loss of viscosity of solutions of homopolymers **P3** (invention) and **P'3** (acid form of ATBS) in contact with different amounts of iron (II) contaminant.

Figure 19 shows the effect of the ATBS form on the loss of viscosity when polymers **P3** (invention) and **P'3** (acid form of ATBS) are aged at 90°C.

Figure 20 shows the effect of the ATBS form on the loss of viscosity of solutions of homopolymers **P5** (invention) and **P'5** (acid form of ATBS) in contact with different amounts of iron (II) contaminant.

## Examples

### Example 1: Synthesis of the 2-acrylamido-2-methylpropanesulphonic acid (ATBS) ($A_H$)

1522 grams of acrylonitrile containing 0.4% by weight of water and 180 g of fuming sulphuric acid with a titre of 104% $H_2SO_4$ (18% oleum) are added to a stirred, double-jacketed 2000 ml reactor. The mixture is stirred for 1 h and cooled by the double jacket of the reactor, which keeps the temperature of the sulphonating mixture at -20°C.
97 g of isobutylene are added to the previous sulphonating mixture at a rate of 1.6 g/minute.
The temperature of the mixture is controlled at 45°C when the isobutylene is added. The particles of the ATBS precipitate out of the mixture and the solids content is approximately 20% by weight. The reaction mixture is filtered through a Büchner funnel and dried under vacuum at 50°C. The solid obtained is 2-acrylamido-2-methylpropanesulphonic acid (ATBS **$A_H$**) in the form of a very fine white powder.
Optical microscope observations (Figure 11) show that the crystals of ATBS **$A_H$** have a needle-shape morphology.

### Example 2a: Formation of the crystalline form of the 2-acrylamido-2-methylpropanesulphonic acid potassium salt (ATBS.K $A_K$2a) (invention)

477 g of a 28% (by weight in water) potassium hydroxide solution are added to a stirred, double-jacketed 1000 ml reactor. 452 g of ATBS **$A_H$** are added to the previous mixture.
The mixture is stirred for 30 min, at 10°C, to form an aqueous solution $SA_2$.
The aqueous solution $SA_2$ is heated to a temperature of 40°C under vacuum of 50 mbar, for 20 min, then the temperature is maintained for 30 min under a vacuum of 50 mbar and cooled to a temperature of 10°C. The cooling time between 40°C and 10°C is 6 h. A suspension $S_1$ of crystals of ATBS.K is obtained. The suspension $S_1$ is filtered on a Robatel vertical centrifuge. A solid of composition $C_1$ is obtained, containing 80% by weight of crystals of the ATBS.K **$A_K$2a.**
Optical microscope observations (Figure 12a) show that the crystals **$A_K$2a** have a columnar and platelet morphology.

### Example 2b: Formation of the crystalline form of the ATBS potassium salt (ATBS.K $A_K$2b)

The crystals of ATBS.K **$A_K$2b** are prepared according to the procedure described in Example 2a except that $SA_2$ is distilled under 700 mbar.
Optical microscope observations (Figure 12b) show that the crystals **$A_K$2b** obtained in these conditions are identical to the crystals of ATBS.K **$A_K$2a** prepared in example 2a.

### Example 2c: Formation of the crystalline form of the ATBS potassium salt (ATBS.K $A_K$2c)

The crystals of ATBS.K **$A_K$2c** are prepared according to the procedure described in Example 2a except that the cooling time is reduced to 3h45.
Optical microscope observations (Figure 12c) show that show that the crystals obtained in these conditions are identical to the crystals of ATBS.K **$A_K$2a.**

**Comparative example 2a: Preparation of crystals of ATBS.K under atmospheric pressure (1 bar) (CE- $A_K$2a) (not obtained)**

The reaction is carried out according to the procedure described in Example 2a except that $SA_2$ is distilled under atmospheric pressure.

At the end of the cooling step, the aqueous solution $SA_2$ does not allow the formation of a suspension S1, and no filtration or centrifuging operation can be carried out to isolate crystals of ATBS potassium salt.

**Comparative example 2b: Preparation of crystals of ATBS potassium salt (CE- $A_K$2b) (not obtained)**

The reaction was carried out according to the conditions described in example 27 of patent application US6331647.

124 g of potassium hydroxide and 0.13 g of hydroquinone monomethyl ether are added to a stirred, double-jacketed 5 000 ml reactor with 400 g of water. The medium is stirred until all the potassium hydroxide is dissolved.

632 g of ATBS $\mathbf{A_H}$ are added to the previous mixture. The mixture is stirred for 30 min, at 10°C, to form an aqueous solution of ATBS.K.

The aqueous solution obtained is filtered in a 3 000 ml reactor equipped for distillation and containing an air purge tube. The content is heated and stirred while air is blown belon the surface at 0.5 cubic feet per h (0.14 $m^3$/h).

The content is heated to 50°C while under a vacuum of 933 mbar (~700 millimeters of mercury). As the water is removed, yellowish honeylike product is obtained. The product is then transfer to a Robatel vertical centrifuge, but no solid is recovered.

No optical microscope observation was possible as no solid was obtained (Figure 12d).

**Comparative example 2c: Preparation of crystals of ATBS potassium salt (CE-$A_K$2c) (not obtained)**

The reaction was carried out according to the conditions described in example 3 of patent application WO2013079507.

100 g of an ATBS.K solution (16.77% by weight) is obtained according to example 1 of WO2013079507.

50g of solvent from the ATBS.K solution are removed at room temperature under reduced pressure and while introducing air into the ATBS.K solution. The solid formed is then filtered and washed with acrylonitrile/methanol and then dried at 50°C overnight.

Optical microscope observations (Figure 12e) show that the dried solid of ATBS.K CE-**$\mathbf{A_K}$2c** does not correspond to the crystals of ATBS.K according to the invention.

**Comparative example 2d: Preparation of ATBS.K CE-$A_K$2d (not according to the invention)**

The reaction is carried out according to the procedure described in Example 2a except that $SA_2$ is distilled under 720 mbar.

A solid of composition C1 is obtained, containing 80% by weight of crystals of the ATBS.K **CE-$\mathbf{A_K}$2d**.

Optical microscope observations (Figure 12f) show that the crystals of ATBS.K **CE-$\mathbf{A_K}$2d** does not correspond to the crystals of ATBS.K according to the invention.

**Comparative example 2e: Preparation of ATBS.K CE-$A_K$2e (not according to the invention)**

The reaction is carried out according to the procedure described in Example 2a except that the cooling time is 3h20.

A solid of composition $C_1$ is obtained, containing 80% by weight of crystals of the ATBS.K **CE-$\mathbf{A_K}$2e.**

Optical microscope observations (Figure 12g) show that the crystals of ATBS.K **CE-$\mathbf{A_K}$2e** does not correspond to the crystals of ATBS.K according to the invention.

**Example 3: NMR analysis of the ATBS $A_H$ and ATBS.K $A_K$2a products from examples 1 and 2a**

ATBS $\mathbf{A_H}$ and ATBS.K **$\mathbf{A_K}$2a** are analysed by proton nuclear magnetic resonance (NMR). The samples are dissolved in $D_2O$. The NMR machine is a Bruker model with a frequency of 400 MHz and is fitted with a 5 mm BBO BB-$^1$H.

The two proton spectra (Figure 1 and Figure 2) are similar and the peak assignments are consistent with the molecular structure of ATBS or the potassium salt.

**Example 4: Analysis by X-ray diffraction of products of ATBS $A_H$ and ATBS.K $A_K$2a from examples 1 and 2a**

ATBS $\mathbf{A_H}$ and crystals of ATBS.K $\mathbf{A_K2a}$ are ground beforehand to form powders and are analysed by X-ray diffraction over an angular range of 10 to 90°. The equipment used is a Rigaku MiniFlex II diffractometer equipped with a copper source.

Crystals of ATBS.K $\mathbf{A_K2a}$ (Figure 4) present an X-ray diffraction pattern with the following characteristic peaks: 13.1°; 14.4°; 16.3°; 19.8°; 23.5°; 24.3°; 26.9°; 27.6°; 29.3°; 30.6°; 31.6°; 34.3°; 36.1°; 41.7°; 44.6°; 46.7° 2 theta (+/- 0.1°).

The X-ray diffraction pattern of the ATBS $\mathbf{A_H}$ (Figure 3) does not have the same peaks.

**Example 5: Fourier-transform infrared measurement of ATBS $A_H$ and ATBS.K $A_K$2a**

The equipment used for the Fourier transform infrared measurement is the Perkin Elmer Spectrum 100 fitted with a single reflection ATR polarization accessory, with an accuracy of 8 $cm^{-1}$.

ATBS $\mathbf{A_H}$ and ATBS.K $\mathbf{A_K2a}$ are sieved to 100 μm. The particles remaining on the sieve are dried and placed in an oven at 60°C for at least 4 h.

A few hundred milligrams of solid are placed on the diamond of the ATR accessory and pressure is applied manually using the accessory.

The following bands (figure 6) are characteristic of the crystalline form of the ATBS potassium salt $\mathbf{A_K2a}$**:** 3293$cm^{-1}$, 3075 $cm^{-1}$, 3000 $cm^{-1}$, 2979 $cm^{-1}$, 1655 $cm^{-1}$, 1625 $cm^{-1}$, 1550 $cm^{-1}$, 1405 $cm^{-1}$, 1209 $cm^{-1}$, 1190 $cm^{-1}$, 1162 $cm^{-1}$, 1048 $cm^{-1}$, 979 $cm^{-1}$, 824 $cm^{-1}$, 803 $cm^{-1}$, 756 $cm^{-1}$, 633 $cm^{-1}$, 523 $cm^{-1}$.

The infrared spectrum of ATBS $\mathbf{A_H}$ (figure 5) does not have the same peaks.

**Example 6: Differential Scanning Calorimetry (DSC)** of the **ATBS $A_H$ and ATBS.K $A_K$2a products from examples 1 and 2a**

The equipment used is a Mettler DSC 3.

ATBS $\mathbf{A_H}$ and crystals of ATBS.K $\mathbf{A_K2a}$ are analysed with a heating ramp of 10°C/minute under a flow of nitrogen. The initial temperature is 30°C, and the product is heated to 350°C.

The thermogram of the crystals of ATBS $\mathbf{A_H}$ (Figure 7) shows a thermal effect at a temperature of 195.15°C, which is generally considered to be the melting/degradation point of 2-acrylamido-2-methylpropanesulphonic acid.

The thermogram of the crystals of ATBS.K $\mathbf{A_K2a}$ (Figure 8) shows 2 thermal phenomena at 79.4°C; 207°C.

In comparison, the thermogram of the crystals in example 1 shows only one degradation peak at 195.15°C followed by two exothermic degradation phenomena at 212.8°C and 288.4°C (Figure 7).

**Example 7: Measurement of the minimum ignition energy (MIE) of the ATBS $A_H$ and ATBS.K $A_K$2a products from examples 1 and 2a**

The minimum ignition energy is measured in accordance with standard NF EN 13821.

The explosimeter is a vertical Hartmann tube. The dust dispersion system is of the mushroom type.

Total induction is less than 25 microhenry. Discharge voltage is between 5 kV and 15 kV. The electrodes are made of brass and spaced apart by a minimum distance of 6 mm.

Different energies and dispersed masses were tested and are summarized in table 1 and table 2.

It is clear that the crystalline form of ATBS.K $\mathbf{A_K2a}$ presents a much lower risk of explosion than the needle form of ATBS $\mathbf{A_H}$ obtained in example 1.

Table 1: Determination of the solid MIE of ATBS $\mathbf{A_H}$

| Energy (mJ) | Dispersed solid mass (g) | Dispersion number | Ignition? Yes (Y) No (N) | Flame | Pressure |
|---|---|---|---|---|---|
| 1000 | 0,5 | 2 | Y | Small | Low |
| 500 | 0,5 | 3 | Y | Average | Average |
| 300 | 0,5 | 3 | Y | Average | Average |
| 100 | 0,5 | 20 | N | - | - |
| 200 | 0,5 | 20 | N | - | - |
| 200 | 1 | 20 | N | - | - |
| 200 | 2 | 20 | N | - | - |

(continued)

| Energy (mJ) | Dispersed solid mass (g) | Dispersion number | Ignition? Yes (Y) No (N) | Flame | Pressure |
|---|---|---|---|---|---|
| 200 | 3 | 7 | Y | | |
| 100 | 3 | 20 | N | - | - |
| 100 | 5 | 20 | N | - | - |
| 100 | 7 | 20 | N | - | - |
| 100 | 10 | 20 | N | - | - |
| 100 | 1 | 20 | N | - | - |
| 100 | 2 | 20 | N | - | - |

Table 2: Determination of the solid MIE of ATBS.K **$A_K$2a**

| Energy (mJ) | Dispersed solid mass (g) | Dispersion number | Ignition? Yes (Y) No (N) | Flame | Pressure |
|---|---|---|---|---|---|
| 1000 | 0,5 | 20 | N | - | - |
| 1000 | 1 | 20 | N | - | - |
| 1000 | 2 | 20 | N | - | - |
| 1000 | 3 | 20 | N | - | - |
| 1000 | 3 | 20 | N | - | - |
| 1000 | 5 | 20 | N | - | - |
| 1000 | 7 | 20 | N | - | - |
| 1000 | 10 | 20 | N | - | - |
| 1000 | 15 | 20 | N | - | - |
| 1000 | 20 | 20 | N | - | - |
| High energy | 0,5 | 3 | Y | Small | Low |

**Example 8: Particle-size measurement of ATBS $A_H$ and ATBS.K $A_K$2a products from examples 1 and 2a**

ATBS **$A_H$** and crystals of ATBS.K **$A_K$2a** are analysed by laser diffraction to determine their particle-size distribution.

The laser diffraction equipment used is a Cilas 1190.

The crystals of ATBS **$A_H$** have a $d_{50}$ value of approximately 40 µm and 90% of the particles are smaller than 100 µm (Figure 9).

The crystals of ATBS.K **$A_K$2a** have a $d_{50}$ value of approximately 600 µm and 90% of the particles are smaller than approximately 1500 µm (Figure 10). The crystals of ATBS.K **$A_K$2a** contain less than 10% of particles smaller than 325 µm.

**Example 9: Evaluation of the corrosivity of different forms of ATBS on carbon steel**

20 g of each ATBS **$A_H$**, ATBS.K **$A_K$2a** and ATBS.K **CE-$A_K$2c to 2e** are deposited on two carbon steel plates measuring 20 x 50 $mm^2$. The coated plates are placed in an oven at 50°C for 2 weeks. At the same time, a control plate is left uncoated but placed under the same temperature conditions.

Photographs of the plates in this situation (Figure 13) show visually more pronounced corrosion on the plate that had been in contact with ATBS **$A_H$** compared to the crystals of ATBS.K **$A_K$2a**. Weighing the plates before and after the contact period confirms these observations.

Crystals ATBS potassium salts **CE-$A_K$2c to 2e** have also been tested. The results are presented in Table 3.

Table 3: Corrosivity of different forms of ATBS on carbon steel

| | Initial weight (g) at t=0 | Final weight (g) t+15 days | % loss |
|---|---|---|---|
| Control plate | 9.5032 | 9.5020 | 0.012 |

(continued)

| | Initial weight (g) at t=0 | Final weight (g) t+15 days | % loss |
|---|---|---|---|
| Plate + ATBS **$A_H$** | 9.3560 | 8.6582 | 7.46 |
| Plate + ATBS.K **$A_K$2a** | 9.5231 | 9.3526 | 1.79 |
| Plate + ATBS.K **CE-$A_K$2c** | 9.4854 | 9.1274 | 3.77 |
| Plate + ATBS.K **CE-$A_K$2d** | 9.3498 | 9.1210 | 2.44 |
| Plate + ATBS.K **CE-$A_K$2e** | 9.4118 | 9.0219 | 4.14 |

**Example 10a: Preparation of a solution of ATBS.K $A_K$2a**

1000 g of the crystalline form of the ATBS.K **$A_K$2a** and 1000 g of water are introduced into a double-jacketed 2000 ml reactor fitted with a condenser, a pH meter and a stirrer. The mixture has a pH greater than 12.

The mixture obtained is a solution of ATBS.K at a concentration of 50% by weight in water.

**Example 10b: Preparation of a solution of ATBS.K CE-$A_K$2c**

The same protocol as in example 10a is reproduced using the crystals of ATBS.K CE-**$A_K$2c**.

**Example 10c: Preparation of a solution of ATBS.K CE-$A_k$2d**

The same protocol as in example 10a is reproduced using the crystals of ATBS.K CE-**$A_K$2d**.

**Example 10d: Preparation of a solution of ATBS.K CE-$A_K$2e**

The same protocol as in example 10a is reproduced but using the crystals of ATBS.K CE-**$A_K$2e**.

**Example 11: Preparation of a solution of ATBS.K from the acid form $A_H$**

800 g of ATBS **$A_H$** and 650 g of water are introduced into a double-jacketed 2000 ml reactor fitted with a condenser, a pH meter and a stirrer. The mixture has a pH less than 1.

A 50% by weight potassium hydroxide in water solution is prepared in a dropping funnel. The caustic solution is added to the reaction mixture over 120 min. The temperature is controlled to less than 30°C.

The final pH of the solution is between 8 and 10.

451 g of 50% by weight potassium hydroxide in water solution are added.

The mixture obtained is a solution of ATBS potassium salt at a concentration of 50% by weight in water.

**Example 12: Influence of the form and structure of the ATBS on storage**

500 g of 50% (by weight in water) of the solutions of ATBS.K prepared according to examples 10a-d and 11 were stored for 12 months in order to compare their stability over time by measuring and monitoring the appearance of homopolymers of ATBS.K.

In parallel, the stability of the different ATBS (acid **$A_H$** or potassium salt **$A_K$2a**) in solid form was also assessed over the same period of time.

In this case, every three months, 500 g of a solution of ATBS.K was freshly prepared according to the preparation process described in example 10a or 11 using the stored ATBS products of **$A_H$**, **$A_K$2a** and **CE-$A_K$2c to 2e.**

Stability of the solid products was also assessed by monitoring the amount of homopolymers of ATBS.K present after that time.

The solutions were analysed by liquid-phase steric exclusion chromatography using an Agilent 1260 chromatograph equipped with Aquagel-OH 20, 30, 40 and 50 columns allowing analysis of anionic polymers up to 600,000 g/mol PEG equivalent.

The solutions of ATBS.K were diluted to 2000 ppm (by weight in water) before being injected. The UV signal at 250 nm at the column outlet was integrated for the polymer peaks and are detailed in tables 4 and 5 below. The larger the signal area, the more polymer is present, and therefore the lower the signal stability of the product over time.

Table 4: Stability of ATBS and ATBS potassium salt stored in solutions

| Sampling time after production | Solution according to example 10a stored (mV.s) | Solution according to example 10b stored (mV.s) | Solution according to example 10c stored (mV.s) | Solution from stored example 10d (mV.s) | Solution according to example 11 stored (mV.s) |
|---|---|---|---|---|---|
| $T_0$ | 0 | 0 | 0 | 0 | 0 |
| $T_0$ + 3 months | 653 | 7899 | 7724 | 7452 | 7533 |
| $T_0$ + 6 months | 15326 | - | - | - | 423236 |
| $T_0$ + 9 months | 25631 | - | - | - | 653126 |
| $T_0$ + 12 months | 65231 | - | - | - | 1523621 |

Table 5: Stability of ATBS and ATBS potassium salt stored in solid form

| Sampling time after production | Newly prepared solution from the stored crystals **$A_K$2a** (mV.s) | Newly prepared solution from stored crystals **CE-$A_K$2c** (mV.s) | Newly prepared solution from the stored crystals **CE-$A_K$2d** (mV.s) | Newly prepared solution from the stored crystals **CE-$A_K$2e** (mV.s) | Newly prepared solution from the stored crystals of **$A_H$** (mV.s) |
|---|---|---|---|---|---|
| $T_0$ | 0 | 0 | 0 | 0 | 0 |
| $T_0$ + 3 months | 200 | 261 | 254 | 251 | 250 |
| $T_0$ + 6 months | 6532 | - | - | - | 7702 |
| $T_0$ + 9 months | 9532 | - | - | - | 10503 |
| $T_0$ + 12 months | 25631 | - | - | - | 256300 |

These results demonstrate that, whether stored in solid or as a solution, the crystalline form of ATBS.K of the present invention presents an improved stability on storage.

**Example 13: Preparation of polymers of acrylamide (AM)/ATBS (75/25 mol%)**

Example 13a: Preparation of polymers **P1 to P3-$A_K$2a** according to the invention

628.3 g of deionized water, 500 g of 50% (by weight in water) acrylamide solution, 16.2 g of urea and 288.7 g of crystals of ATBS.K **$A_K$2a** are added to a 2000 ml beaker.
The resulting solution is cooled to between 0 and 5°C and transferred to an adiabatic polymerization reactor, where it is bubbled with nitrogen for 30 min to remove all traces of dissolved oxygen.
The following are then added to the reactor:

- 0.75 g of 2,2'-azobisisobutyronitrile,
- 1.5 ml of a 5 g/L solution (in water) of 2,2'-azobis[2-(2-imidazolin-2-yl)propane] dihydrochloride,
- 1.5 ml of a 3 g/L solution (in water) of sodium hypophosphite,
- 2.25 ml of a 1 g/L solution (in water) of tert-butyl hydroperoxide,
- 2.25 ml of a 1 g/L solution (in water) of ammonium iron(II) sulphate (Mohr's salt).

After a few minutes, the nitrogen inlet is shut off and the reactor is closed. The polymerization reaction takes place for 1 to 5 h until a temperature peak is reached. The rubbery gel obtained is chopped into particles of between 1 and 6 mm in size.
The gel is then dried and ground to obtain polymer **P1-$A_K$2a** in powder form.
Polymers **P2- $A_K$2a** and **P3- $A_K$2a** are obtained using the method for obtaining polymer **P1-$A_K$2a** by varying the amount of sodium hypophosphite.

**P2-$A_K$2a:** 1.2 ml of a 3 g/l solution of sodium hypophosphite
**P3-$A_K$2a:** 1.5 ml of a 1 g/l solution of sodium hypophosphite

Example 13b: Preparation of comparative polymers of ATBS/AM (75/25)

The polymers **P'1-$A_H$, P'2-$A_H$** and **P'3-$A_H$** are obtained according to the protocol described for the synthesis of polymers **P1** to **P3-$A_K$2a** using 243 g of ATBS **$A_H$** and 131.7 g of 50% by weight potassium hydroxide in water solution.

Polymers **P'1-CEA$_K$2c, P'2-CEA$_K$2c**, **P'3-CEA$_K$2c** are obtained according to the protocol described for the synthesis of polymers **P1** to **P3-$A_K$2a** using ATBS **CE-$A_K$2c.**

Polymers **P'1-CEA$_K$2d**, **P'2-CEA$_K$2d**, **P'3-CEA$_K$2d** are obtained according to the protocol described for the synthesis of polymers P1 to **P3-$A_K$2a**using ATBS **CE-$A_K$2d**.

Polymers **P'1-CEA$_K$2e**, **P'2-CEA$_K$2e**, **P'3-CEA$_K$2e** are obtained according to the protocol described for the synthesis of polymers **P1** to **P3-$A_K$2a** using ATBS **CE-$A_K$2e**

**Example 14: Preparation of homopolymers of ATBS**

Polymers **P4-$A_K$2a** and **P5-$A_K$2a** are prepared according to the protocol described in Example 13a for the preparation of polymer **P1-$A_K$2a** with the quantities of monomer and excipients being adjusted to reach the desired molar composition of 100 mol% of ATBS **(P4-Ax2a:** 20 ml of a 1 g/l solution of sodium hypophosphite; **P5-$A_K$2a:** 3 ml of a 1 g/l solution of sodium hypophosphite).

The polymers **P'4-$A_H$** and **P'5-$A_H$** are obtained according to the protocol described for the synthesis of polymers P1 to P3-$A_K$2a using respectively 352.1 and 243 g of ATBS **$A_H$** and 190.8 g of 50% by weight potassium hydroxide in water solution.

Polymers **P'4-CEA$_K$2c** and **P'5-CEA$_K$2c** are obtained according to the protocol described for the synthesis of polymers P1 to P3-$A_K$2a using ATBS **CE-$A_K$2c.**

Polymers **P'4-CEA$_K$2d** and **P'5-CEA$_K$2d** are obtained according to the protocol described for the synthesis of polymers P1 to P3-$A_K$2ausing ATBS **CE-$A_K$2d**.

Polymers **P'4-CEA$_K$2e** and **P'5-CEA$_K$2e** are obtained according to the protocol described for the synthesis of polymers **P1** to **P3-$A_K$2a** using ATBS **CE-$A_K$2e**

**Example 15: Preparation of polymers of AM/ATBS/acrylic acid (AA) (71/9/20 mol%)**

Polymers **P6-$A_K$2a** is prepared according to the protocol described in Example 13a for the preparation of polymers **P1** with the quantities of monomers and excipients being adjusted to reach the desired molar composition of AM/ATBS/AA (71/9/20).

The polymers **P'6-$A_H$** is obtained according to the protocol described for the synthesis of polymers **P1-$A_K$2a** using respectively 105.2 g of ATBS **$A_H$** and 181.4 g of 50% by weight potassium hydroxide in water solution.

Polymers **P'6-CEA$_K$2c** is obtained according to the protocol described for the synthesis of polymers P1 to **P3-$A_K$2a** using ATBS **CE-$A_K$2c.**

Polymers **P'6-CEA$_K$2d** is obtained according to the protocol described for the synthesis of polymers P1 to **P3-$A_K$2a** using ATBS **CE-$A_K$2d**.

Polymers **P'6-CEA$_K$2e** is obtained according to the protocol described for the synthesis of polymers **P1** to **P3-$A_K$2a** using ATBS **CE-$A_K$2e**

**Example 16: Preparation of post-hydrolyzed polymers of AM/ATBS (90/10 mol%)**

Polymers **P7-$A_K$2a** is prepared according to the protocol described in Example 13a for the preparation of polymers **P1** with the quantities of monomers and excipients being adjusted to reach the desired molar composition of AM/ATBS (90/10).

500.0 g of pre-chopped gel is then mixed with 22.5 g of 50% sodium hydroxide solution, and the mixture is heated and maintained at a temperature of 90°C for 90 min.

The gel is then dried and ground to obtain polymer **P7-$A_K$2a** in powder form.

Polymers **P'7-$A_H$** is prepared according to the protocol described for the preparation of polymers **P7-$A_K$2a** using 93.1 g of ATBS **$A_H$** and 50.1 g of 50% by weight potassium hydroxide in water solution.

Polymers **P'7-CEA$_K$2c** is prepared according to the protocol described for the synthesis of polymers **P7-$A_K$2a** using ATBS **CE-$A_K$2c.**

Polymers **P'7-CEA$_K$2d** is prepared according to the protocol described for the synthesis of polymers **P7-$A_K$2a** using ATBS **CE-$A_K$2d**.

Polymers **P'7-CEA$_K$2e** is prepared according to the protocol described for the synthesis of polymers **P7-$A_K$2a** using ATBS **CE-$A_K$2e**.

**Example 17: Viscosity measurement of solutions of polymers of AM/ATBS** (75/25 **mol%)**

The viscosity of the polymers prepared in example13 is measured at 25°C in a 0.5 M aqueous sodium chloride solution using a Brookfield LVT viscometer fitted with a UL adapter at 60 rpm.

Preparation of the polymer solutions:

500 mg of dried polymers are dissolved in a beaker containing 290 ml of deionized water at a stirring speed of 500 rpm.

29.25 g of sodium chloride are added to the prepared solutions.

The solutions are left to stir for 10 min at 700 rpm to dissolve the salt completely.

The prepared solutions are filtered through a 200 μm mesh.

16 ml of the prepared solutions is transferred to a cylindrical tube and used to carry out a viscosity measurement. Results of viscosity of the polymer solutions is presented in Table 6.

Table 6: Viscosity of ATBS polymer solutions. Inv = invention and CE = comparative example

| Polymer | ATBS used | Viscosity (cps) |
|---|---|---|
| **P1-$A_K$2a** (inv) | Crystals of ATBS.K **$A_K$2a** | 7.1 |
| **P'1-$A_H$** (CE) | ATBS **$A_H$** | 4.5 |
| **P'1-CE$A_K$2c** (CE) | ATBS.K **CE-$A_K$2c** | 4.6 |
| **P'1-CE$A_K$2d** (CE) | ATBS.K **CE-$A_K$2d** | 4.2 |
| **P'1-CE$A_K$2e** (CE) | ATBS.K **CE-$A_K$2e** | 4.6 |
| **P2-$A_K$2a** (inv) | Crystals of ATBS.K **$A_K$2a** | 5.0 |
| **P'2-$A_H$** (CE) | ATBS **$A_H$** | 3.2 |
| **P'2-CE$A_K$2c** (CE) | ATBS.K **CE-$A_K$2c** | 3.3 |
| **P'2-CE$A_K$2d** (CE) | ATBS.K **CE-$A_K$2d** | 3.1 |
| **P'2-CE$A_K$2e** (CE) | ATBS.K **CE-$A_K$2e** | 2.9 |
| **P3-$A_K$2a** (inv) | Crystals of ATBS.K **$A_K$2a** | 9.9 |
| **P'3-$A_H$** (CE) | ATBS **$A_H$** | 7.3 |
| **P'3-CE$A_K$2c** (CE) | ATBS.K **CE-$A_K$2c** | 7.6 |
| **P'3-CE$A_K$2d** (CE) | ATBS.K **CE-$A_K$2d** | 7.4 |
| **P'3-CE$A_K$2e** (CE) | ATBS.K **CE-$A_K$2e** | 7.4 |

Polymers prepared using the novel crystalline form of the ATBS.K have a higher viscosity than polymers prepared from the conventional form of ATBS.K or other crystalline form of the potassium salt.

**Example 18: Preparation of fracturing fluids of polymers of ATBS**

Polymers of examples 13 to 16 are placed in solution while stirring at a concentration of 10,000 ppm by weight in a brine composed of water, 85 g of sodium chloride (NaCl) and 33.1 g of calcium chloride ($CaCl_2$, $2H_2O$) per litre of brine.

The resulting saline polymer solutions are then injected at a concentration of 0.05 pptg (parts per thousand gallons; 1 gallon = 3.78541 litres) into the brine, which is recirculated for the flow loop tests that follow.

**Example 19: Flow loop friction reduction tests of polymers of ATBS**

In order to assess the friction reduction of each polymer, the flow loop tank is filled with 20 L of brine (brine described in example 18).

The brine is then recirculated through the flow loop at a rate of 24 gallons per minute. The polymer is added to the recirculating brine at a concentration of 0.5 pptg.

The percentage reduction in friction is thus determined by measuring pressure variations within the flow loop.

Figures 14 to 17 are graphs showing the percentage reduction in friction as a function of time for polymers **P1, P4,**

**P6** and **P7-$A_K$2a** according to the invention and comparative examples polymers **P'1, P'4, P'6** and **P'7-$A_H$.**

These Figures show that injection fluids according to the invention provide improved friction reduction. Indeed, friction reduction is improved when the polymers contain ATBS in the crystalline form of ATBS.K.

**Example 20: Measurement of the filter ratio (FR) of solutions of polymers of AM/ATBS (75/25 mol)**

Filtration tests were carried out on polymers described in example 13.

The polymer solutions were obtained at an active concentration of 1000 ppm in a brine containing water, 30,000 ppm NaCl and 3,000 ppm $CaCl_2$,$2H_2O$ (ppm by weight). The filter ratio (FR) was measured on filters with a pore size of 1.2 μm, which is representative of low-permeability deposits. Results are presented in Table 7.

Table 7: Filter ratio of copolymers of AM/ATBS

| Polymer | ATBS used | Molecular weight (in millions Da) | FR |
|---|---|---|---|
| **P1-$A_K$2a** (inv) | Crystals of ATBS.K **$A_K$2a** | 6.7 | 1.08 |
| **P2-$A_K$2a** (inv) | Crystals of ATBS.K **$A_K$2a** | 9.2 | 1.06 |
| **P3-$A_K$2a** (inv) | Crystals of ATBS.K **$A_K$2a** | 11.5 | 1.07 |
| **P'1-$A_H$** (CE) | ATBS **$A_H$** | 6.6 | 1.13 |
| **P'2-$A_H$** (CE) | ATBS **$A_H$** | 9.2 | 1.17 |
| **P'3-$A_H$** (CE) | ATBS **$A_H$** | 11.4 | 1.45 |
| **P'1-CEA$_K$2c** (CE) | ATBS.K **CE-$A_K$2c** | 6.7 | 1.14 |
| **P'2-CEA$_K$2c** (CE) | ATBS.K **CE-$A_K$2c** | 9.2 | 1.17 |
| **P'3-CEA$_K$2c** (CE) | ATBS.K **CE-$A_K$2c** | 11.4 | 1.46 |
| **P'1-CEA$_K$2d** (CE) | ATBS.K **CE-$A_K$2d** | 6.5 | 1.15 |
| **P'2-CEA$_K$2d** (CE) | ATBS.K **CE-$A_K$2d** | 9.3 | 1.17 |
| **P'3-CEA$_K$2d** (CE) | ATBS.K **CE-$A_K$2d** | 11.5 | 1.42 |
| **P'1-CEA$_K$2e** (CE) | ATBS.K **CE-$A_K$2e** | 6.7 | 1.15 |
| **P'2-CEA$_K$2e** (CE) | ATBS.K **CE-$A_K$2e** | 9.1 | 1.18 |
| **P'3-CEA$_K$2e** (CE) | ATBS.K **CE-$A_K$2e** | 11.5 | 1.47 |

It can be seen in table 7 that, for equivalent molecular weights, the polymers obtained from the crystalline form of the ATBS.K according to the invention **(P1, P2** and **P3-$A_K$2a)** always have a lower FR than the polymers obtained from the non-crystalline form of the ATBS.K or from a different crystalline form of ATBS.K. This difference becomes increasingly significant as the molecular weight of the polymer increases.

**Example 21: Measurement of the resistance to chemical degradation of solutions of polymers of AM/ATBS (75/25) of equivalent molecular weights**

Tests of the resistance to chemical degradation of polymer **P3-$A_K$2a** and comparative polymers of the **P'3** type of example 13 were carried out under aerobic conditions in the presence of different concentrations of iron(II) (2, 5, 10 and 20 ppm) in a brine composed of water, 37,000 ppm of NaCl, 5,000 ppm of $Na_2SO_4$ and 200 ppm of $NaHCO_3$ (ppm by weight). The results obtained after 24 h of contact between the solutions of polymers **P3-$A_K$2a** and **P'3-$A_H$** with the iron contaminant are represented in Figure 18.

The results of chemical degradation for all polymers **P3** are also detailed in Table 8.

Table 8: Measurement of the resistance to chemical degradation of polymer **P3-$A_K$2a** of the invention and comparative examples polymers **P'3-$A_H$** and **P'3-CEA$_K$2c to 2e**

| Polymer | Iron (II) concentration (ppm) | viscosity loss (%) |
|---|---|---|
| **P3-$A_K$2a** (inv) | 2 | 5 |
| **P'3-$A_H$** (CE) | 2 | 8 |

(continued)

| Polymer | Iron (II) concentration (ppm) | viscosity loss (%) |
|---|---|---|
| **P'3-CEA$_K$2c** (CE) | 2 | 8.5 |
| **P'3-CEA$_K$2d** (CE) | 2 | 8 |
| **P'3-CEA$_K$2e** (CE) | 2 | 9 |
| **P3-A$_K$2a** (inv) | 5 | 6 |
| **P'3-A$_H$** (CE) | 5 | 11 |
| **P'3-CEA$_K$2c** (CE) | 5 | 10 |
| **P'3-CEA$_K$2d** (CE) | 5 | 10.5 |
| **P'3-CEA$_K$2e** (CE) | 5 | 11 |
| **P3-A$_K$2a** (inv) | 10 | 12.5 |
| **P'3-A$_H$** (CE) | 10 | 16 |
| **P'3-CEA$_K$2c** (CE) | 10 | 15.5 |
| **P'3-CEAx2d** (CE) | 10 | 15 |
| **P'3-CEA$_K$2e** (CE) | 10 | 15.5 |
| **P3-A$_K$2a** (inv) | 20 | 25.5 |
| **P'3-A$_H$** (CE) | 20 | 36 |
| **P'3-CEA$_K$2c** (CE) | 20 | 38 |
| **P'3-CEA$_K$2d** (CE) | 20 | 39 |
| **P'3-CEA$_K$2e** (CE) | 20 | 39 |

Results, illustrated on Figure 18 as well as in Table 8, demonstrate that for each iron(II) concentration, polymer **P3-A$_K$2a** loses less viscosity than the equivalent comparative polymers **P'3-A$_H$** and **P'3-CEA$_K$2c** to **2e.**

**Example 22: Measurement of the resistance to thermal degradation of solutions of polymers of AM/ATBS (75/25) of equivalent molecular weights**

Tests of the resistance to thermal degradation of polymer **P3-A$_K$2a** and comparative polymers of the **P'3** type of example 13 were carried out under anaerobic conditions at an active concentration of 2,000 ppm in a brine composed of 30,000 ppm of NaCl and 3,000 ppm of $CaCl_2,2H_2O$ (ppm by weight). The polymer solutions were aged for 6 months at 90°C.

The results of the loss of viscosity of solutions of polymer **P3-A$_K$2a** and **P'3-A$_H$** are illustrated on Figure 19 and the results for all **P3** polymers are detailed in Table 9 below.

Table 9: Measurement of the resistance to thermal degradation of polymer **P3-A$_K$2a** of the invention and comparative examples polymers **P'3-A$_H$** and **P'3-CEA$_K$2c** to **2e**

| Polymer | ATBS used | viscosity loss (%) |
|---|---|---|
| **P3-A$_K$2a** (inv) | Crystals of ATBS.K **A$_K$2a** | 34.5 |
| **P'3-A$_H$** (CE) | ATBS **A$_H$** | 47 |
| **P'3-CEA$_K$2c** (CE) | ATBS.K **CE-A$_K$2c** | 46.5 |
| **P'3-CEA$_K$2d** (CE) | ATBS.K **CE-A$_K$2d** | 49 |
| **P'3-CEA$_K$2e** (CE) | ATBS.K **CE-A$_K$2e** | 50 |

Results on Fig. 19 and in Table 9 show that polymer **P3-A$_K$2a** loses less viscosity than the equivalent comparative polymers **P'3-A$_H$** and **P'3-CEA$_K$2c** to **2e.**

**Example 23: Measurement of the filter ratio of solutions of homopolymers of ATBS**

Filtration tests were carried out according to the procedure described in Example 20 on polymers **P4-P5** prepared in Example 14. Results are presented in Table 10.

Table 10: Filter ratio of homopolymers of ATBS

| Polymer | ATBS used | Molecular weight (in millions Da) | Filtration ratio |
|---|---|---|---|
| **P4-$A_K$2a** (inv) | Crystals of ATBS.K **$A_K$2a** | 3.1 | 1.07 |
| **P5-$A_K$2a** (inv) | Crystals of ATBS.K **$A_K$2a** | 5.5 | 1.10 |
| **P'4-$A_H$** (CE) | ATBS **$A_H$** | 3.0 | 1.18 |
| **P'5-$A_H$** (CE) | ATBS **$A_H$** | 5.4 | 1.63 |
| **P'4-CEA$_K$2c** (CE) | ATBS.K **CE-$A_K$2c** | 3.1 | 1.19 |
| P'5-**CEA$_K$2c** (CE) | ATBS.K **CE-$A_K$2c** | 5.3 | 1.61 |
| **P'4-CEA$_K$2d** (CE) | ATBS.K **CE-$A_K$2d** | 3.1 | 1.20 |
| **P'5-CEA$_K$2d** (CE) | ATBS.K **CE-$A_K$2d** | 5.3 | 1.59 |
| **P'4-CEA$_K$2e** (CE) | ATBS.K **CE-$A_K$2e** | 3.1 | 1.17 |
| **P'5-CEA$_K$2e** (CE) | ATBS.K **CE-$A_K$2e** | 5.3 | 1.61 |

It can be seen in table 10 that, for equivalent molecular weights, the polymers obtained from the crystalline form of the ATBS.K according to the invention **(P4/5-$A_K$2a)** have always a lower FR than the equivalent comparative polymers **P'4/5-$A_H$** and **P'4/5-CEA$_K$2c** to **2e. This** difference becomes increasingly significant as the molecular weight of the polymer increases.

**Example 24: Measurement of the resistance to chemical degradation of solutions of polymers of AM/ATBS/AA (71/9/20)**

Tests of the resistance to chemical degradation were carried out according to the procedure described in Example 21 on polymers **P6** and **P'6** prepared in Example 15. The results obtained after 24 h of contact between the solutions of polymer **P6-$A_K$2a** and **P'6-$A_H$** with the iron contaminant are shown in Figure 20.

The results of chemical degradation for all polymers **P6** are also detailed in Table 11.

Table 11: Measurement of the resistance to thermal degradation of polymer **P6-$A_K$2a** of the invention and comparative examples polymers **P'6-$A_H$** and **P'6-CEA$_K$2c to 2e**

| Polymer | ATBS used | Iron (II) concentration (ppm) | viscosity loss (%) |
|---|---|---|---|
| **P6-$A_K$2a** (inv) | Crystals of ATBS.K **$A_K$2a** | 2 | 11 |
| **P'6-$A_H$** (CE) | ATBS **$A_H$** | 2 | 12 |
| **P'6-CEA$_K$2c** (CE) | ATBS.K **CE-$A_K$2c** | 2 | 13 |
| **P'6-CEA$_K$2d** (CE) | ATBS.K **CE-$A_K$2d** | 2 | 12.5 |
| **P'6-CEA$_K$2e** (CE) | ATBS.K **CE-$A_K$2e** | 2 | 13 |
| **P6-$A_K$2a** (inv) | Crystals of ATBS.K **$A_K$2a** | 5 | 16 |
| **P'6-$A_H$** (CE) | ATBS **$A_H$** | 5 | 23 |
| **P'6-CEA$_K$2c** (CE) | ATBS.K **CE-$A_K$2c** | 5 | 21 |
| **P'6-CEA$_K$2d** (CE) | ATBS.K **CE-$A_K$2d** | 5 | 22 |
| **P'6-CEA$_K$2e** (CE) | ATBS.K **CE-$A_K$2e** | 5 | 21 |
| **P6-$A_K$2a** (inv) | Crystals of ATBS.K **$A_K$2a** | 10 | 25 |
| **P'6-$A_H$** (CE) | ATBS **$A_H$** | 10 | 38 |
| **P'6-CEA$_K$2c** (CE) | ATBS.K **CE-$A_K$2c** | 10 | 39 |

(continued)

| Polymer | ATBS used | Iron (II) concentration (ppm) | viscosity loss (%) |
|---|---|---|---|
| **P'6-CEA$_K$2d** (CE) | ATBS.K **CE-A$_K$2d** | 10 | 37 |
| **P'6-CEA$_K$2e** (CE) | ATBS.K **CE-A$_K$2e** | 10 | 39 |
| **P6-A$_K$2a** (inv) | Crystals of ATBS.K **A$_K$2a** | 20 | 31 |
| **P'6-A$_H$** (CE) | ATBS **A$_H$** | 20 | 49 |
| **P'6-CEA$_K$2c** (CE) | ATBS.K **CE-A$_K$2c** | 20 | 47 |
| **P'6-CEA$_K$2d** (CE) | ATBS.K **CE-A$_K$2d** | 20 | 51 |
| **P'6-CEA$_K$2e** (CE) | ATBS.K **CE-A$_K$2e** | 20 | 51 |

Results, illustrated on Figure 20 as well as in Table 11, demonstrate that for each iron(II) concentration, polymer **P6-A$_K$2a** loses less viscosity than the equivalent comparative polymers **P'6-A$_H$** and **P'6-CEA$_K$2c** to **2e.**

**Example 25: Measurement of the filter ratio of solutions of polymers of post-hydrolyzed AM/ATBS (90/10 mol%)**

Filtration tests were carried out according to the procedure described in Example 20 on polymers prepared in Example 16. Results are presented in Table 12.

Table 12: Filter ratio of post-hydrolyzed polymers of AM/ATBS (90/10)

| Polymer | ATBS used | Molecular weight (in millions Da) | Filtration ratio |
|---|---|---|---|
| **P7-A$_K$2a** (inv) | Crystals of ATBS.K **A$_K$2a** | 26 | 1.09 |
| **P'7-A$_H$** (CE) | ATBS **A$_H$** | 22 | 1.14 |
| **P'7-CEA$_K$2c** (CE) | ATBS.K **CE-A$_K$2c** | 21 | 1.18 |
| **P'7-CEA$_K$2d** (CE) | ATBS.K **CE-A$_K$2d** | 20 | 1.17 |
| **P'7-CEA$_K$2e** (CE) | ATBS.K **CE-A$_K$2e** | 20 | 1.16 |

The results show that higher molecular weight polymer **P7-A$_K$2a** of the invention has a lower FR in comparison to the FR of comparative polymers **P'7-A$_H$** and **P'7-CEA$_K$2c** to **2e.**

**Example 26: Treatment of a coal mining effluent**

Polymers **P1-A$_K$2a, P'1-A$_H$** and **P'1-CEA$_K$2c** to 2e are dissolved in tap water to obtain aqueous solutions with a concentration of 0.4% by weight of polymer relative to the total weight of the solution. The two solutions are mechanically stirred at 500 rpm until the polymers are completely solubilized and clear, homogeneous solutions are obtained.

A series of flocculation tests is carried out on a coal mine effluent with a solids content of 18.2% by weight.

A quantity of each solution, corresponding to a polymer dosage of 280 g of polymer per tonne of mine effluent dry matter, is added to 200 g of mine effluent, and complete mixing is then carried out manually until optimum flocculation and water release are observed.

The result is expressed as NWR (Net Water Release), which corresponds to the total quantity of water recovered 1 h after the flocculation test minus the quantity of water induced during incorporation of the aqueous polymer solution into the suspension. The same NWR is calculated after 24 h, giving a good indication of the maximum release of water.

The results are resumed in Table 13.

Table 13: Net Water Release results using polymer **P1-A$_K$2a** of the invention and comparative polymers **P'1-A$_H$** and **P'1-CEA$_K$2c** to **2e.**

| Polymer | NWR (mL) | |
|---|---|---|
| | **1h** | **24 h** |
| **P1-A$_K$2a** (inv) | 65 | 85 |

(continued)

| Polymer | NWR (mL) | |
|---|---|---|
| | 1h | 24 h |
| **P'1-A$_H$** (CE) | 55 | 80 |
| **P'1-CEA$_K$2c** (CE) | 54 | 81 |
| **P'1-CEAx2d** (CE) | 53 | 78 |
| **P' 1-CEA$_K$2e** (CE) | 54 | 80 |

The results of this experiment clearly demonstrate that the use of ATBS in the crystalline form of ATBS.K according to the invention makes it possible to obtain a more effective polymer for flocculating a mining effluent from a coal mine.

**Example 27: Treatment of red mud from a Bayer process**

Another series of tests is carried out on red mud from a Bayer process, with a solids content of 22.8% by weight according to the protocol described in Example 26 using 740 g of polymer per ton of red mud dry matter. The results are presented in Table 14.

Table 14: Net Water Release results using polymer **P1-A$_K$2a** of the invention and comparative polymers **P'1-A$_H$** and **P'1-CEA$_K$2c** to **2e.**

| Polymer | NWR (mL) | |
|---|---|---|
| | 1h | 24 h |
| **P1-A$_K$2a** (inv) | 40 | 55 |
| **P'1-A$_H$** (CE) | 35 | 48 |
| **P'1-CEA$_K$2c** (CE) | 36 | 46 |
| **P'1-CEA$_K$2d** (CE) | 35 | 47 |
| **P'1-CEA$_K$2e** (CE) | 35 | 47.5 |

The results of this experiment clearly demonstrate that the use of ATBS in crystalline form according to the invention makes it possible to obtain a more effective polymer for flocculating red mud from a Bayer process.

## Claims

1. Crystalline form of 2-acrylamido-2-methylpropanesulphonic acid potassium salt having an X-ray powder diffraction pattern comprising peaks at 13,1°; 14,4°; 16,3°; 19,8°; 23,5°; 24,3°; 26,9°; 27,6°; 29,3°; 30,6°; 31,6°; 34,3°; 36,1°; 41,7°; 44,6°; 46,7° 2-theta (+/-0.1°).

2. Crystalline form of 2-acrylamido-2-methylpropanesulphonic acid potassium salt ATBS.K according to claim *1,* ***characterized* in that** it has a Fourier-transform infrared spectrum comprising peaks at 3293cm$^{-1}$, 3075 cm$^{-1}$, 3000 cm$^{-1}$, 2979 cm$^{-1}$, 1655 cm$^{-1}$, 1625 cm$^{-1}$, 1550 cm$^{-1}$, 1405 cm$^{-1}$, 1209 cm$^{-1}$, 1190 cm$^{-1}$, 1162 cm$^{-1}$, 1048 cm$^{-1}$, 979 cm$^{-1}$, 824 cm$^{-1}$, 803 cm$^{-1}$, 756 cm$^{-1}$, 633 cm$^{-1}$, 523 cm$^{-1}$ (+/- 8 cm$^{-1}$).

3. Crystalline form of ATBS.K according to claim 1 or 2, ***characterized* in that** it has a minimum ignition energy greater than 500 mJ.

4. Crystalline form of ATBS.K according to one of claims 1 to 3, ***characterized* in that** it exhibits 2 thermal phenomena with the differential scanning calorimetry technique, at 79.4°C; 207°C (+/-10°C).

5. Method for producing the crystalline form of ATBS.K according to one of claims 1 to 4, comprising at least the following successive steps:

1) mixing of 2-acrylamido-2-methylpropanesulphonic acid with an aqueous solution $SA_1$ and at least one potassium salt base in order to form an aqueous solution or an aqueous suspension $SA_2$;
2) distillation, at a pressure of 700 mbar or less, of the aqueous solution or of the aqueous suspension $SA_2$ in order to form a suspension $S_1$ and cooling of the aqueous solution or aqueous suspension $SA_2$ or a suspension $S_1$ wherein the temperature of the cooling step is decreased at a ramp of between 0.1 and 8°C/hour;
3) solid-liquid separation of the suspension $S_1$ and isolation of the crystals of the suspension $S_1$ obtained at the end of step 2) in the form of a composition $C_1$.

**6.** Method according to claim 5, ***characterized in that*** the quantity of ATBS.K in the aqueous solution or the aqueous suspension $SA_2$ is between 10 and 90% by weight relative to the total weight of the aqueous solution or of the aqueous suspension $SA_2$.

**7.** Method according to claim 5 or 6, ***characterized* in that** in step 2), the aqueous solution or the aqueous suspension $SA_2$ is heated, advantageously at a temperature of between 5°C and 95°C.

**8.** Method according to any one of claims 5 to 7, ***characterized* in that** the cooling step of step 2) is carried out at a temperature of between 5°C and 95°C.

**9.** Method according to any one of claims 5 to 8, ***characterized* in that** the suspension $S_1$ comprises between 30 and 90% by weight of crystalline form of ATBS.K, relative to the total weight of the suspension $S_1$.

**10.** Method according to one of claims 5 to 9, ***characterized* in that** the potassium salt is chosen from potassium hydroxide, potassium carbonate, potassium bicarbonate or mixtures thereof.

## Patentansprüche

**1.** Kristalline Form des Kaliumsalzes der 2-Acrylamido-2-methylpropansulfonsäure, dessen Peaks der 2-Theta-Winkel (+/- 0,1°) im Röntgendiffraktionsbild bei 13,1°; 14,4°; 16,3°; 19,8°; 23,5°; 24,3°; 26,9°; 27,6°; 29,3°; 30,6°; 31,6°; 34,3°; 36,1°; 41,7°; 44,6°; 46,7° liegen.

**2.** Kristalline Form des Kaliumsalzes der 2-Acrylamido-2-methylpropansulfonsäure ATBS.K nach Anspruch 1, **dadurch *gekennzeichnet,* dass** die Peaks im Fourier-Transform-Infrarotspektrum bei 3.293 $cm^{-1}$, 3.075 $cm^{-1}$, 3.000 $cm^{-1}$, 2.979 $cm^{-1}$, 1.655 $cm^{-1}$, 1.625 $cm^{-1}$, 1.550 $cm^{-1}$, 1.405 $cm^{-1}$, 1.209 $cm^{-1}$, 1.190 $cm^{-1}$, 1.162 $cm^{-1}$, 1.048 $cm^{-1}$, 979 $cm^{-1}$, 824 $cm^{-1}$, 803 $cm^{-1}$, 756 $cm^{-1}$, 633 $cm^{-1}$, 523 $cm^{-1}$ (+/- 8 $cm^{-1}$) liegen.

**3.** Kristalline Form von ATBS.K nach Anspruch 1 oder 2, ***dadurch gekennzeichnet,* dass** es eine Mindestzündenergie von mehr als 500 mJ aufweist.

**4.** Kristalline Form von ATBS.K nach einem der Ansprüche 1 bis 3, **dadurch *gekennzeichnet,* dass** es beim Verfahren der Differential-Scanning-Kalorimetrie zwei thermische Phänomene bei 79,4 °C und 207 °C (+/- 10 °C) aufweist.

**5.** Verfahren zur Herstellung der kristallinen Form von ATBS.K nach einem der Ansprüche 1 bis 4, das mindestens folgende aufeinanderfolgende Schritte umfasst:

1) Mischen von 2-Acrylamido-2-methylpropansulfonsäure mit einer wässrigen Lösung $SA_1$ und mindestens einer Kaliumsalzbasis, um eine wässrige Lösung oder eine wässrige Suspension $SA_2$ zu bilden;
2) Destillation der wässrigen Lösung oder der wässrigen Suspension $SA_2$ bei einem Druck von 700 mbar oder weniger, um eine Suspension $S_1$ zu bilden, und Abkühlung der wässrigen Lösung oder der wässrigen Suspension $SA_2$ oder einer Suspension $S_1$, wobei die Temperatur des Abkühlungsschritts kontinuierlich zwischen 0,1 bis 8 °C/Stunde verringert wird;
3) Fest-Flüssig-Trennung der Suspension $S_1$ und Isolierung der Kristalle der Suspension $S_1$, die am Ende von Schritt 2) in Form einer Zusammensetzung $C_1$ erhalten wurden.

**6.** Verfahren nach Anspruch 5, **dadurch *gekennzeichnet,* dass** das ATBS.K in der wässrigen Lösung oder in der wässrigen Suspension $SA_2$ in einer Menge zwischen 10 und 90 Gew.-% bezogen auf das Gesamtgewicht der wässrigen Lösung oder der wässrigen Suspension $SA_2$ vorhanden ist.

7. Verfahren nach Anspruch 5 oder 6, **dadurch *gekennzeichnet,* dass** die wässrige Lösung oder die wässrige Suspension $SA_2$ in Schritt 2) erhitzt wird, vorzugsweise auf eine Temperatur zwischen 5 °C und 95 °C.

8. Verfahren nach einem der Ansprüche 5 bis 7, **dadurch *gekennzeichnet,* dass** der Abkühlungsschritt von Schritt 2) bei einer Temperatur zwischen 5 °C und 95 °C durchgeführt wird.

9. Verfahren nach einem der Ansprüche 5 bis 8, **dadurch *gekennzeichnet,* dass** die Suspension $S_1$ zwischen 30 und 90 Gew.-% der kristallinen Form von ATBS.K, bezogen auf das Gesamtgewicht der Suspension $S_1$, umfasst.

10. Verfahren nach einem der Ansprüche 5 bis 9, **dadurch *gekennzeichnet,* dass** das Kaliumsalz aus Kaliumhydroxid, Kaliumcarbonat, Kaliumbicarbonat oder Mischungen davon ausgewählt ist.

**Revendications**

1. Forme cristalline du sel de potassium de l'acide 2-acrylamido-2-méthylpropane sulfonique ayant un diagramme de diffraction des rayons X sur poudre comprenant des pics à 13,1° ; 14,4° ; 16,3° ; 19,8° ; 23,5° ; 24,3° ; 26,9° ; 27,6° ; 29,3° ; 30,6° ; 31,6° ; 34,3° ; 36,1° ; 41,7° ; 44,6° ; 46,7° degrés 2-thêta (+/- 0,1°).

2. Forme cristalline du sel de potassium de l'acide 2-acrylamido-2-méthylpropane sulfonique ATBS.K selon la revendication *1,* ***caractérisée* en ce qu'**elle présente un spectre infra-rouge à transformée de Fourier comprenant des pics à 3293$cm^{-1}$, 3075$cm^{-1}$, 3000$cm^{-1}$, 2979$cm^{-1}$, 1655$cm^{-1}$, 1625$cm^{-1}$, 1550$cm^{-1}$, 1405$cm^{-1}$, 1209$cm^{-1}$, 1190$cm^{-1}$, 1162$cm^{-1}$, 1048$cm^{-1}$, 979$cm^{-1}$, 824$cm^{-1}$, 803$cm^{-1}$, 756$cm^{-1}$, 633$cm^{-1}$, 523$cm^{-1}$ (+/- 8$cm^{-1}$).

3. Forme cristalline d'ATBS.K selon la revendication 1 ou 2, ***caractérisée* en ce qu'**elle présente une énergie minimale d'inflammation supérieure à 500 mJ.

4. Forme cristalline d'ATBS.K selon l'une des revendications 1 à *3,* ***caractérisée* en ce qu'**elle présente 2 phénomènes thermiques avec la technique de calorimétrie différentielle à balayage, à 79,4°C, 207°C (+/-10°C).

5. Procédé de fabrication de la forme cristalline d'ATBS.K selon l'une des revendications 1 à 4, comprenant au moins les étapes successives suivantes ;

   1) mélange d'acide 2-acrylamido-2-méthylpropane sulfonique avec une solution aqueuse $SA_1$ et au moins une base de sel de potassium afin de former une solution aqueuse ou une suspension aqueuse $SA_2$ ;
   2) distillation, à une pression de 700 mbar ou moins, de la solution aqueuse ou de la suspension aqueuse $SA_2$ afin de former une suspension $S_1$ et refroidissement de la solution aqueuse $SA_2$ ou de la suspension aqueuse $S_1$, la température de l'étape de refroidissement étant baissée avec une rampe entre 0,1 et 8°C/heure ;
   3) séparation solide / liquide de la suspension $S_1$ et isolement des cristaux de la suspension $S_1$ obtenue à l'issue de l'étape 2) sous la forme d'une composition $C_1$.

6. Procédé selon la revendication 5, ***caractérisé* en ce que** la quantité d'ATBS.K dans la solution aqueuse ou la suspension aqueuse $SA_2$ est comprise entre 10% et 90% en poids, par rapport au poids de la solution aqueuse ou de la suspension aqueuse $SA_2$.

7. Procédé selon la revendication 5 ou 6, ***caractérisé* en ce que**, dans l'étape 2), la solution aqueuse ou la suspension aqueuse $SA_2$ est chauffée, avantageusement à une température entre 5°C et 95°C.

8. Procédé selon l'une des revendications 5 à 7, ***caractérisé* en ce que** l'étape de refroidissement de l'étape 2) est réalisée à une température entre 5°C et 95°C.

9. Procédé selon l'une des revendications 5 à *8,* ***caractérisé* en ce que** la suspension $S_1$ comprend entre 30 et 90% en poids de la forme cristalline d'ATBS.K, par rapport au poids de la suspension $S_1$.

10. Procédé selon l'une des revendications 5 à *9,* ***caractérisé* en ce que** le sel de potassium est choisi parmi l'hydroxyde de potassium, le carbonate de potassium, le bicarbonate de potassium ou leurs mélanges.

[Fig. 1]

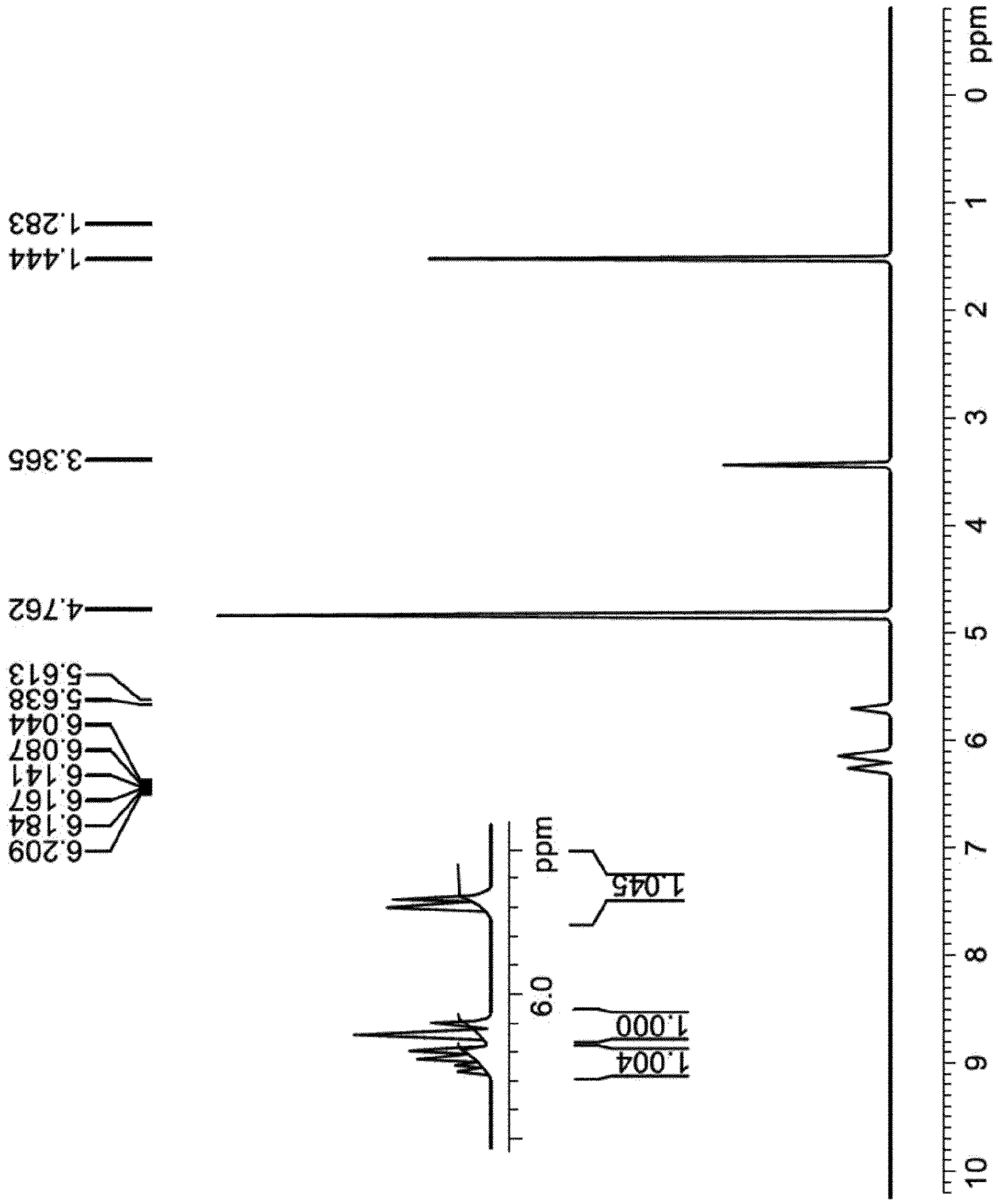
1.283
1.444
3.365
4.762
5.613
5.638
6.044
6.087
6.141
6.167
6.184
6.209
1.045
1.000
1.004
6.0
ppm
0 1 2 3 4 5 6 7 8 9 10 ppm

[Fig. 2]

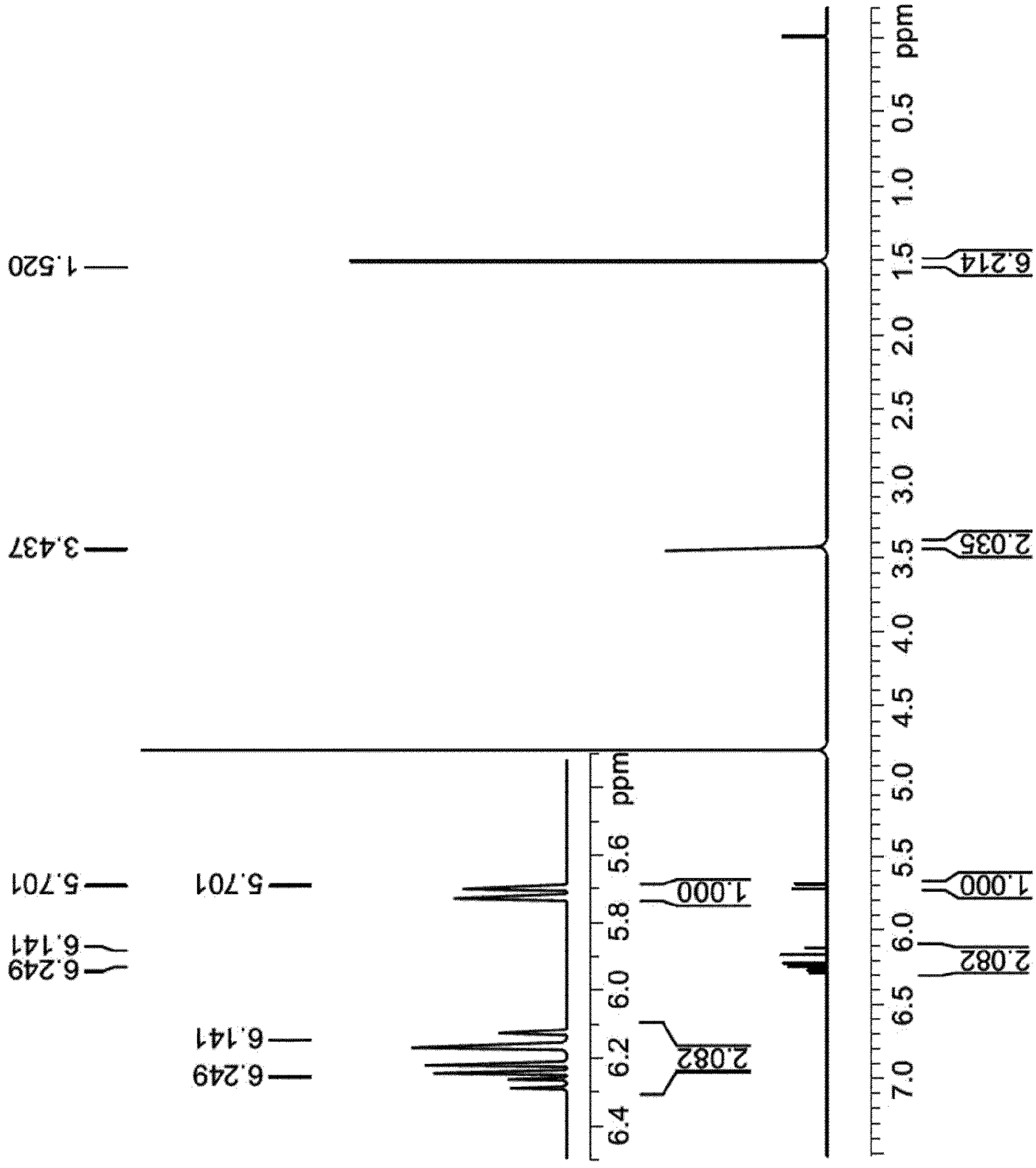
1.520
3.437
5.701
6.141
6.249
5.701
6.141
6.249
ppm
5.6
5.8
6.0
6.2
6.4
1.000
2.082
ppm
0.5
1.0
1.5
2.0
2.5
3.0
3.5
4.0
4.5
5.0
5.5
6.0
6.5
7.0
6.214
2.035
1.000
2.082

[Fig. 3]

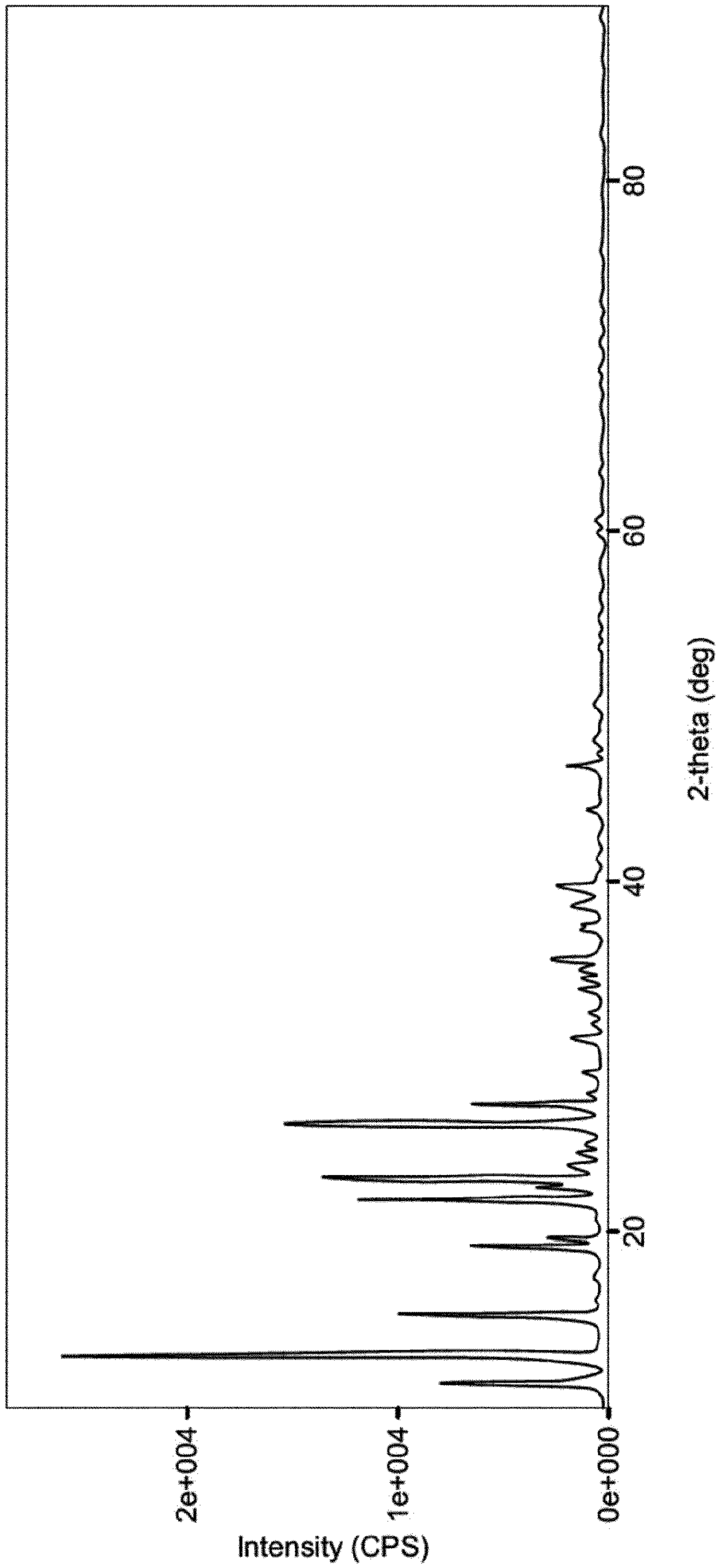

[Fig. 4]

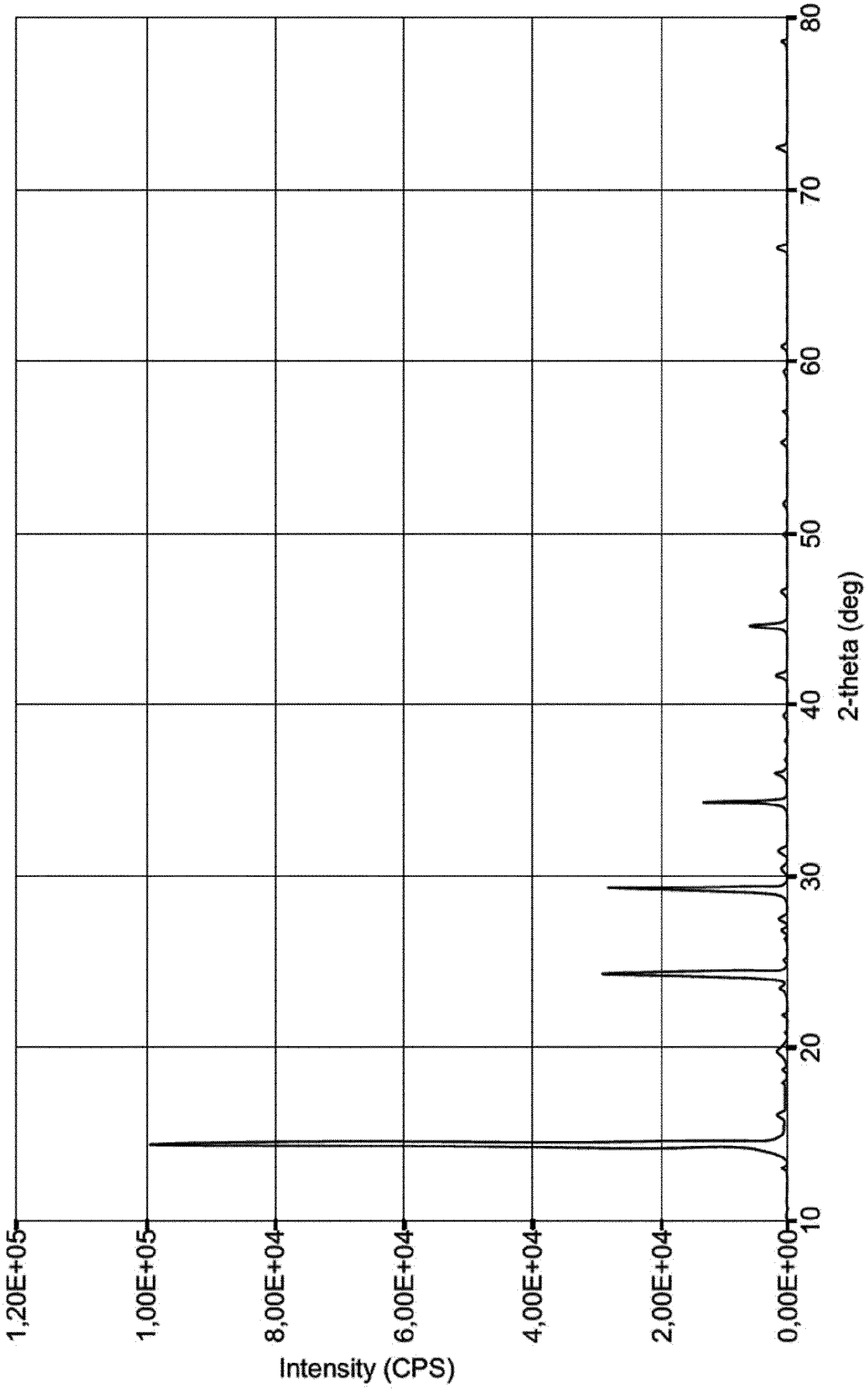
Intensity (CPS)
0,00E+00
2,00E+04
4,00E+04
6,00E+04
8,00E+04
1,00E+05
1,20E+05
2-theta (deg)
10
20
30
40
50
60
70
80

[Fig. 5]

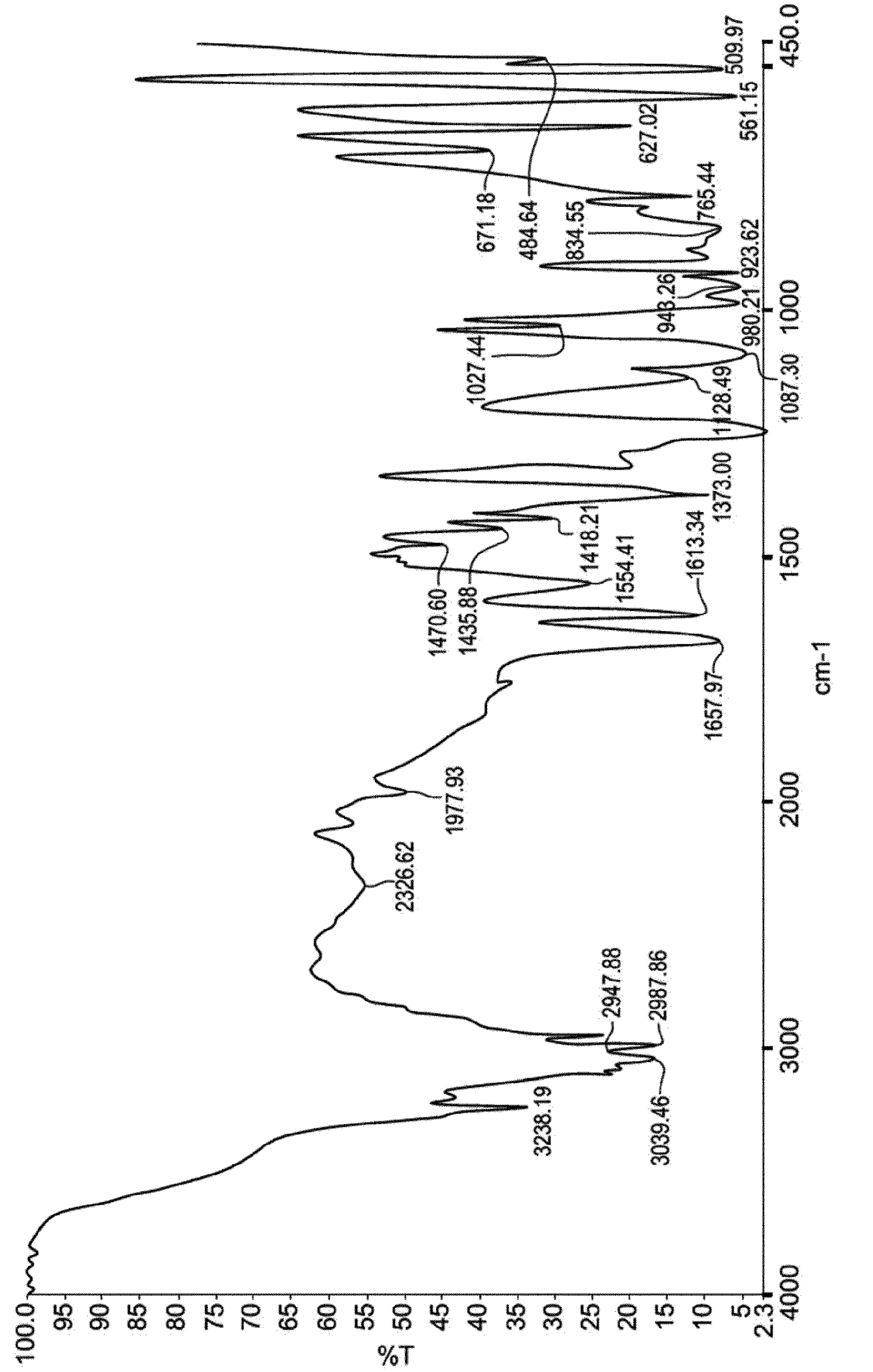
%T
cm-1
3238.19
3039.46
2947.88
2987.86
2326.62
1977.93
1657.97
1613.34
1554.41
1470.60
1435.88
1418.21
1373.00
1128.49
1087.30
1027.44
980.21
943.26
923.62
834.55
765.44
671.18
484.64
627.02
561.15
509.97

[Fig. 6]

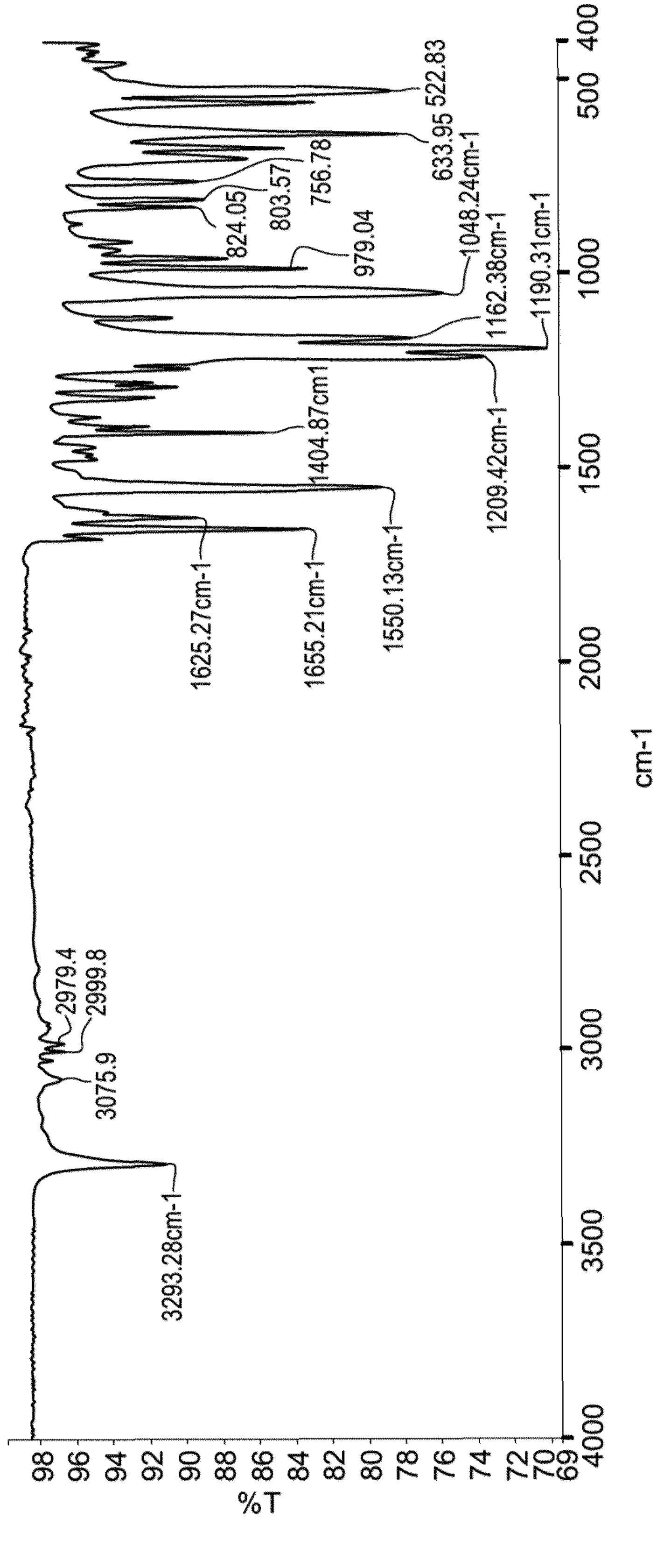

%T
cm-1
3293.28cm-1
3075.9
2999.8
2979.4
1655.21cm-1
1625.27cm-1
1550.13cm-1
1404.87cm1
1209.42cm-1
1190.31cm-1
1162.38cm-1
1048.24cm-1
979.04
824.05
803.57
756.78
633.95
522.83

[Fig. 7]

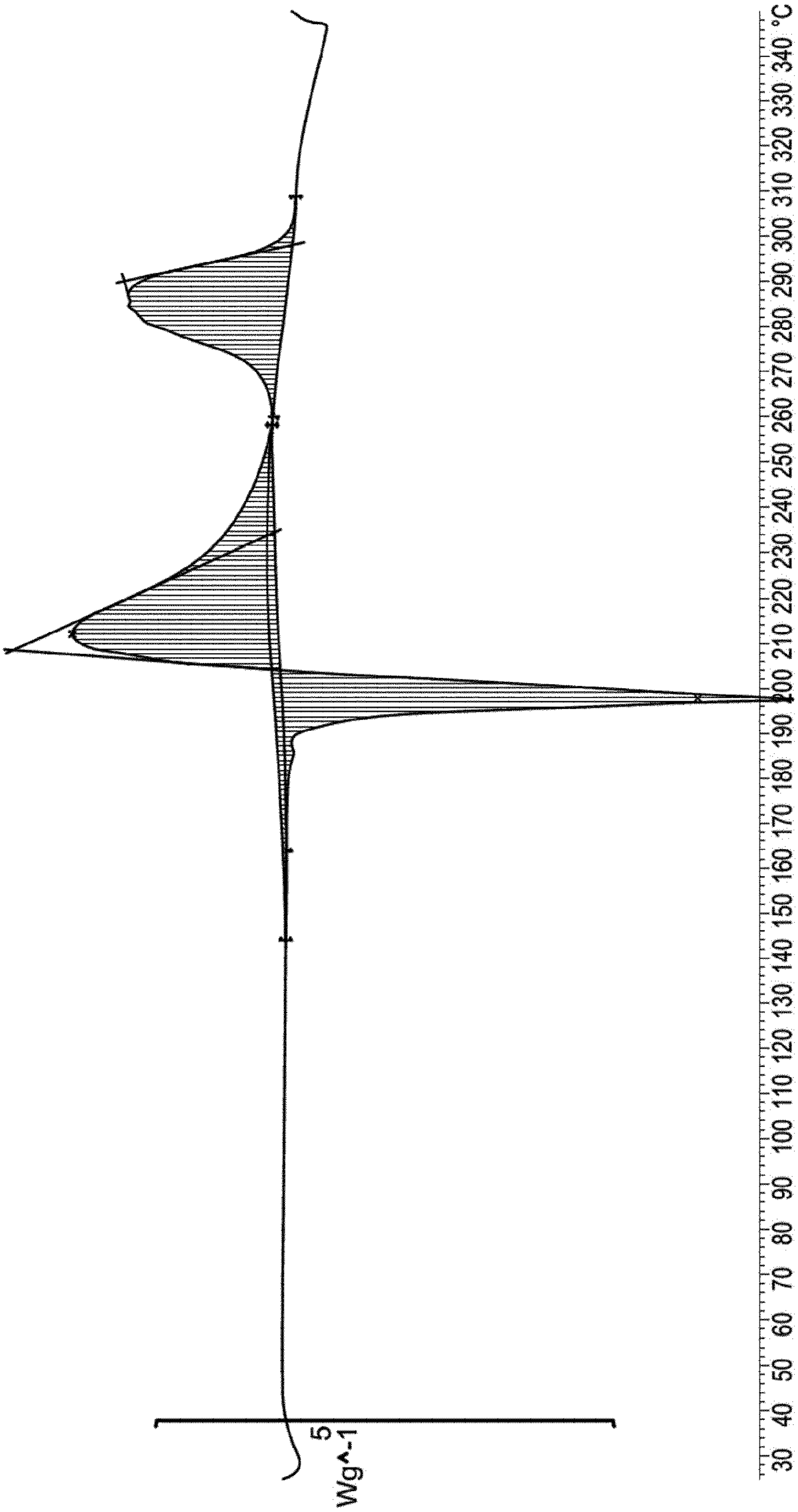

Wg^-1
5
30 40 50 60 70 80 90 100 110 120 130 140 150 160 170 180 190 200 210 220 230 240 250 260 270 280 290 300 310 320 330 340 °C

[Fig. 8]

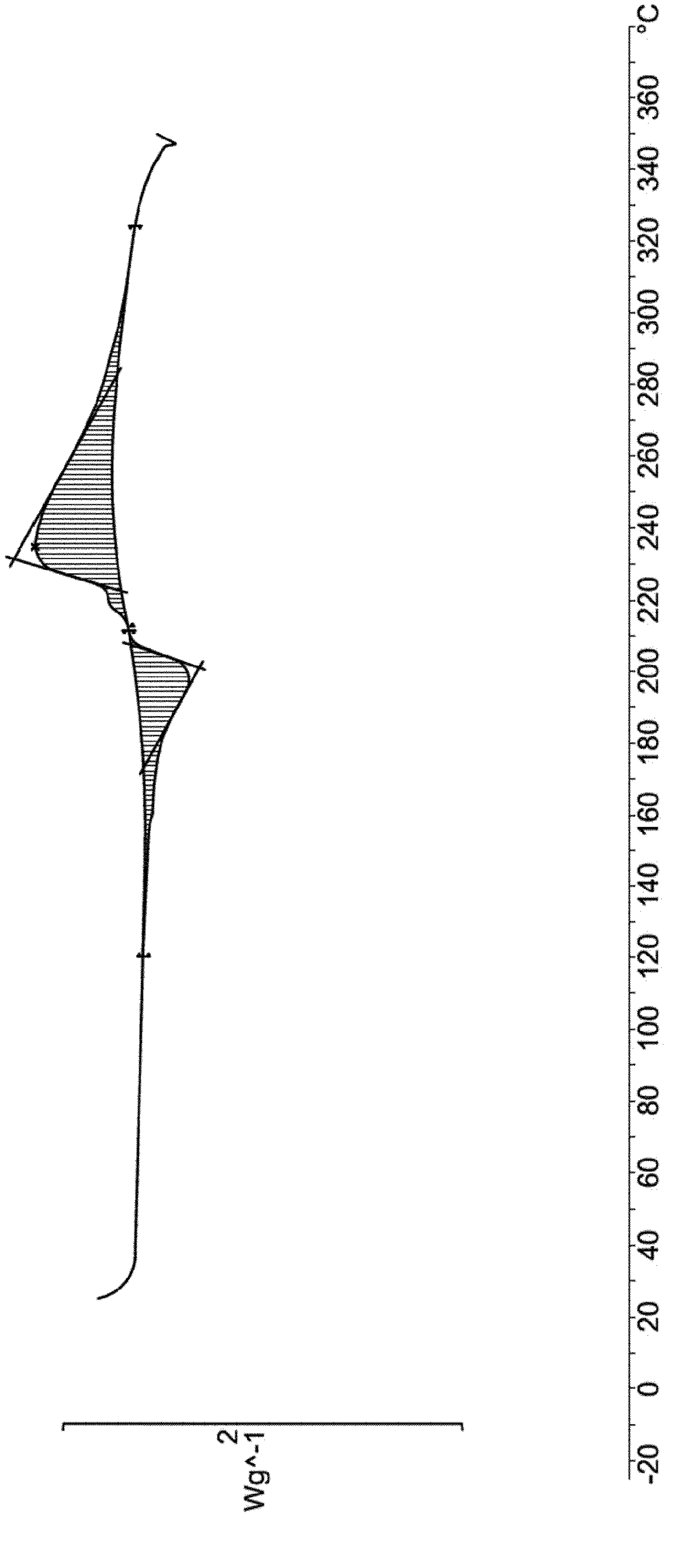
°C
360
340
320
300
280
260
240
220
200
180
160
140
120
100
80
60
40
20
0
-20
2
Wg^-1

[Fig. 9]

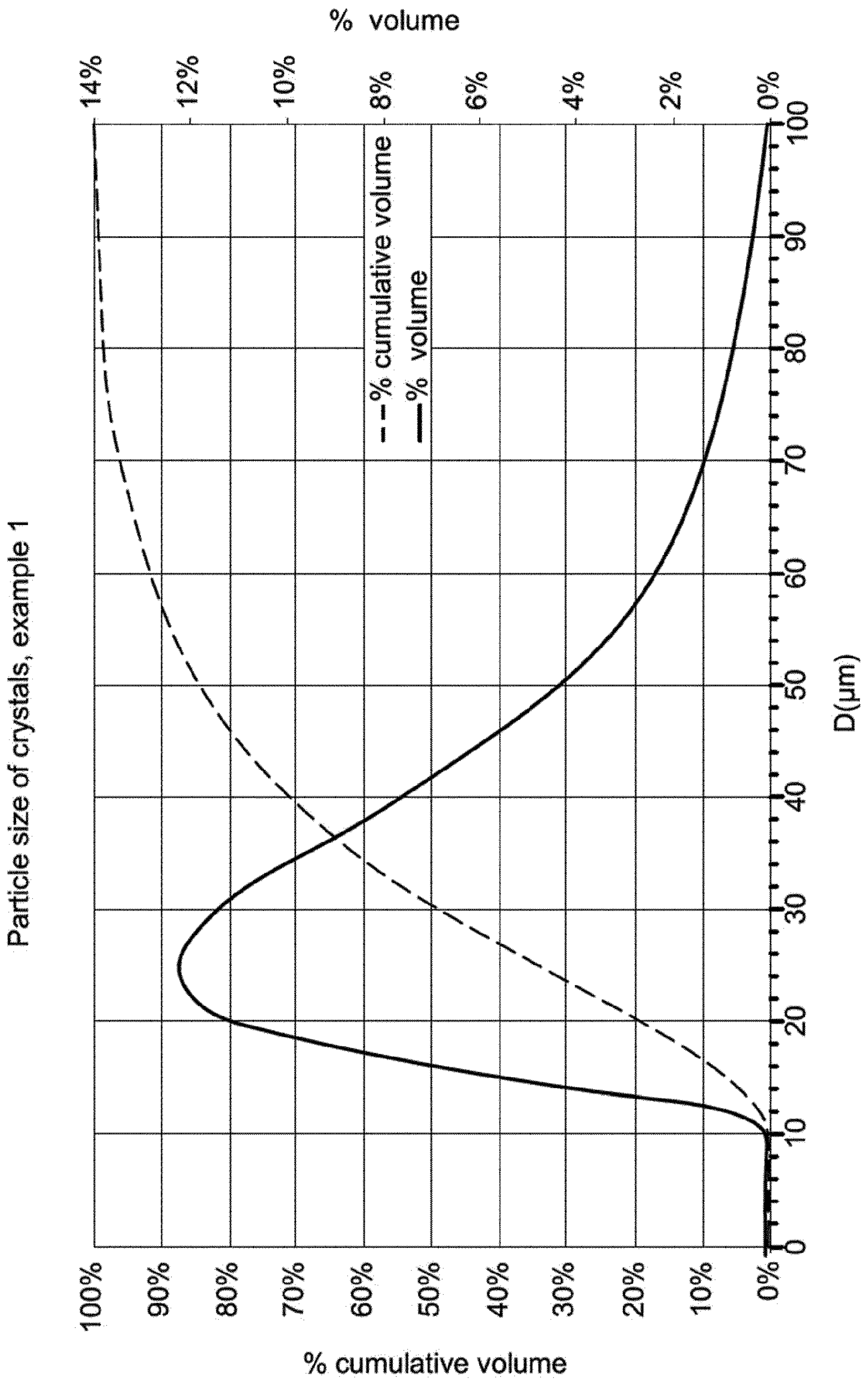
Particle size of crystals, example 1
% volume
14%
12%
10%
8%
6%
4%
2%
0%
- - % cumulative volume
— % volume
100%
90%
80%
70%
60%
50%
40%
30%
20%
10%
0%
% cumulative volume
0
10
20
30
40
50
60
70
80
90
100
D(µm)

[Fig. 10]

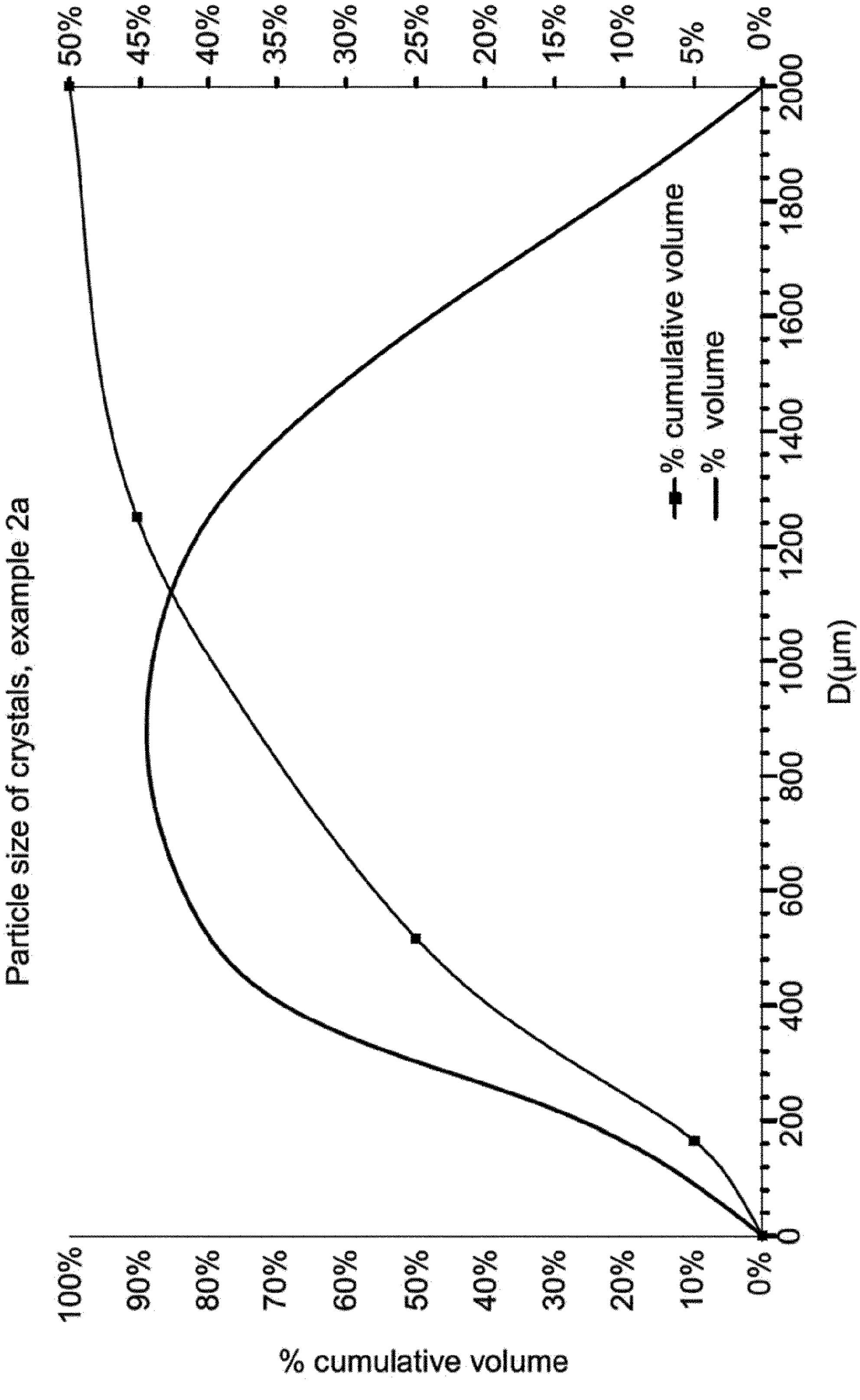
Particle size of crystals, example 2a
% cumulative volume
% volume
D(µm)
0
200
400
600
800
1000
1200
1400
1600
1800
2000
100%
90%
80%
70%
60%
50%
40%
30%
20%
10%
0%
50%
45%
40%
35%
30%
25%
20%
15%
10%
5%
0%
% cumulative volume

[Fig. 11]

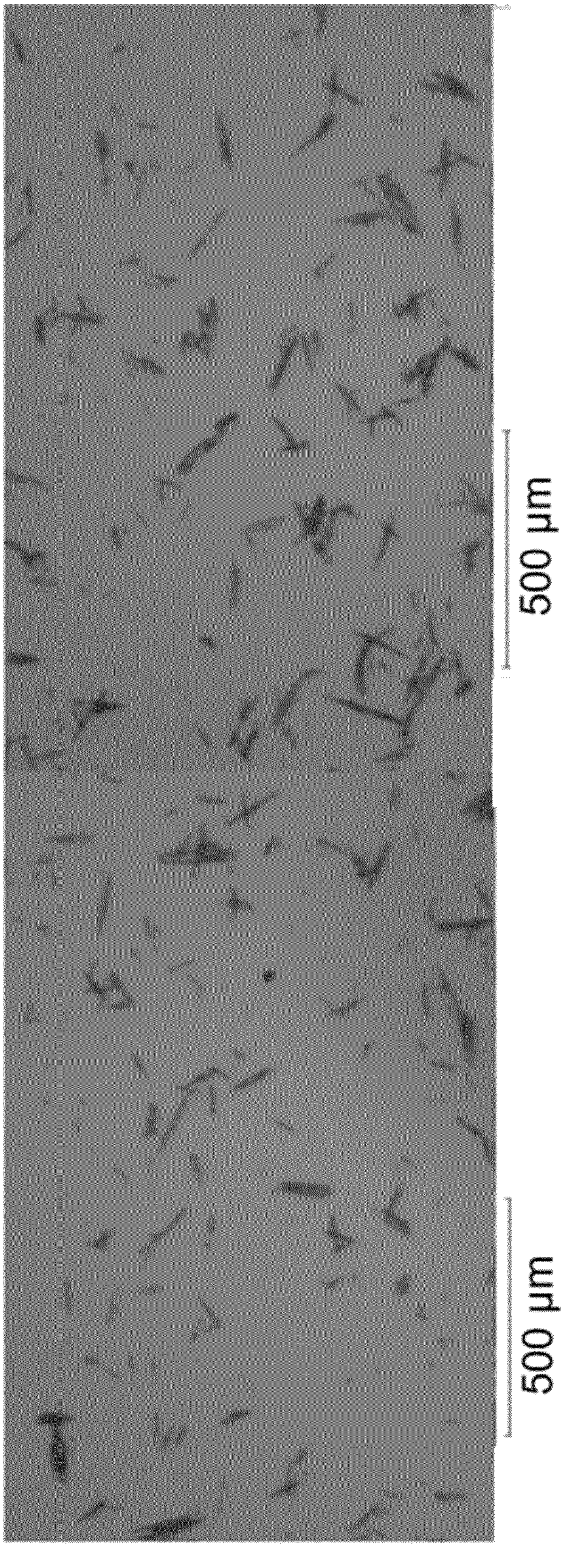

[Fig. 12a]

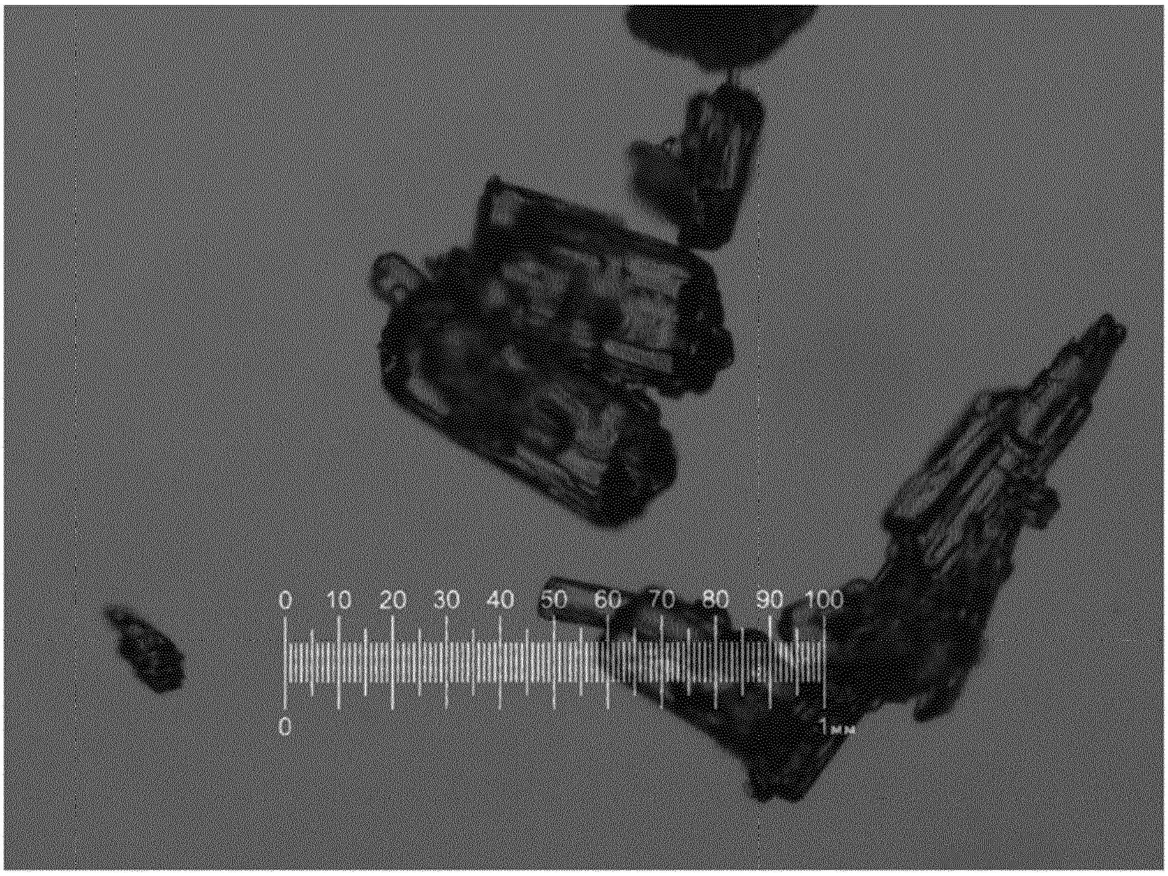


[Fig 12b]

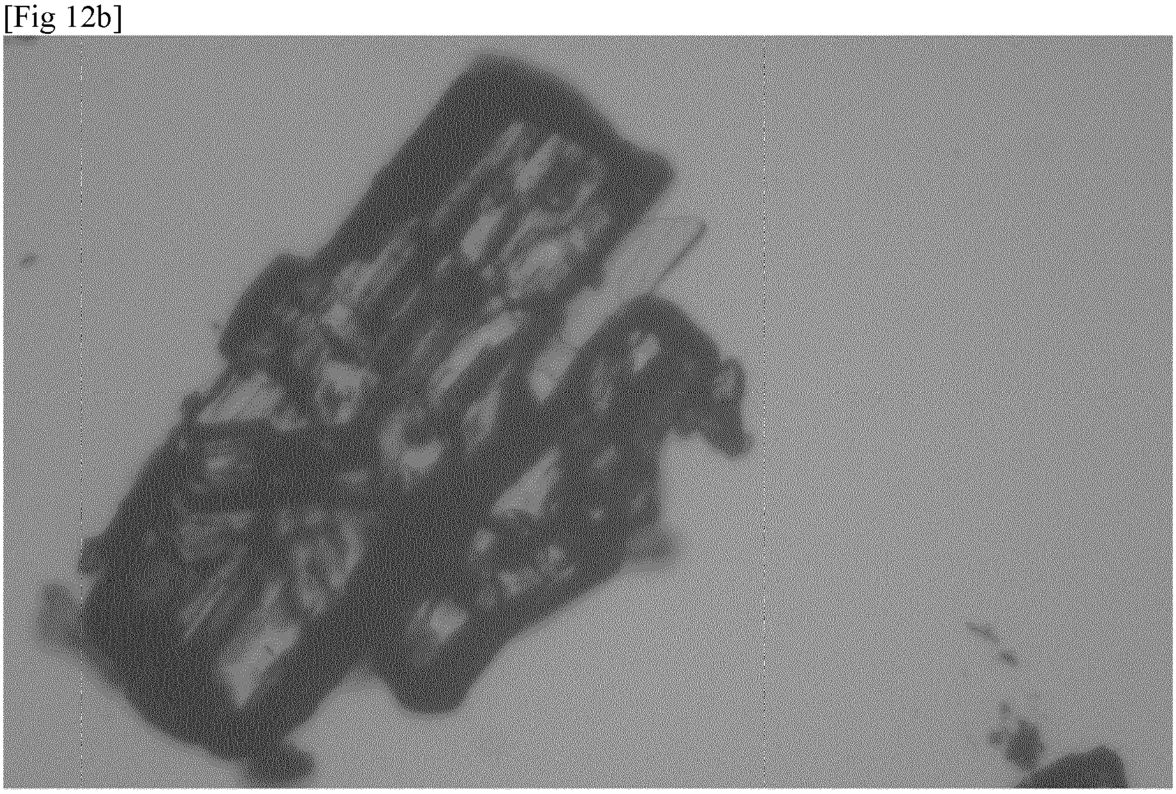

[Fig 12c]

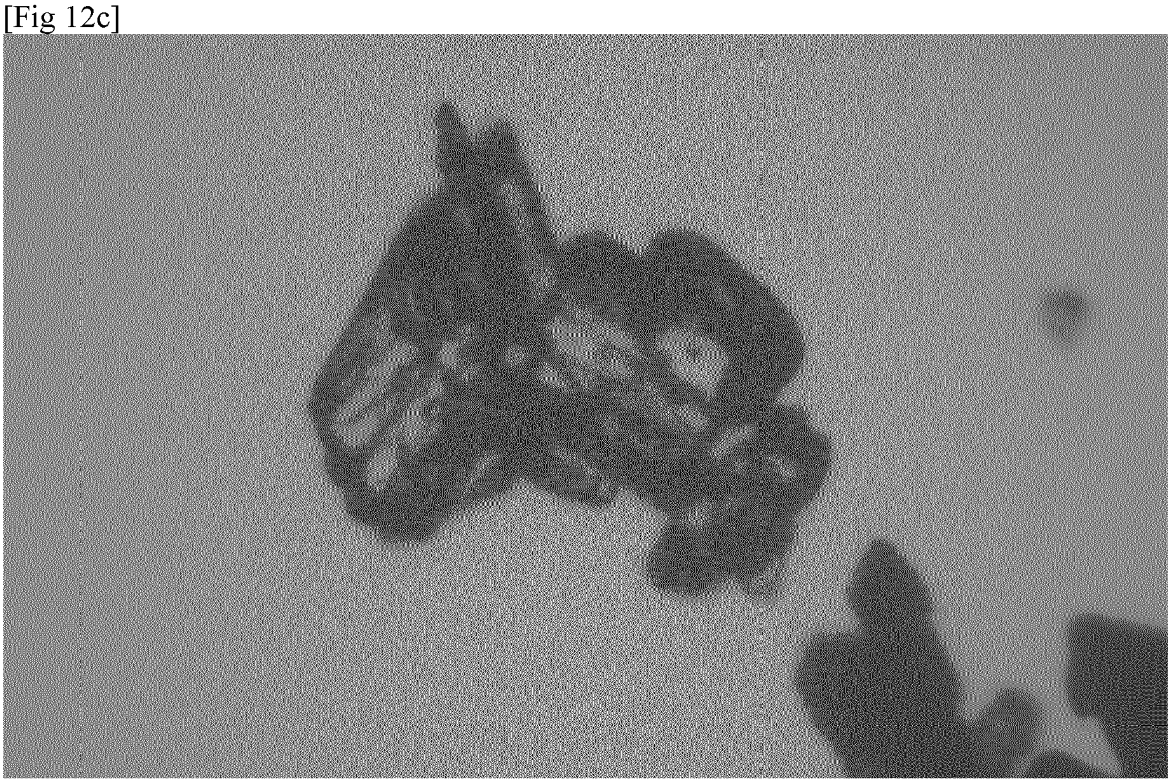

[Fig 12d]

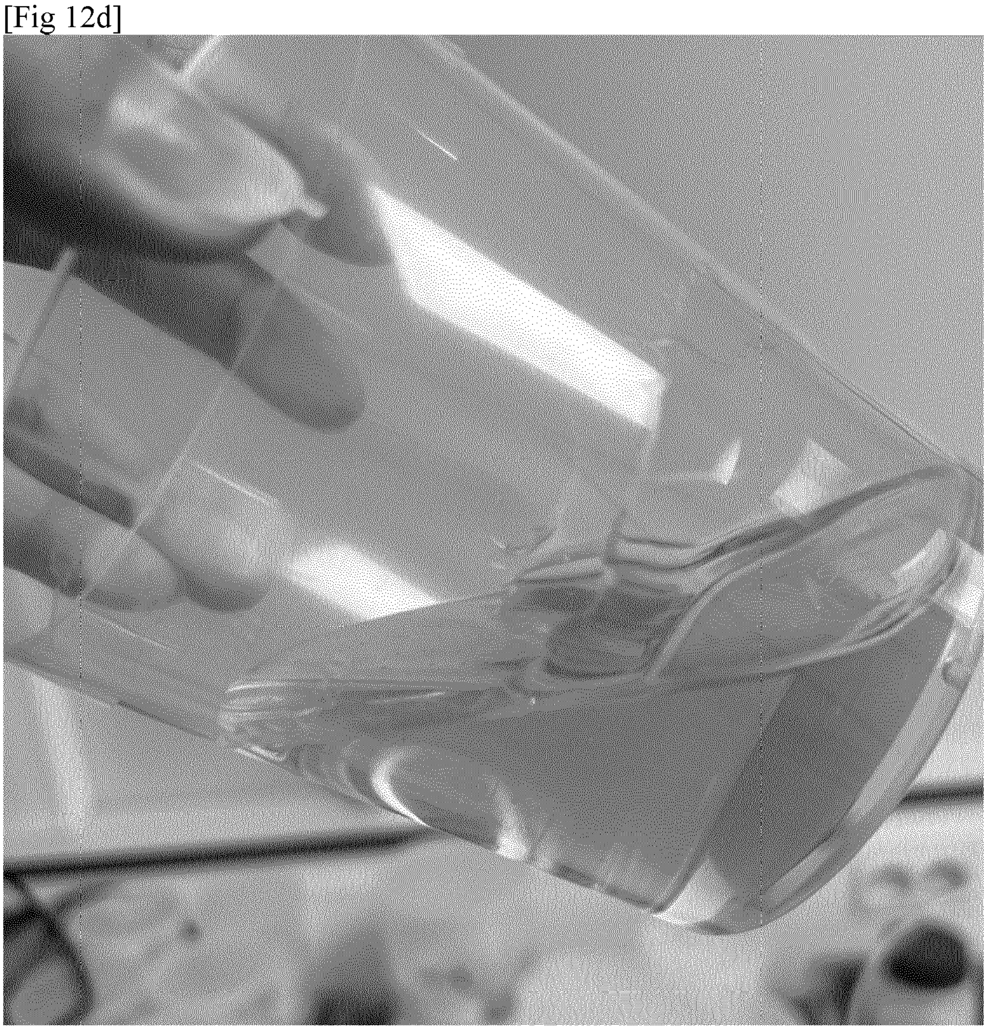

[Fig 12e]

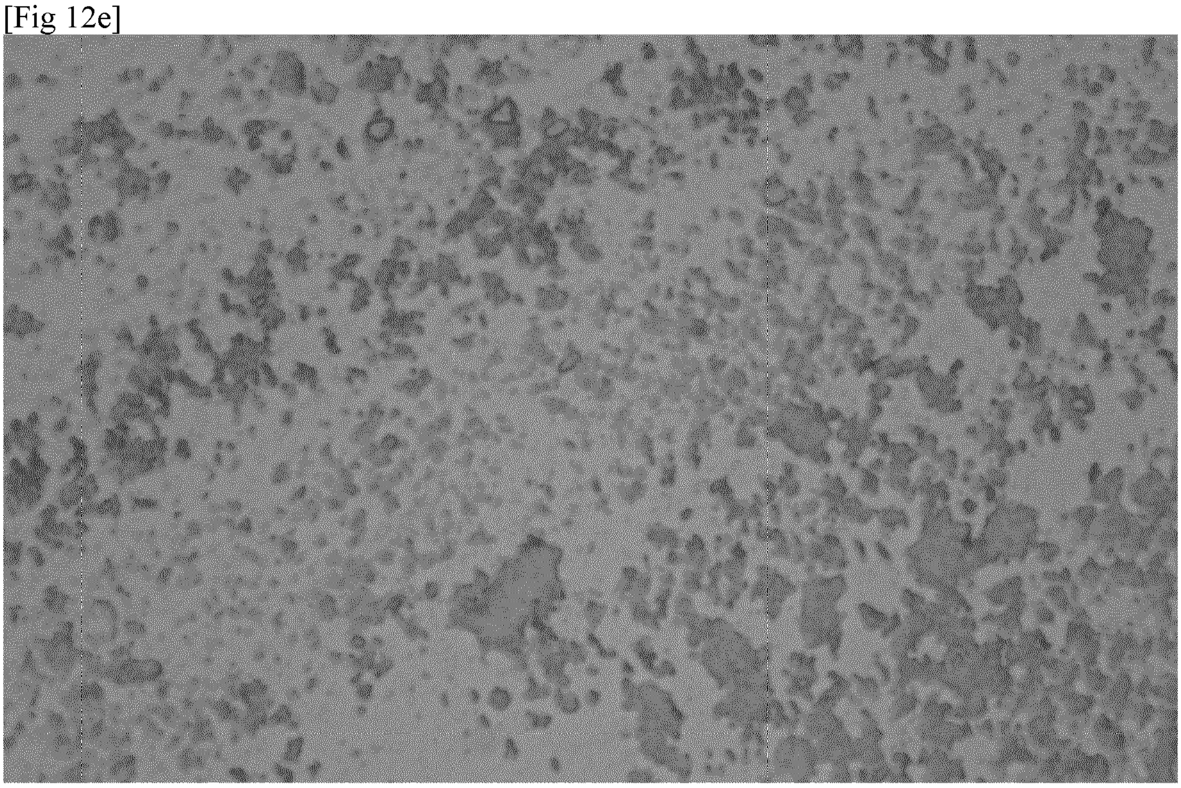

[Fig 12f]

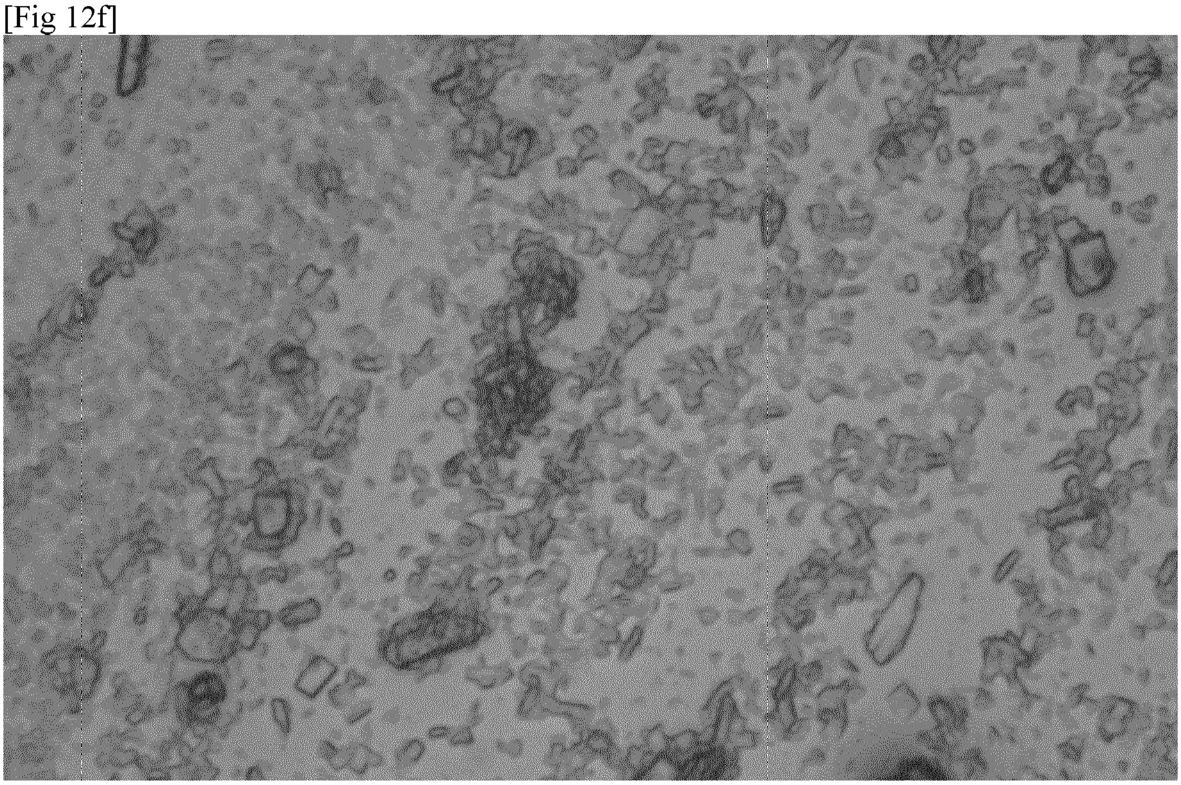

[Fig 12g]

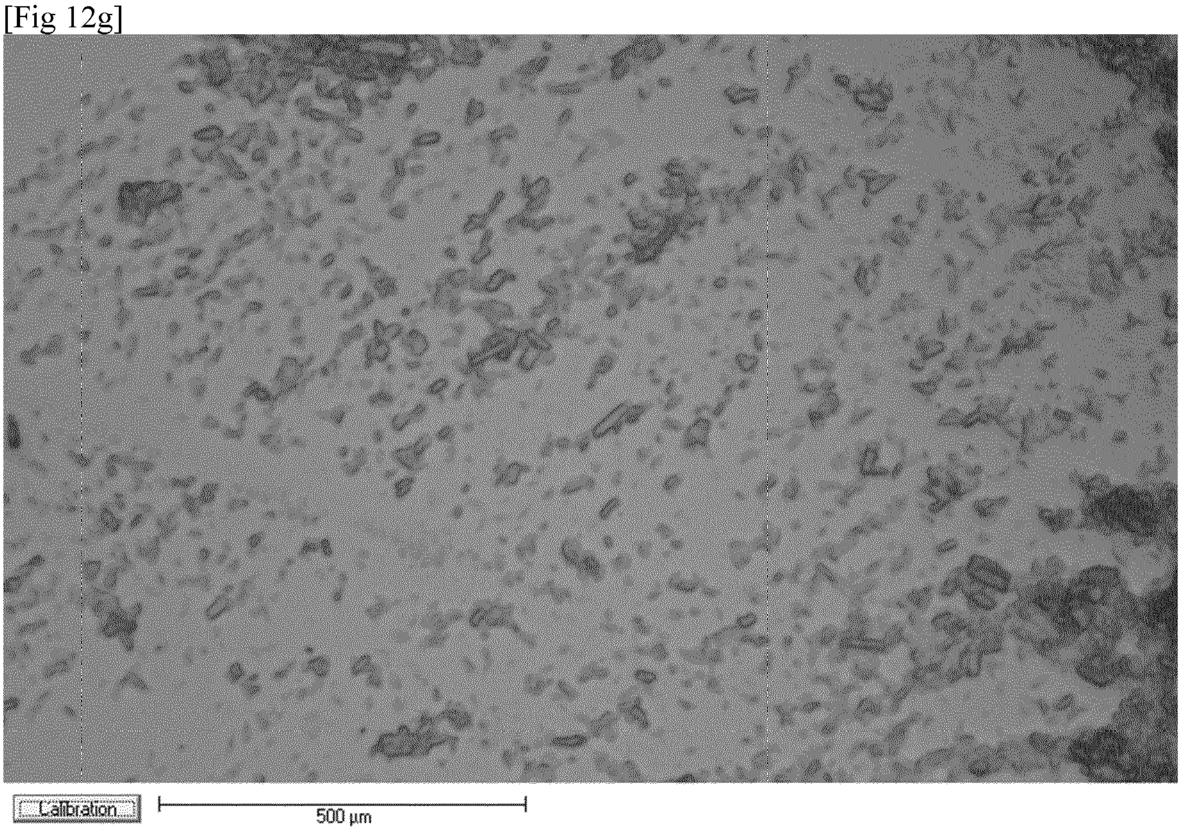


[Fig. 13]

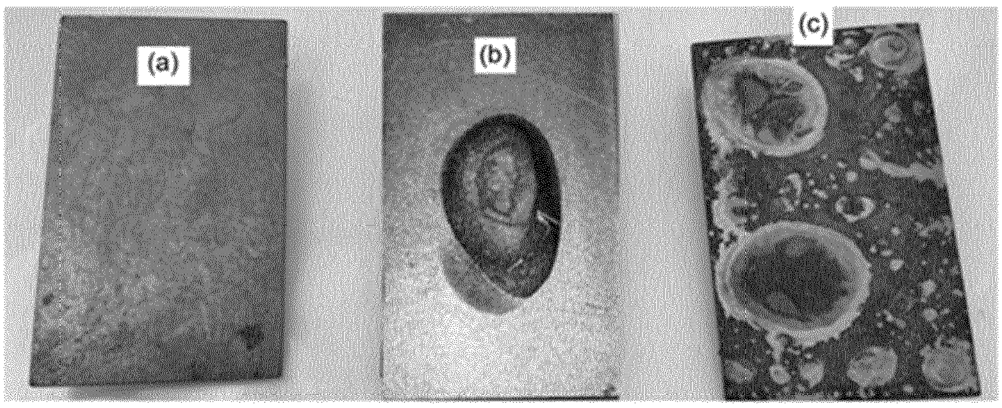


(a) Control plate

(b) Plate in the presence of ATBS.K according to example 2a

(c) Plate in the presence of ATBS according to example 1

[Fig. 14]

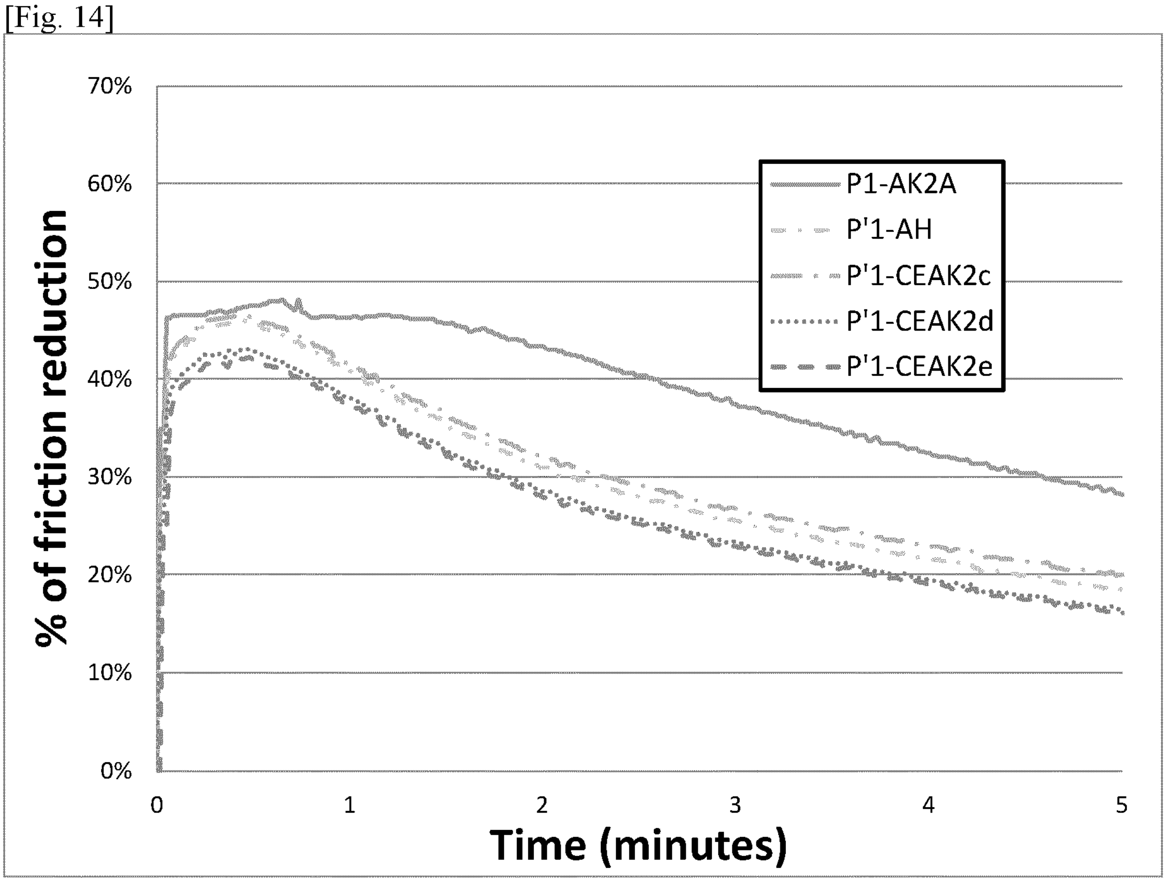
70%
60%
50%
40%
30%
20%
10%
0%
% of friction reduction
0
1
2
3
4
5
Time (minutes)
P1-AK2A
P'1-AH
P'1-CEAK2c
P'1-CEAK2d
P'1-CEAK2e

[Fig. 15]

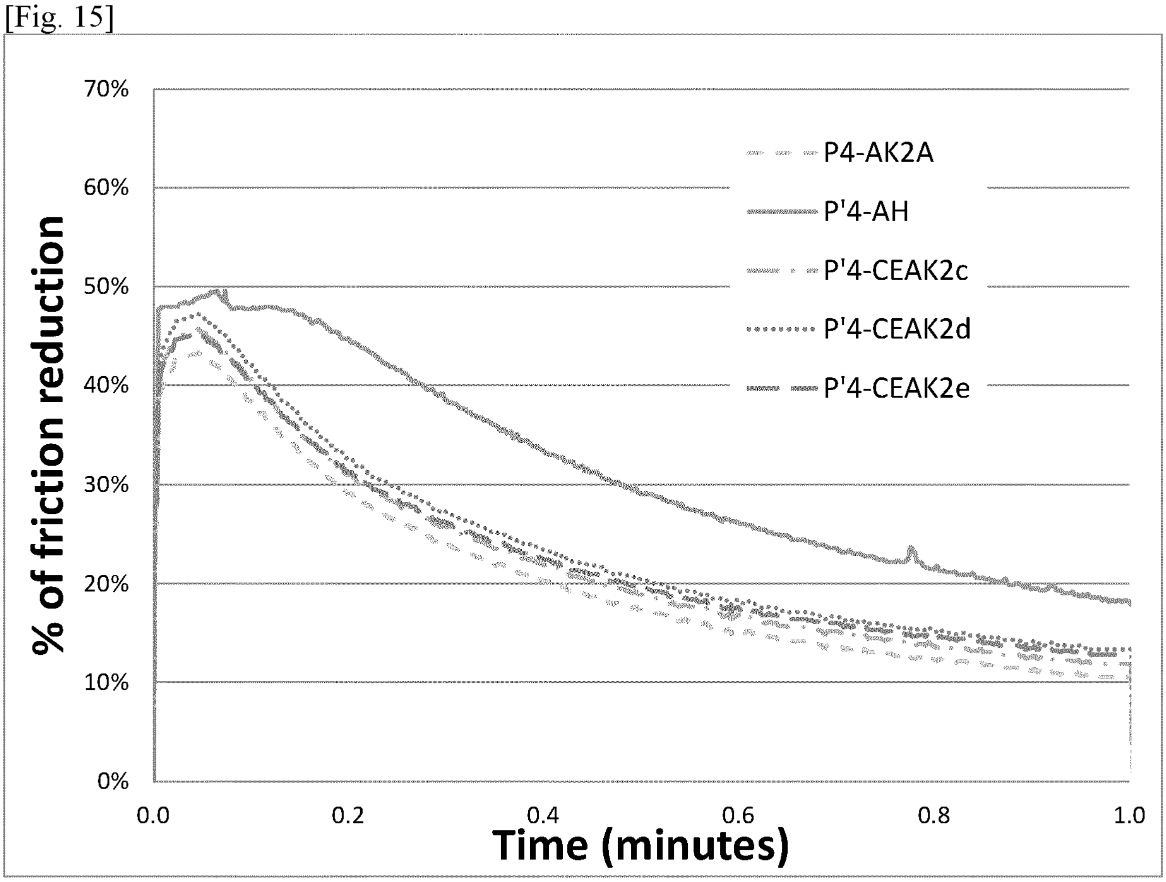
70%
60%
50%
40%
30%
20%
10%
0%
% of friction reduction
0.0
0.2
0.4
0.6
0.8
1.0
Time (minutes)
P4-AK2A
P'4-AH
P'4-CEAK2c
P'4-CEAK2d
P'4-CEAK2e

[Fig. 16]

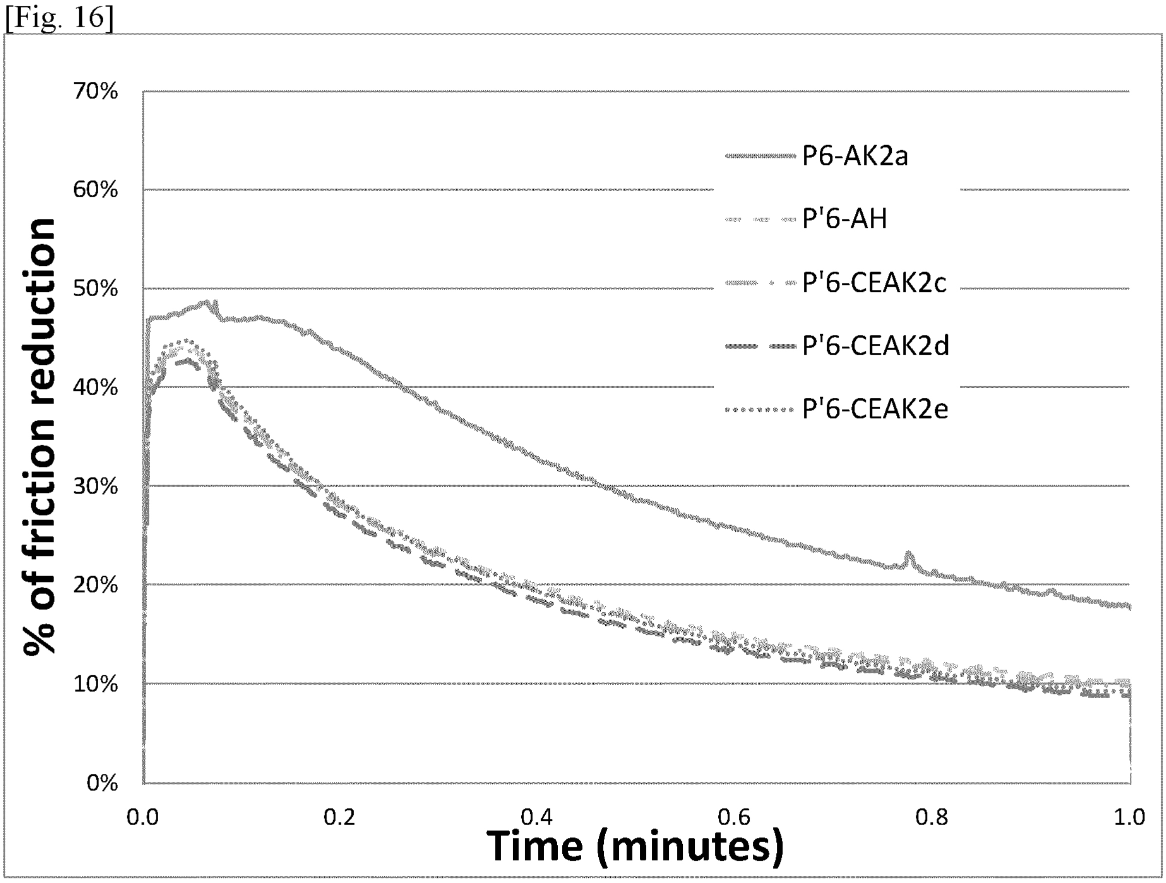
70%
60%
50%
40%
30%
20%
10%
0%
% of friction reduction
0.0
0.2
0.4
0.6
0.8
1.0
Time (minutes)
P6-AK2a
P'6-AH
P'6-CEAK2c
P'6-CEAK2d
P'6-CEAK2e

[Fig. 17]

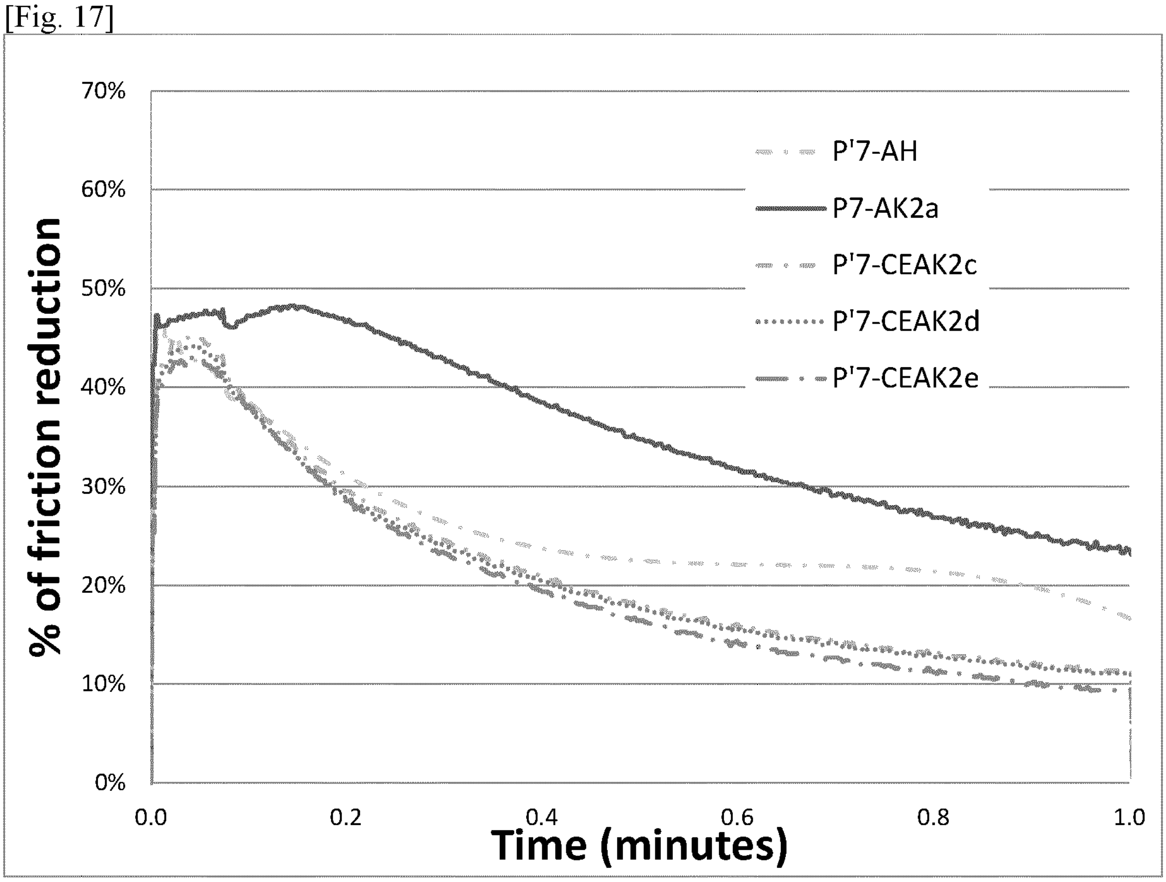
70%
60%
50%
40%
30%
20%
10%
0%
% of friction reduction
0.0
0.2
0.4
0.6
0.8
1.0
Time (minutes)
P'7-AH
P7-AK2a
P'7-CEAK2c
P'7-CEAK2d
P'7-CEAK2e

[Fig. 18]

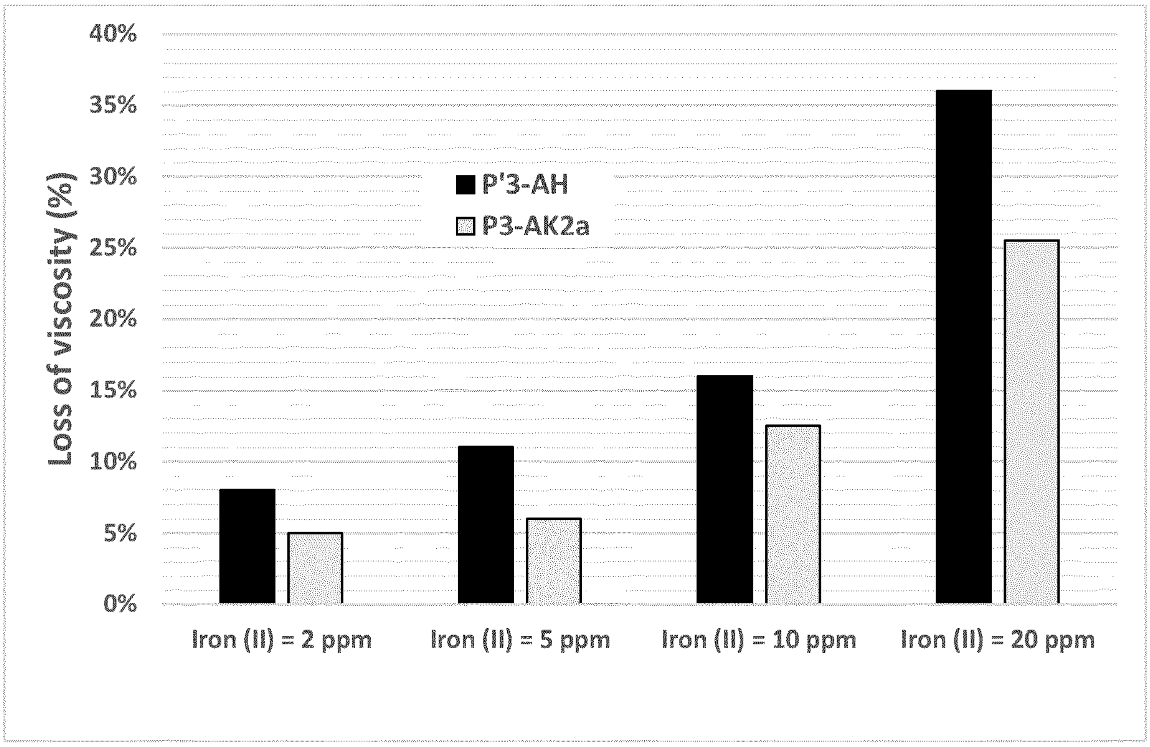
40%
35%
30%
25%
20%
15%
10%
5%
0%
Loss of viscosity (%)
P'3-AH
P3-AK2a
Iron (II) = 2 ppm
Iron (II) = 5 ppm
Iron (II) = 10 ppm
Iron (II) = 20 ppm

[Fig. 19]

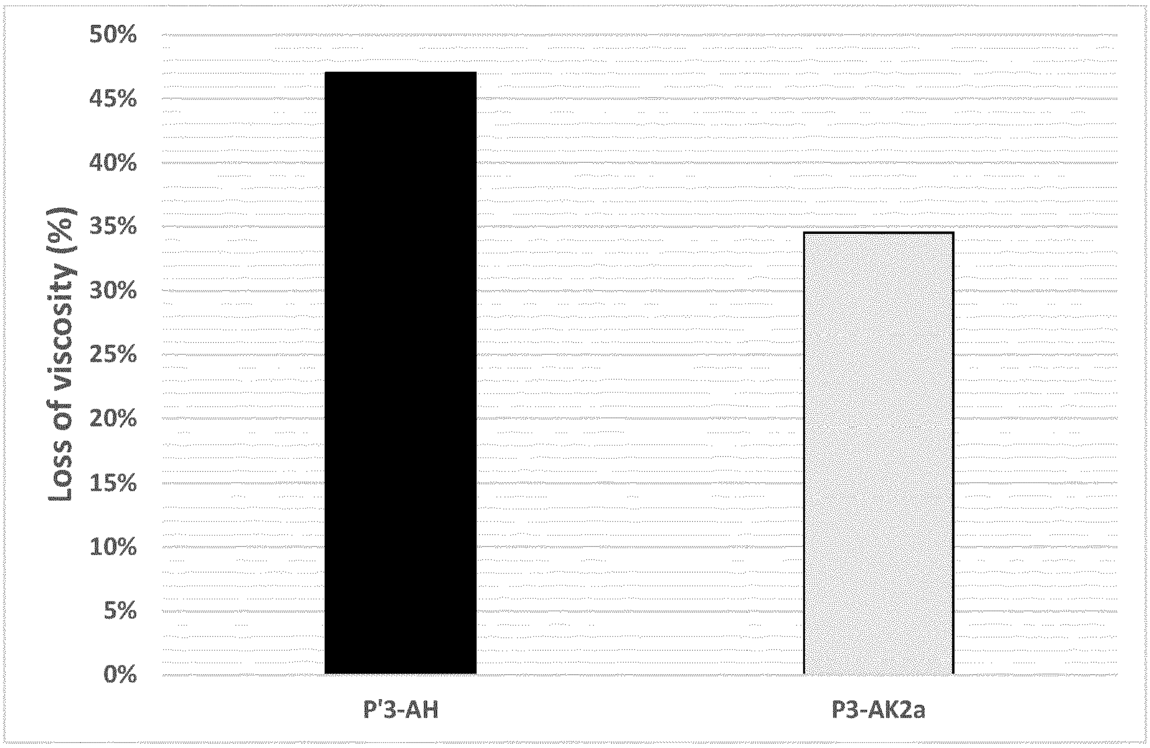
50%
45%
40%
35%
30%
25%
20%
15%
10%
5%
0%
Loss of viscosity (%)
P'3-AH
P3-AK2a

[Fig. 20]

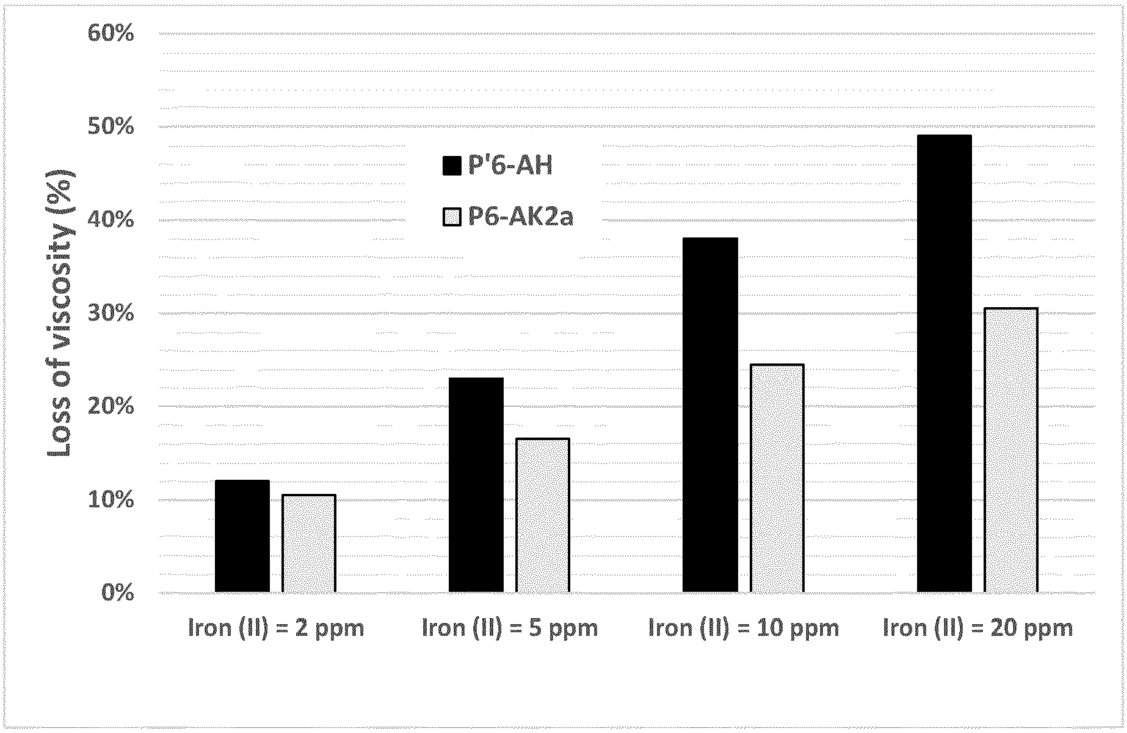
60%
50%
40%
30%
20%
10%
0%
Loss of viscosity (%)
P'6-AH
P6-AK2a
Iron (II) = 2 ppm
Iron (II) = 5 ppm
Iron (II) = 10 ppm
Iron (II) = 20 ppm

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6448347 B
- CN 102351744
- US 6331647 B
- WO 2009072480 A
- JP 2008307822 A
- JP 2003137857 A
- WO 2018172676 A
- WO 2021123599 A
- EP 2203245 A
- US 8186871 B
- WO 2018020175 A
- WO 2010133258 A
- WO 2013079507 A